(19) Europäisches Patentamt
European Patent Office
Office européen des brevets

(11) EP 0 623 140 B1

(12) **FASCICULE DE BREVET EUROPEEN**

(45) Date de publication et mention de la délivrance du brevet:
**22.04.1998 Bulletin 1998/17**

(21) Numéro de dépôt: **93902339.6**

(22) Date de dépôt: **17.12.1992**

(51) Int Cl.⁶: **C07J 43/00**, A61K 31/58, C07J 31/00, A61K 31/565, C07J 41/00

(86) Numéro de dépôt international:
**PCT/FR92/01193**

(87) Numéro de publication internationale:
**WO 93/13123 (08.07.1993 Gazette 1993/16)**

(54) **NOUVEAUX 19-NOR STEROIDES AYANT EN POSITION 11BETA UNE CHAINE THIOCARBONEE, LEUR PROCEDE DE PREPARATION ET LES INTERMEDIAIRES DE CE PROCEDE, LEUR APPLICATION COMME MEDICAMENTS ET LES COMPOSITIONS**

NEUE 19-NOR STEROIDE, DIE AN DER 11-BETA STELLE AN EINE THIOKOHLENSTOFFGRUPPE GEBUNDEN SIND, EIN VERFAHREN ZU IHRER HERSTELLUNG UND ZWISCHENPRODUKTE DAZU

NOVEL 19-NOR STEROIDS HAVING A THIOCARBON-CONTAINING CHAIN IN THE 11BETA POSITION, A METHOD FOR PREPARING SAME AND INTERMEDIATES THEREFOR, MEDICINAL USES THEREOF AND COMPOSITIONS CONTAINING SAID STEROIDS

(84) Etats contractants désignés:
**AT BE CH DE DK ES FR GB GR IE IT LI LU NL PT SE**

(30) Priorité: **20.12.1991 FR 9115856**

(43) Date de publication de la demande:
**09.11.1994 Bulletin 1994/45**

(73) Titulaire: **HOECHST MARION ROUSSEL**
**92800 Puteaux (FR)**

(72) Inventeurs:
- **CLAUSSNER, André**
  **93250 Villemomble (FR)**
- **NIQUE, François**
  **94170 Le Perreux (FR)**
- **TEUTSCH, Jean-Georges**
  **93500 Pantin (FR)**
- **VAN DE VELDE, Patrick**
  **75019 Paris (FR)**

(74) Mandataire: **Vieillefosse, Jean-Claude et al**
**Hoechst Marion Roussel**
**Département des Brevets**
**102, Route de Noisy**
**93235 Romainville Cédex (FR)**

(56) Documents cités:
**EP-A- 0 097 572** **EP-A- 0 384 842**
**EP-A- 0 471 612**

- **JOURNAL OF MEDICINAL CHEMISTRY. vol. 33, no. 12, Décembre 1990, WASHINGTON US pages 3155 - 3160 R. HANSON ET AL 'Synthesis and Estrogen Receptor Binding of Novel 11-beta-Substituted Estra-1,3,5(10)-triene-3-17-beta-Diols'**
- **JOURNAL OF THE CHEMICAL SOCIETY, CHEMICAL COMMUNICATIONS. no. 18, 15 Septembre 1989, LETCHWORTH GB pages 1330 - 1331 E. NAPOLITANO ET AL 'Synthesis of 1,11-bet a-Ethanoestra-1,3,5(10)-triene-3,17-beta-d iol: A Novel Bridged Steroid Derivative.'**

Remarques:
Le dossier contient des informations techniques présentées postérieurement au dépôt de la demande et ne figurant pas dans le présent fascicule.

EP 0 623 140 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention concerne de nouveaux 19-Nor stéroïdes ayant en position 11béta une chaîne thiocarbonée, leur procédé de préparation et les intermédiaires de ce procédé, leur application comme médicaments et les compositions pharmaceutiques les contenant.

La demande de brevet européen EP 0.384.842 décrit des 19-Nor stéroïdes ayant en 11béta une chaîne carbonée comportant une fonction amide ou carbamate.

L'invention a pour objet les composés de formule (I) :

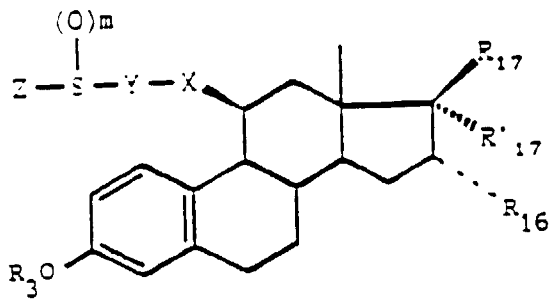

(I)

dans laquelle $R_{17}$ et $R'_{17}$ sont tels que :

- soit $R_{17}$ et $R'_{17}$ forment ensemble une fonction cétone, une fonction oxime, une fonction hydrazono ou un radical méthylène,
- soit $R_{17}$ est un radical hydroxyle, un radical hydroxyméthyle ou un radical acyloxy ayant au plus 12 atomes de carbone et $R'_{17}$ représente un atome d'hydrogène, un radical alkyle, alcényle ou alcynyle ayant au plus 8 atomes de carbone, chacun de ces substituants étant éventuellement substitué,
- $R_3$ représente un atome d'hydrogène, un radical alkyle linéaire ou ramifié ou un radical alkyle cyclique ayant au plus 8 atomes de carbone ou un radical acyle ayant au plus 12 atomes de carbone,
- $R_{16}$ représente un atome d'hydrogène, un atome d'halogène ou un radical alkyle ayant au plus 8 atomes de carbone,

m a la valeur 0, 1 ou 2,
X, Y et Z sont tels que :

- X représente un radical méthylène, un groupement arylène ou arylènoxy ayant au plus 10 atomes de carbone lié au stéroïde par un atome de carbone,
- Y représente une chaîne aliphatique linéaire ou ramifiée, saturée ou insaturée, renfermant de 1 à 18 atomes de carbone, éventuellement interrompue par un atome d'oxygène,
- Z représente :
  un radical alkyle linéaire ou ramifié, renfermant de 1 à 8 atomes de carbone et éventuellement substitué ou un radical aryle ou arylalkyle, chacun de ces radicaux étant éventuellement substitué, dans lesquels le radical alkyle renferme au plus 6 atomes de carbone et le radical aryle représente un radical monocyclique comprenant 5 ou 6 chaînons ou un radical constitué de cycles condensés comprenant 8 à 10 chaînons, ces radicaux renfermant éventuellement 1 ou plusieurs hétéroatomes choisis parmi les atomes d'oxygène, d'azote ou de soufre,

les radicaux alkyle que peuvent représenter $R'_{17}$ et Z, les radicaux alcényle ou alcynyle que peut représenter $R'_{17}$ et les radicaux aryle ou arylalkyle que peut représenter Z étant éventuellement substitués par un ou plusieurs radicaux choisis parmi les radicaux :

- halogènes,
- amino, alkylamino ou dialkylamino dans lesquels le ou les radicaux alkyle ont de 1 à 4 atomes de carbone,
- hydroxyle,
- carboxy libres, estérifiés ou salifiés,
- alkyle ayant de 1 à 8 atomes de carbone, éventuellement substitué par un ou plusieurs atomes d'halogène,
- oxo, cyano, nitro,
- acyle ou acyloxy ayant au plus 12 atomes de carbone,

- alkoxy ou alkylthio ayant de 1 à 4 atomes de carbone,
- alcényle ou alcynyle ayant au plus 4 atomes de carbone,
- aryle tel que défini ci-dessus,

et les sels d'addition de ceux-ci.

Par radical acyloxy il peut s'agir notamment du dérivé d'un acide aliphatique ou cycloaliphatique saturé ou insaturé et notamment d'un acide alcanoïque tel que par exemple l'acide acétique, propionique, butyrique ou isobutyrique, valérique ou undécylique, d'un acide hydroxyalcanoïque tel que par exemple l'acide hydroxyacétique ; d'un acide cycloalcoylcarboxylique ou cycloalcoylalcanoïque tel que par exemple l'acide cyclopropyl, cyclopentyl ou cyclohexylcarboxylique, cyclopentyle ou cyclohexyle acétique ou propionique, d'un acide benzoïque, d'un acide salicylique ou d'un acide phénylalcanoïque tel que l'acide phényle acétique ou phényle propionique, d'un amino acide tel que l'acide diéthylamino acétique ou aspartique, de l'acide formique ou d'un diacide éventuellement salifié, tel que par exemple l'acide butanedioïque ou le sel monosodique de celui-ci. Il s'agit de préférence du dérivé de l'acide acétique, propionique ou butyrique.

Par radical acyle, on entend les radicaux correspondants aux radicaux acyloxy précédents.

Par radical alkyle, il peut s'agir du radical méthyle, éthyle, propyle, isopropyle, butyle, isobutyle, n-pentyle, n-hexyle, 2-méthyl pentyle, 2,3-diméthyl butyle, n-heptyle, 2-méthyl hexyle, 2,2-diméthyl pentyle, 3,3-diméthyl pentyle, 3-éthylpentyle, n-octyle, 2,2-diméthylhexyle, 3,3-diméthylhexyle, 3-méthyl 3-éthyl pentyle.

Il s'agit de préférence du radical méthyle.

Lorsque $R_3$ représente un radical alkyle cyclique, il peut s'agir d'un radical cyclopropyle, cyclobutyle, cyclopentyle ou cyclohexyle. Il s'agit de préférence du radical cyclopentyle.

Par radical alkoxy, il peut s'agir notamment des radicaux méthoxy, éthoxy, propyloxy, butyloxy et par radical alkylthio, il peut s'agir notamment des radicaux méthylthio, éthylthio, propylthio ou butylthio.

Par radical alcényle, il peut s'agir d'un radical vinyle, propényle, isopropényle, allyle, 2-méthyl allyle, butényle ou isobutényle. Il s'agit de préférence du radical vinyle ou propényle.

Par radical alcynyle, il peut s'agir du radical éthynyle, propynyle, propargyle, butynyle ou isobutynyle. Il s'agit de préférence du radical éthynyle ou propynyle.

Lorsque $R_{16}$ représente un atome d'halogène, il peut s'agir d'un atome de brome, de chlore, de fluor ou d'iode ; il s'agit de préférence d'un atome de brome.

Lorsque X représente un groupement arylène, il s'agit de préférence du radical phénylène.

Lorsque X représente un groupement arylènoxy, il s'agit de préférence du radical phénylènoxy.

Lorsque Y représente une chaîne aliphatique linéaire ou ramifiée, saturée ou insaturée, il peut s'agir du radical méthylène, éthylène, propylène, isopropylène, butylène, isobutylène, ou tert-butylène, n-pentylène, n-hexylène, 2-méthyl pentylène, 2,3-diméthyl butylène, n-heptylène, 2-méthyl hexylène, 2,2-diméthyl pentylène, 3,3-diméthyl pentylène, 3-éthylpentylène, n-octylène, 2,2-diméthyl hexylène, 3,3-diméthyl hexylène, 3-méthyl 3-éthyl pentylène, nonylène, 2,4-diméthyl heptylène, n-décylène, n-undécylène, n-dodécylène, n-tridécylène, n-tétradécylène, n-pentadécylène, n-hexadécylène, n-heptadécylène ou n-octadécylène, de préférence n-pentylène, n-hexylène, n-heptylène, n-octylène ou n-nonylène.

Il peut s'agir également des radicaux vinylène, isopropénylène, allylène, 2-méthyl allylène, isobuténylène, buténylène, penténylène, hexénylène, hepténylène ou octénylène, de préférence hexénylène.

Il peut s'agir également des radicaux éthynylène, propynylène, propargénylène, butynylène, isobutynylène, pentynylène, hexynylène, heptynylène ou octynylène, de préférence propynylène, hexynylène ou octynylène.

Il peut s'agir d'une chaîne interrompue par un atome d'oxygène par exemple d'un radical oxapolyméthylène, de préférence le radical oxy diéthylène.

Lorsque Z représente un radical alkyle linéaire ou ramifié, il peut s'agir des radicaux indiqués ci-dessus, de préférence propyle, butyle, n-pentyle.

Lorsque Z représente un groupement arylalkyle, le radical alkyle peut être l'un des radicaux définis ci-dessus, notamment un radical méthyle, éthyle, propyle, isopropyle, butyle, isobutyle, n-pentyle ou n-hexyle, il s'agit de préférence du radical méthyle ou éthyle.

Par groupement aryle qui peut être compris dans un groupement arylalkyle, on entend :

- un radical monocyclique carbocyclique, par exemple le radical phényle,

- un radical monocyclique hétérocyclique, par exemple les radicaux thiényle, furyle, pyrannyle, pyrrolyle, imidazolyle, pyrazolyle, pyridyle, pyrazinyle, pyrimidinyle, pyridazinyle, thiazolyle, oxazolyle, furazannyle, thiazolyle, triazolyle, tétrazolyle, ainsi que les isomères de position du ou des hétéroatomes que ces radicaux peuvent renfermer,

- un radical constitué de cycles condensés carbocycliques, par exemple le radical naphtyle,

- un radical constitué de cycles condensés hétérocycliques, par exemple le benzofurannyle, le benzothiényle, le benzimidazolyle, le benzothiazolyle, l'isobenzofurannyle, le chroményle, l'indolizinyle, l'isoindolyle, le 3H-indolyle, l'indolyle, l'indazolyle, le purinyle, le quinolizinyle, l'isoquinolyle, le quinolyle, le phtalazinyle, le naphtyridinyle, le quinoxalinyle, le quinazolinyle, le cinnolinyle, le ptéridinyle, l'indolinyle, l'isoindolinyle, l'imidazopyridyle, l'imidazopyrimidinyle ou encore les systèmes polycycliques condensés constitués de monocycliques hétérocycliques tels que définis, par exemple, ci-dessus comme par exemple le furo[2,3-b]pyrrole ou le thiéno[2,3-b]furanne.

Lorsque Z représente un groupement aryle ou arylalkyle, on peut citer comme exemple de tel radical aryle, en particulier, les radicaux phényle, furyle tel que 2-furyle, imidazolyle tel que 2-imidazolyle, pyridyle tel que 2-pyridyle, 3-pyridyle ou 4-pyridyle, pyrimidinyle tel que pyrimid-2-yle, thiazolyle tel que thiazol-2-yle, triazolyle tel que triazol-2-yle, tétrazolyle tel que tétrazol-2-yle, benzimidazolyle tel que benzimidazol-2-yle, benzothiazolyle tel que benzothiazol-2-yle, purinyle tel que purin-7-yle ou quinolyle tel que 4-quinolyle et comme exemple de radicaux arylalkyle, on peut encore citer en particulier les radicaux méthyle ou éthyle substitués par l'un des radicaux aryles ci-dessus.

L'expression éventuellement substitué appliquée aux radicaux alkyle que peuvent représenter $R'_{17}$ et Z, alcényle ou alcynyle que peut représenter $R'_{17}$ et aryle ou arylalkyle que peut représenter Z indique que ceux-ci peuvent éventuellement être substitués par un ou plusieurs radicaux identiques ou différents choisis parmi les radicaux indiqués précédemment et notamment,

- halogène : fluor, chlore, brome, iode,
- amino, alkylamino tel que méthylamino ou éthylamino, dialkylamino tel que diméthylamino, diéthylamino, méthyléthylamino,
- hydroxyle,
- carboxy libre, estérifié tel que alkoxy carbonyle par exemple méthoxy carbonyle ou éthoxycarbonyle, ou salifié par exemple par un atome de sodium ou de potassium,
- alkyle ayant de 1 à 8 atomes de carbone tel que méthyle, éthyle, propyle, isopropyle, butyle, isobutyle, tert-butyle, éventuellement substitué par un ou plusieurs atomes d'halogène, par exemple le fluor tel que le trifluorométhyle,
- oxo, cyano, nitro,
- acyle tel que formyle, acétyle, propionyle, butyryle, benzoyle,
- acyloxy tel que acétoxy, radical de formule $-O-CO-(CH_2)_n-COOH$ dans lequel n = 1 à 5,
- alkoxy tel que méthoxy, éthoxy, propyloxy, isopropyloxy, butyloxy,
- alkylthio tel que méthylthio, éthylthio, propylthio, isopropylthio, butylthio,
- alcényle tel que vinyle, propényle,
- alcynyle tel que éthynyle, propynyle,
- aryle tel que phényle, furyle, thiényle.

Comme exemple de tels radicaux substitués, on peut citer par exemple un radical alkyle susbtitué par un ou plusieurs atomes d'halogène, par exemple le fluor, tel que le radical trifluoroéthyle, trifluorobutyle, pentafluoropropyle, pentafluorobutyle, pentafluoropentyle, heptafluorobutyle ou nonafluorobutyle, un radical alkyle substitué par un radical aryle, par exemple le radical phényle, lui-même substitué par un ou plusieurs atomes d'halogène et/ou radicaux alkyle substitués par un ou plusieurs atomes d'halogène, tel que radical pentafluorobenzyle, pentafluorophényléthyle, pentafluorophénylpropyle, 4-trifluorométhyle 2,3,5,6-tétrafluorobenzyle.

On peut également citer par exemple un radical aryle substitué par un ou plusieurs atomes d'halogène par exemple le chlore tel que le 2,3,5,6-tétrachloro 4-pyridyle, par un groupement amino tel que le 4,6-diamino 2-pyrimidinyle, par un hydroxyle tel que le 2,6-dihydroxy 4-pyrimidinyle, par un carboxyle estérifié par exemple un méthoxycarbonyle tel que le 2-méthoxycarbonyl phényle, par un alkyle par exemple un méthyle tel que le N-méthyl imidazolyle, le N-méthyl triazolyle ou le N-méthyl tétrazolyle, éventuellement substitué par un halogène par exemple le fluor tel que le 7-(trifluorométhyl) 4-quinolyle, par un groupe oxo et un radical alkyle par exemple un radical méthyle tel que le 1,3-diméthyl 2,6-dioxopurin-7-yle.

L'invention s'étend naturellement aux sels des composés de formule (I), comme par exemple les sels formés, lorsque les composés de formule (I) comportent une fonction amino, avec les acides chlorhydrique, bromhydrique, nitrique, sulfurique, phosphorique, acétique, formique, propionique, benzoïque, maléique, fumarique, succinique, tartrique, citrique, oxalique, glyoxylique, aspartique, alcanesulfoniques tels que les acides méthane ou éthanesulfoniques, arènesulfoniques, tels que les acides benzène ou paratoluène sulfoniques et arylcarboxyliques, ou lorsque les composés de formule (I) comportent une fonction acide, avec les sels des métaux alcalins ou alcaline terreux ou d'ammonium éventuellement substitué.

Parmi les composés de formule (I) préférés de l'invention, on peut citer notamment les composés pour lesquels $R_3$ et $R_{16}$ représentent un atome d'hydrogène.

Parmi les composés de formule (I) préférés de l'invention, on peut citer particulièrement les composés pour lesquels m = 1 et ceux pour lesquels m = 2.

Parmi les composés de l'invention, on peut citer notamment les composés de formule (I), pour lesquels $R_{17}$ est un radical hydroxyle et $R'_{17}$ est un atome d'hydrogène ou un radical méthyle.

Parmi les composés de l'invention, on peut citer particuliérement les composés de formule (I), pour lesquels X représente un radical méthylène et Y est une chaîne linéaire saturée renfermant de 7 à 9 atomes de carbone, ceux pour lesquels X représente un radical phénylène et Y représente une chaîne linéaire saturée ou insaturée renfermant de 3 à 8 atomes de carbone, étant entendu que, lorsque la chaîne est insaturée, elle comporte un groupement vinylène ou éthynylène lié directement au radical phénylène et ceux pour lesquels X représente un radical phénylènoxy et Y représente une chaîne linéaire saturée renfermant de 4 à 7 atomes de carbone, éventuellement interrompue par un atome d'oxygène. Lorsque X représente un radical phénylènoxy, Y renferme de préférence 5 ou 6 atomes de carbone.

Parmi les composés de l'invention, on peut citer spécialement les composés de formule (I) pour lesquels Z représente un radical alkyle linéaire renfermant de 1 à 5 atomes de carbone, un radical trifluoroalkyle renfermant de 2 à 4 atomes de carbone, un radical pentafluoroalkyle renfermant 4 ou 5 atomes de carbone, un radical nonafluoroalkyle renfermant de 1 à 4 atomes de carbone,

- un radical comprenant un radical phényle substitué choisi parmi :

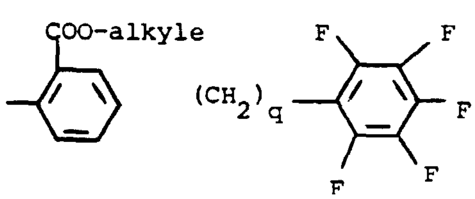
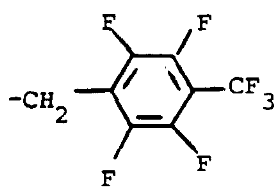
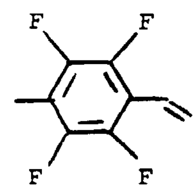

dans lequel alkyle représente un radical alkyle renfermant de 1 à 4 atomes de carbone et q représente les valeurs 1, 2 ou 3.

- un radical comprenant un hétérocycle à 5 chaînons choisi parmi :

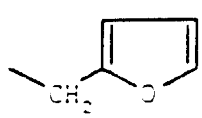
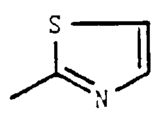
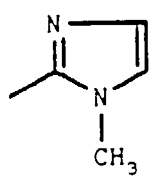
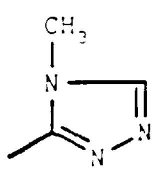
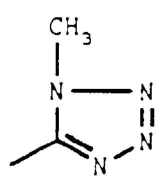

- un radical comprenant un hétérocycle à 6 chaînons choisi parmi :

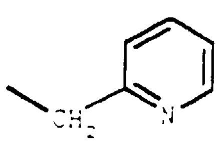
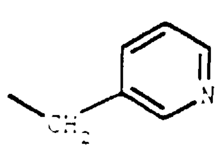
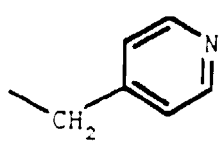
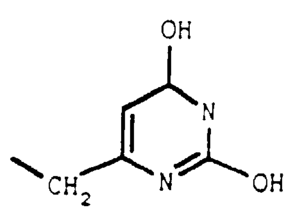

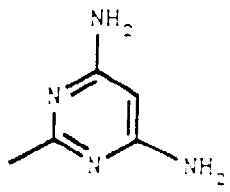

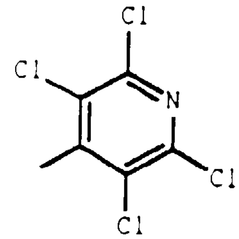

- un radical comprenant un hétérocycle à deux cycles condensés choisi parmi :

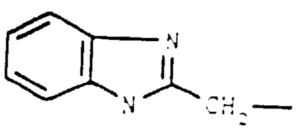

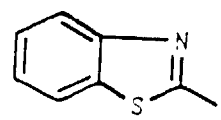

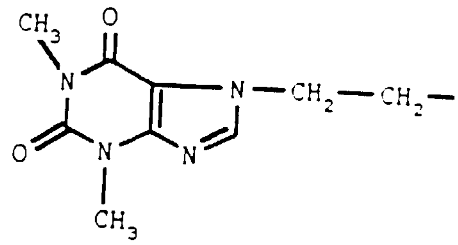

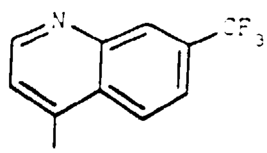

Parmi les valeurs préférées de Z, on peut citer plus particulièrement la valeur pentyle et la valeur pentafluoroalkyle et notamment les valeurs pentafluoropropyle, pentafluorobutyle ou pentafluoropentyle.

Parmi les composés préférés de l'invention, on peut donc citer les composés dont la préparation est donnée plus loin dans la partie expérimentale et plus particulièrement :

le 11béta-[8-[(2-pyridinylméthyl) thio] octyl] estra-1,3,5(10)-triène-3,17béta-diol,
le 11béta-[4-[3-[(1-méthyl 1H-imidazol-2-yl) thio] 1-propynyl] phényl] estra-1,3,5(10)-triène-3,17béta-diol,
le 11béta-[4-[5-[(2-furanylméthyl) thio] pentyloxy] phényl] estra-1,3,5(10)-triène-3,17béta-diol,
le 11béta-[4-[5-[(2-pyridinylméthyl) sulfinyl] pentyloxy] phényl] estra-1,3,5(10)-triène-3,17béta-diol,
le 11béta-[4-[5-[(3-pyridinylméthyl) sulfinyl] pentyloxy] phényl] estra-1,3,5(10)-triène-3,17béta-diol,
le 11béta-[4-[6-[(4,4,5,5,5-pentafluoro pentyl) sulfinyl] hexyloxy] phényl] estra-1,3,5(10)-triène-3,17béta-diol,
le 11béta-[4-[5-[(4,4,5,5,5-pentafluoro pentyl) sulfonyl] pentyloxy] phényl] estra-1,3,5(10)-triène-3,17béta-diol,
le 11béta-[4-[5-(pentylsulfonyl] pentyloxy] phényl] estra-1,3,5(10)-triène-3,17béta-diol,
le 11béta-[4-[5-[(4,4,5,5,5-pentafluoro pentyl) sulfonyl] pentyloxy] phényl] 17alpha-méthyl estra-1,3,5(10)-triène-3,17béta-diol.

L'invention a également pour objet un procédé de préparation des composés de formule (I) caractérisé en ce que l'on soumet un composé de formule (II) :

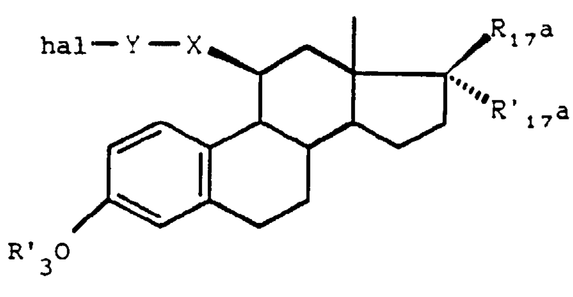

(II)

dans laquelle :

- $R_{17a}$ et $R'_{17a}$ ont les significations indiquées précédemment pour $R_{17}$ et $R'_{17}$ et dans lesquelles les éventuelles fonctions réactives sont éventuellement protégées,
- X et Y ont la même signification que précédemment,
- $R'_3$ représente un atome d'hydrogène ou un groupement protecteur de la fonction hydroxyle,
- hal représente un atome d'halogène ou un groupement pseudohalogène tel qu'un tosylate,

à l'action d'un mercaptan de formule (III) :

$$Za\text{-}SH \qquad (III)$$

ou un sel de celui-ci, dans laquelle Za a la signification indiquée précédemment pour Z dans lequel les éventuelles fonctions réactives sont éventuellement protégées, puis, le cas échéant, à l'action d'un agent d'élimination des groupements protecteurs, pour obtenir le composé de formule (IA) correspondant au produit de formule (I) dans laquelle m = 0, produit de formule (IA) que l'on soumet, si désiré et si nécessaire, à l'une quelconque des réactions suivantes, dans un ordre quelconque :

- réduction de la fonction cétone que peuvent représenter ensemble $R_{17}$ et $R'_{17}$,
- addition sur la fonction cétone que peut représenter $R_{17}$ et $R'_{17}$ d'un complexe métallique de formule (IV) :

$$M\text{-}R'_{17a} \qquad (IV)$$

  dans laquelle M représente un atome métallique et $R'_{17a}$ a la même signification que précédemment, étant entendu qu'il ne s'agit pas d'un atome d'hydrogène,
- transformation de la fonction cétone que peut représenter ensemble $R_{17}$ et $R'_{17}$ en fonction oxime, en groupement hydrazono ou en groupement méthylène,
- acylation sélective en position 17 lorsque $R_{17}$ est un hydroxyle,
- halogénation ou alkylation en position 16,
- alkylation ou acylation du radical hydroxyle en position 3,
- réduction partielle ou totale du groupement éthynylène, lorsque Y représente une chaîne insaturée,
- oxydation du soufre en sulfoxyde ou en sulfone,
- salification éventuelle par un acide ou une base.

Les composés de formule (IA) correspondant aux composés de formule (I) dans laquelle m = 0, sont obtenus en faisant réagir un composé de formule (II), dans laquelle hal est par exemple un atome de chlore, un atome de brome ou un tosylate, $OR_3$ est un hydroxyle éventuellement protégé par exemple par un radical acétyle, tertiobutyle ou tétrahydropyrannyle et $R_{17a}$, $R'_{17a}$ ont les fonctions réactives éventuellement protégées par des méthodes usuelles, avec un composé de formule (III), de préférence sous forme de sel obtenu par addition par exemple de carbonate de sodium, de soude ou de méthylate ou d'éthylate de sodium, la réaction étant effectuée dans un solvant neutre par exemple le méthanol, la diméthylformamide, l'hexaméthylphosphorotriamide (HMPT) ou l'acétonitrile ($CH_3CN$), seul ou en mélange, en opérant, par exemple à environ 50°C ou au reflux, si nécessaire en présence d'un réactif tel que l'iodure de sodium, puis le cas échéant en soumettant le composé obtenu à l'action d'un agent de déprotection des fonctions réactives.

Selon les valeurs du groupement protecteur $R'_3$, les produits de formule ($I_A$) peuvent représenter des produits de formule (I).

Les groupements protecteurs que l'on peut utiliser pour protéger les fonctions réactives, telles que par exemple les fonctions amino ou hydroxyle, sont choisis parmi les groupements usuels de la chimie organique et plus particulièrement de la chimie des peptides. On trouvera une liste non exhaustive de ces groupements ainsi que les méthodes d'élimination correspondantes dans le brevet français FR 2 499 995 dont le contenu est incorporé dans la présente demande à titre de référence. On peut citer par exemple les radicaux tétrahydropyrannyle ou trityle.

Dans un mode de réalisation préférée de l'invention :

- on utilise les composés de formule (II) comprenant une chaîne en 11béta terminée par un halogène ou un pseudohalogène tel que par exemple le groupe $-O-So_2-\phi-CH_3$ dans lequel $\Phi$ représente un radical phénylène, comme illustré dans les exemples ci-après, ainsi que les composés de formule (III) cités ci-après dans la partie expérimentale. D'une manière non exhaustive, on peut citer l'ester méthylique de l'acide 2-mercapto benzoïque, le furfurylmercaptan, le 2-thiazoline-2-thiol, le 2-mercapto thiazole, le 2-mercapto 1-méthyl imidazole, le 1-méthyl 5-mercapto- 1,3,4-triazole, le 1-méthyl 5-mercapto 1,2,3,4-tétrazole, le 2-pyridineméthanethiol, le 4, 6-diamino pyrimidine-3-thiol, le 2,3,5,6-tétrachloro pyridine-4-thiol, le 2-mercapto benzothiazole, le 7-trifluorométhyl 4-quinolinethiol et le 4,4,5,5,5-pentafluoropentanethiol.

Lorsque le composé de formule (IA) comporte des fonctions réactives protégées, on obtient le composé déprotégé correspondant par action d'agents usuels, par exemple un agent de saponification tel que la potasse en milieu alcoolique ou un agent d'hydrolyse tel que l'acide chlorhydrique. Les méthodes de déprotection utilisables sont décrites également dans le brevet français FR 2 499 995.

Lorsque le composé de formule (IA) comporte une fonction cétone en 17, on obtient :

- le composé 17béta hydroxylé correspondant, par exemple par action d'un agent de réduction tel que le borohydrure de sodium dans un solvant neutre tel que le méthanol,
- le composé correspondant comportant un radical $R'_{17}$ représentant un radical alkyle, alcényle ou alcynyle, éventuellement substitué est obtenu, par addition d'un composé (IV), tel que par exemple un complexe du lithium, selon le procédé décrit dans le brevet européen EP 57115,
- le composé correspondant comportant une fonction oxime en position 17, par exemple par action de l'hydroxylamine sous forme de sel tel que le chlorhydrate, en présence d'une base faible au sein d'un alcool à la température de reflux,
- le composé correspondant comportant une fonction hydrazono en position 17, par exemple par action d'un dérivé de l'hydrazine et notamment l'hydrate d'hydrazine en présence d'un acide tel que l'acide paratoluènesulfonique,
- Le composé correspondant comportant une fonction méthylène en position 17, en utilisant selone une réaction de Wittig, par exemple le bromure de méthyltriphénylphsophonium en milieu basique.

Lorsque le composé de formule (IA) comporte une fonction hydroxyle en 3 ou en 17, on obtient le composé 3 ou 17 bêta acyloxylé correspondant, par action d'un agent d'acylation sélective par exemple l'anhydride acétique dans la pyridine.

Lorsque le composé de formule (IA) comporte un groupement éthynylène, on obtient le composé correspondant ayant une liaison vinylique ou le composé saturé correspondant par action d'un agent de réduction partielle ou totale, soit à l'aide d'hydrogène en présence de palladium, sur charbon actif ou sur sulfate de baryum et éventuellement en présente d'une base telle que la pyridine ou la quinoléine, dans le cas d'une réduction partielle, soit à l'aide d'hydroxyde de palladium seul dans la cas d'une réduction totale.

On peut obtenir les composés comportant un radical alkyle en position 16, par exemple par action d'un halogénure d'alkyle, tel que l'iodure de méthyle ou d'éthyle en présence d'un complexe du lithium.

On peut obtenir le composé comportant un halogène en position 16, par exemple par action du brome en milieu acide notamment au sein de l'acide acétique ou encore par action d'un agent d'halogénation tel que le N-bromo ou N-chloro succinimide ou le N-bromo ou N-chloro acétamide ou encore l'hypochlorite de terbutyle.

On peut soumettre les composés de formule (IA) à un agent de sulfoxydation, par exemple le métapériodate de sodium ou l'acide métachloroperbenzoïque pour obtenir les composés de formule (I) dans laquelle m = 1, ou à un agent de sulfonation, par exemple l'acide perphtalique ou l'acide métachloroperbenzoïque pour obtenir les composés de formule (I) dans laquelle m = 2.

L'invention concerne également un procédé de préparation des composés de formule (I), caractérisé en ce que l'on soumet un composé de formule (V) :

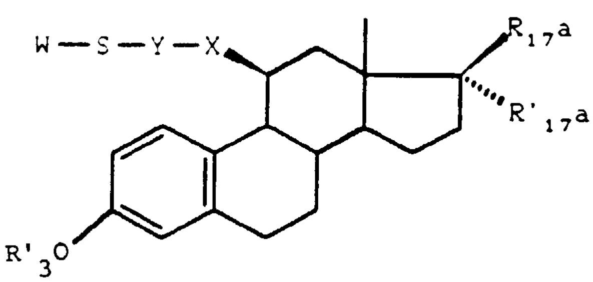

(V)

dans laquelle :

- $R_{17a}$ et $R'_{17a}$ ont les significations indiquées précédemment pour $R_{17}$ et $R'_{17}$ dans lesquelles les éventuelles fonctions réactives sont éventuellement protégées,

- X et Y ont la même signification que précédemment,
- $R'_3$ représente un atome d'hydrogène ou un groupement protecteur de la fonction hydroxyle,
- W représente un atome d'hydrogène ou un radical acyle -COR dans lequel R représente un radical alkyle qui comporte de 1 à 5 atomes de carbone,

à l'action d'un composé de formule (VI) :

$$\text{Za-hal'} \qquad \text{(VI)}$$

ou un sel de celui-ci, dans laquelle Za a la signification indiquée précédemment pour Z dans lequel les éventuelles fonctions réactives sont éventuellement protégées et hal' représente un groupement réactif tel qu'un halogène ou un pseudohalogène tel qu'un groupement mésylate ou tosylate, en présence d'une base, puis le cas échéant à l'action d'un agent d'élimination des groupements protecteurs, pour obtenir le composé de formule (IA) telle que définie ci-dessus et que, si désiré et si nécessaire, on soumet à l'une quelconque des réactions indiquées pour le composé de formule (IA) ci-dessus.

Selon cette variante de procédé de l'invention, les composés de formule (IA) correspondants aux composés de formule (I) dans laquelle m = 0 sont obtenus en faisant réagir un composé de formule (V) dans laquelle lorsque W est un groupement acyle il peut s'agir par exemple d'un radical acétyle, propionyle, butyryle ; $OR'_3$ est un hydroxyle éventuellement protégé, par exemple, par un radical acétyle, tertiobutyle ou tétrahydropyrannyle ; les fonctions réactives que comportent $R_{17a}$ et $R'_{17a}$ sont éventuellement protégées par des méthodes usuelles, avec un composé de formule (VI) ou un sel de celui-ci, par exemple un chlorhydrate, la réaction s'effectuant dans un solvant tel que le méthanol ou le diméthylformamide que l'on chauffe éventuellement par exemple à environ 50°C ou au reflux et en présence d'une base telle que le méthylate de sodium ou la soude concentrée.

Dans un mode de réalisation préférée de l'invention :

- on utilise les composés de formule (V) comportant une chaîne en 11béta terminée par un groupement thiol ou thioacétyle ainsi que les composés de formule (VI) cités ci-après dans la partie expérimentale.

D'une manière non exhaustive, on peut encore citer comme composés de formule (VI) soit le 3-chlorométhylpyridine, le 4-chlorométhylpyridine, le 6-(chlorométhyl) uracile, le 2-chlorométhylbenzimidazole, le 7-(2-chloroéthyl) théophylline et les sels d'addition de ceux-ci tel que le chlorhydrate, soit l'iodopentane, soit le mésylate, le tosylate ou un halogénure de pentafluoropentyle ou de trifluorobutyle de préférence le 4,4,5,5,5-pentafluoroiodopentane, le 4,4,4-trifluoroiodobutane comme illustré dans les exemples ci-après.

Le 4,4,5,5,5-pentafluoro iodopentane dont la préparation est décrite plus loin dans la partie expérimentale est obtenu par ioduration de l'alcool correspondant 4,4,5,5,5-fluoropentanol, produit intermédiaire que l'on peut préparer par exemple, en soumettant à une réaction d'hydrogénation, en présence d'un catalyseur tel que par exemple le nickel de Raney dans le méthanol ou l'oxyde de platine dans l'éthanol, le produit partiellement insaturé correspondant 4,4,5,5,5-pentafluoro-2-pentèn-1-ol qui est décrit par T. Kitazume et al., J. Am. Chem. Soc 1985, 107, 5186-5191.

L'alcool intermédiaire saturé ci-dessus peut être également préparé selon un procédé décrit par N, O. Brace, Journal of Fluorine Chemistry 20 (1982), 313-327, en partant de iodopentafluoroéthane que l'on condense sous pression à de l'alcool allylique, en présence d'un catalyseur tel que par exemple l'azaisobutyronitrile (AIBN), le nickel de Raney dans l'éthanol, le tétra kis (triphénylphosphino) palladium dans l'hexane ou l'étain et l'acétate d'argent dans le méthanol pour obtenir le 2-iodo-4,4,5,5,5-pentafluoropentanol que l'on soumet à une réaction d'hydrogénation par exemple par l'hydrogène en présence de palladium à 10 % de magnésie dans le méthanol ou de nickel de Raney dans l'éthanol ou par réaction avec l'hydrure de tributyl étain en présence d'azoisobutyronitrile.

Les composés de formule (IA) obtenus selon la variante du procédé sont ensuite, si nécessaire, soumis à l'une des réactions indiquées ci-dessus pour obtenir les composés de formule (I).

Les composés de formule (I) présentent d'intéressantes propriétés pharmacologiques. L'étude des produits sur les récepteurs hormonaux a permis de mettre en évidence qu'ils possèdent en particulier une remarquable activité anti-oestrogène et des propriétés antiprolifératives, comme le montrent les résultats des tests donnés plus loin. Ils possèdent également une activité anti-nidatoire.

Ces propriétés rendent les composés de formule (I) utilisables dans le contrôle de fertilité, par exemple dans certaines formes de stérilité anovulatoire, dans le contrôle des naissances, par exemple comme contraceptif et notamment comme pilule post-coïtale ainsi que dans le traitement de carcinomes hormonaux-dépendants, comme par exemple les carcinomes mammaires et leurs métastases et dans le traitement des tumeurs bénignes du sein.

L'invention a donc pour objet les produits de formule (I) à titre de médicament.

Parmi les médicaments de l'invention on peut citer particulièrement les composés décrits dans la partie expérimentale et en particulier les produits des exemples. Parmi ces produits, l'invention a particulièrement pour objet à titre de médicament, les composés de formule (I) suivants :

le 11béta-[8-[(2-pyridinylméthyl) thio] octyl] estra1,3,5(10)-triène-3,17béta-diol,
le 11béta-[4-[3-[(1-méthyl lH-imidazol-2-yl) thio] 1-propynyl] phényl] estra-1,3,5(10)-triène-3,17béta-diol,
le 11béta-[4-[5-[(2-furanylméthyl) thio] pentyloxy] phényl] estra-1,3,5(10)-triène-3,17béta-diol,
le 11béta-[4-[5-[(2-pyridinylméthyl) sulfinyl] pentyloxy] phényl] estra-1,3,5(10)-triène-3,17béta-diol,
le 11béta-[4-[5-[(3-pyridinylméthyl) sulfinyl] pentyloxy] phényl] estra-1,3,5(10)-triène-3,17béta-diol,
le 11béta-[4-[6-[(4,4,5,5,5-pentafluoro pentyl) sulfinyl] hexyloxy] phényl] estra-1,3,5(10)-triène-3,17béta-diol,
le 11béta-[4-[5-[(4,4,5,5,5-pentafluoro pentyl) sulfonyl] pentyloxy] phényl] estra-1,3,5(10)-triène-3,17béta-diol,
le 11béta-[4-[5-(pentylsulfonyl] pentyloxy] phényl] estra-1,3,5(10)-triène-3,17béta-diol,
le 11béta-[4-[5-[(4,4,5,5,5-pentafluoro pentyl) sulfonyl] pentyloxy] phényl] 17alpha-méthyl estra-1,3,5(10)-triène-3,17béta-diol.

La posologie utile varie en fonction de l'affection à traiter et de la voie d'administration ; elle peut varier par exemple de 1 à 100 mg par jour chez l'adulte par voie orale.

L'invention s'étend aux compositions pharmaceutiques renfermant comme principe actif au moins l'un des médicaments tel que défini ci-dessus.

Les composés de formule (I) sont utilisés par voie digestive, parentérale ou locale, par exemple par voie percutanée. Ils peuvent être prescrits sous forme de comprimés simples ou dragéifiés, de gélules, de granulés, de suppositoires, d'ovules, de préparations injectables, de pommades, de crèmes, de gels, de microsphères, d'implants, de patchs, lesquels sont préparés selon les méthodes usuelles.

Le ou les principes actifs peuvent y être incorporés à des excipients habituellement employés dans ces compositions parmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Certains des intermédiaires de formule (II) ou (V) sont des produits nouveaux et l'invention a donc également pour objet les composés de formules (II) et (V) telles que définies précédemment.

Les composés intermédiaires nouveaux de formule (II) tels que définis précédemment sont préparés selon un mode opératoire dont un exemple est donné plus loin.

D'une manière générale, les composés de formule (II) peuvent être préparés selon le procédé suivant :

- soit l'on soumet un composé de formule (VII) :

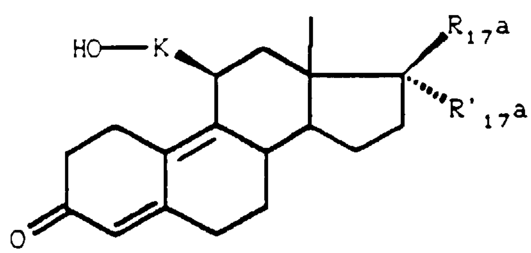

(VII)

dans laquelle $R_{17a}$ et $R'_{17a}$ ont la signification donnée précédemment, K représente une chaîne aliphatique renfermant de 2 à 19 atomes de carbone liée au noyau stéroïde par un radical méthylène ou un groupement arylène,

a) à l'action d'un agent d'activation de la fonction alcool, par exemple le chlorure de tosyle dans la pyridine, ou à un agent de formation d'un halogénure par exemple le tétrachlorure ou le tétrabromure de carbone en présence de triphénylphosphine dans un solvant neutre, lorsque K est une chaîne aliphatique,
b) à l'action d'un composé halogéné de formule (VIII) :

Br-Y-hal (VIII)

dans laquelle Y a la signification indiquée ci-dessus et hal représente un halogène, en présence d'une base, lorsque K est un groupement arylène,

puis, dans les deux cas, à un agent d'aromatisation tel que l'hydroxyde de palladium sur magnésie dans du méthanol ou le mélange bromure d'acétyle - anhydride acétique, pour obtenir respectivement les composés de formule (II) dans laquelle X représente un radical méthylène et les composés de formule (II) dans laquelle X représente est un groupement arylènoxy,

- soit l'on soumet le composé de formule (VII) telle que définie ci-dessus d'abord à la réaction d'aromatisation ci-dessus puis à la réaction a) ou b) telle qu'indiquée ci-dessus,
- soit l'on soumet un composé de formule (IX) :

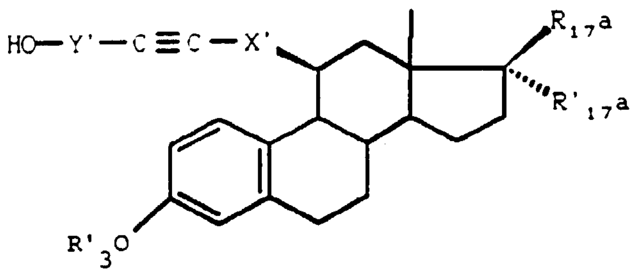

(IX)

dans laquelle R'$_3$, $R_{17a}$ et $R'_{17a}$ ont la signification donnée précédemment, X' représente un groupement arylène et Y' représente Y tel que défini ci-dessus comportant 2 atomes de carbone de moins au niveau de la liaison avec le groupement arylène que représente X', à l'action d'un agent d'activation de la fonction alcool, par exemple le tétrachlorure de carbone en présence de triphénylphosphine dans un solvant neutre ou le chlorure de tosyle dans la pyridine, pour obtenir les composés de formule (II) dans laquelle X est un groupement arylène et Y est lié directement à X par un groupement éthynylène, produits que l'on peut soumettre, si désiré, à une réaction partielle ou totale de la triple liaison.

Certains des produits de formule (VII) nécessaires à la mise en oeuvre du procédé sont des produits connus. Leur préparation est décrite à la préparation des produits de l'exemple 43 et de l'exemple 50, dans la demande de brevet européen EP 384842 dont le contenu est incorporé dans la présente demande à titre de référence. Les produits de formule (VII) nouveaux peuvent être préparés par analogie, par exemple, en suivant la méthode décrite dans la demande de brevet ci-dessus.

Les produits de formule (IX) nécessaires à la mise en oeuvre du procédé sont des produits que l'on prépare, selon des méthodes connues, à partir de produits de formule (X) :

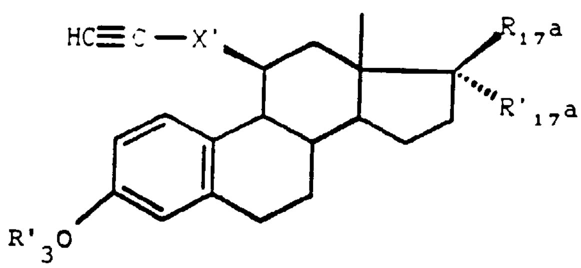

(X)

dans laquelle R'$_3$, $R_{17a}$, $R'_{17a}$ et X' ont la signification donnée précédemment.

A partir des produits de formule (X) on obtient les produits de formule (IX) en faisant agir par exemple soit le paraformaldéhyde en présence de chlorure de butyle et de lithium dans un solvant neutre, lorsque Y' représente un radical méthylène, soit un halogénure de formule Rp-O-Y'-$Hal_1$ dans laquelle $Hal_1$ est un halogène et Rp un groupement protecteur de la fonction alcool en présence d'une base forte, lorsque Y' représente une chaîne aliphatique ayant au moins 2 atomes de carbone. Des exemples d'une telle préparation sont donnés ci-après dans la partie expérimentale.

On peut également obtenir directement certains produits de formule (II) dans laquelle hal représente un halogène en faisant réagir un halogénure de formule $Hal_2$-Y'-$Hal_1$ dans laquelle $Hal_1$ et $Hal_2$ représentent respectivement un halogène, en présence d'une base forte.

Des produits de formule (X) sont décrits par exemple dans la demande de brevet européen EP 384842 et d'autres produits de formule (X) peuvent être préparés par analogie, par exemple en suivant la méthode décrite à la préparation de l'exemple 31 de la demande ci-dessus.

Les composés intermédiaires nouveaux de formule (V) tels que définis précédemment sont préparés selon un

mode opératoire dont un exemple est donné plus loin.

D'une manière générale, les composés de formule (V) peuvent être préparés selon le procédé suivant :

On soumet un composé de formule (II') correspondant au composé de formule (II) définie ci-dessus dans laquelle hal représente un atome de chlore ou de brome,

- soit à l'action d'un agent d'ioduration, par exemple l'iodure de sodium dans un solvant neutre tel que l'acétone ou la méthyléthylcétone que l'on chauffe éventuellement au reflux, puis à l'action d'un sel d'acide thiocarboxylique de formule (XI) :

R-CO-SH (XI)

dans laquelle R a la signification donnée précédemment, pour obtenir le composé de formule (V) dans lequel W est un radical acyle,
- soit à l'action du composé de formule (XI) indiquée ci-dessus pour obtenir le composé disulfure de formule (XII) :

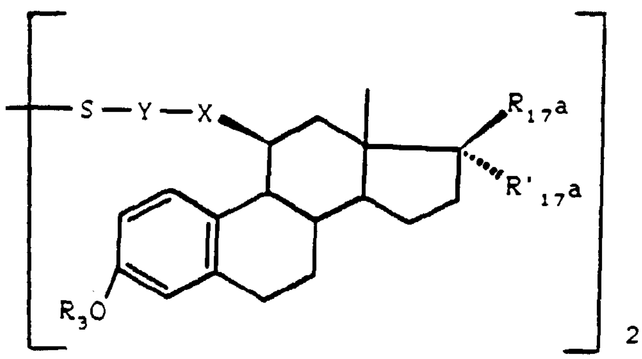

(XII)

dans laquelle $R_3$, X, Y, $R_{17a}$ et $R'_{17a}$ ont la signification donnée précédemment, que l'on soumet à un agent de réduction du disulfure, par exemple la tri-n-butylphosphine dans un solvant neutre tel qu'un solvant organique aqueux pour obtenir le composé de formule (V) dans laquelle W est un atome d'hydrogène.

En plus des exemples suivants qui illustrent l'invention sans toutefois la limiter, les produits de formule (I) dans laquelle $R_{17}$ représente un hydroxyle, $R'_{17}$ représente un atome d'hydrogène, m a les valeurs 0, 1 ou 2 et X, Y, Z ont les valeurs données dans le tableau ci-dessous constituent des produits pouvant être obtenus dans le cadre de la présente invention :

| - X | -Y | - Z |
|---|---|---|
| - Φ | $-C{\equiv}C-(CH_2)_4$ | $-(CH_2)_3-CF_2-CF_3$ |
| - Φ | $-(CH_2)_6$ | $-(CH_2)_3-CF_2-CF_3$ |
| - ΦO | $-(CH_2)_6$ | $-CH_2-CF_2-CF_2-CF_3$ |
| - ΦO | $-(CH_2)_5$ | $-CH_2-CF_2-CF_2-CF_3$ |
| - ΦO | $-(CH_2)_7$ | $-CH_2-CF_2-CF_2-CF_3$ |

dans lequel Φ a la signification indiquée précédemment.

**EXEMPLE 1 : 11béta-[(8-[(2-pyridinylméthyl) thio] octyl] estra-1,3,5(10)-triène-3,17béta-diol**

Stade A : 17béta-acétyloxy 11béta-[8-(acétyloxy) octyl] estra-4,9-dièn-3-one

A une solution de 1,4 g de 17béta-(acétyloxy) 11béta-(8-hydroxy octyl)-estra-4,9-dièn-3-one (obtenu par un procédé analogue, au stade D de la préparation de l'exemple 50 de la demande de brevet européen EP 384 842) dans 10 $cm^3$ de pyridine on ajoute 3,6 $cm^3$ d'anhydride acétique et 95 mg de 4-(diméthylamino) pyridine. On agite la solution pendant une heure à température ambiante, ajoute 5 $cm^3$ d'eau et 5 $cm^3$ de méthanol, agite dix minutes à 0°/+5°C, coule dans une solution aqueuse saturée de chlorure d'ammonium, extrait avec de l'acétate d'éthyle, lave avec une

solution saturée de chlorure de sodium, sèche et évapore à sec sous pression réduite après avoir entraîné la pyridine avec du toluène. On obtient 1,61 g de produit brut que l'on chromatographie sur silice éluant acétate d'éthyle-cyclohexane 3-7. On recueille ainsi 1,28 g du produit recherché.

| | |
|---|---|
| **Spectre I.R. :** | dans $CHCl_3$ |
| C=O | 1735 $cm^{-1}$ |
| diènone | 1660-1605 $cm^{-1}$ |

Stade B : Diacétate de 11béta-[8-(acétyloxy) octyl] estra-1,3,5(10)-triène-3,17béta-diol

On dissout 1,273 g du produit obtenu au stade précédent dans 13 $cm^3$ de chlorure de méthylène avec 1,3 $cm^3$ d'anhydride acétique et 0,65 $cm^3$ de bromure d'acétyle, on agite 10 minutes à 0°C puis 1 heure 30 à température ambiante, refroidit et ajoute 2 gouttes d'eau et 3 $cm^3$ de méthanol. On coule dans une solution saturée de bicarbonate de sodium, extrait avec du chlorure de méthylène, lave à l'eau, sèche et évapore à sec sous pression réduite. On obtient 1,387 g du produit recherché.

**Spectre I.R. :**

| | |
|---|---|
| C=O | 1740 - 1730 $cm^{-1}$ |
| Aromatique | 1610 - 1590 - 1500 $cm^{-1}$ |

Stade C : 11béta-(8-hydroxy octyl) estra-1,3,5(10)-triène-3,17béta-diol

A une solution de 1,377 g du produit obtenu au stade précédent dans 42 $cm^3$ de méthanol, on ajoute lentement 20 $cm^3$ de soude 2 N et agite pendant 2 heures 30 à température ambiante, ajoute 21,5 $cm^3$ d'acide chlorhydrique 2 N puis coule dans une solution saturée de bicarbonate de sodium, extrait avec de l'acétate d'éthyle, lave à l'eau saturée de chlorure de sodium, sèche et évapore à sec sous pression réduite. On obtient 1,119 g du produit recherché utilisé tel quel pour le stade suivant.

Stade D : 11béta- (8-bromo octyl) estra-1,3,5(10)-triène-3,17béta-diol

A une solution de 1,107 g du produit obtenu au stade précédent dans 32,5 $cm^3$ d'acétonitrile avec 8,1 $cm^3$ de tétrahydrofuranne et 2,561 g de tétrabromure de carbone à 40°C, on ajoute goutte à goutte une solution de 2,88 g de triphényl phosphine dans 8,1 $cm^3$ de chlorure de méthylène. On agite une heure à température ambiante et évapore à sec sous pression réduite. On obtient 6,05 g de produit que l'on chromatographie sur silice (éluant acétate d'éthyle-cyclohexane 3/7). On obtient 283 mg du produit attendu.

**Spectre I.R. :**

| | |
|---|---|
| OH | 3605 $cm^{-1}$ |
| Aromatique | 1615-1600-1590-1500 $cm^{-1}$ |

Stade E : 11béta-[8-[(2-pyridinylméthyl) thio] octyl] estra-1,3,5(10)-triène-3,17béta-diol

On agite 5 minutes un mélange de 125 mg de 2-pyridine méthane thiol et 0,98 $cm^3$ d'une solution à 51,3 mg/$cm^3$ de méthylate de sodium dans le méthanol puis ajoute à température ambiante une solution de 155 mg du produit obtenu au stade précédent dans 4 $cm^3$ de méthanol, ajoute 60 mg d'iodure de sodium et agite le tout pendant une heure au reflux. On refroidit, coule dans l'eau, extrait avec de l'acétate d'éthyle, lave à l'eau saturée de chlorure de sodium et évapore à sec sous pression réduite. On recueille 224 mg de produit que l'on chromatographie sur silice (éluant chlorure de méthylène-méthanol 97,5/2,5 puis à nouveau avec acétate d'éthyle-cyclohexane 40/60).

**Spectre I.R. :**

| | |
|---|---|
| OH | 3604 $cm^{-1}$ |
| | 1616 |
| Aromatiques | 1598 |
| | 1584 |
| hétérocycles | 1571 |
| | 1498 |

**EXEMPLE 2 : 11béta-[4-[3-[(1-méthyl 1H-imidazol-2-yl) thio] 1-propynyl] phényl] estra-1,3,5(10)-triène-3,17béta-diol**

Stade A : 3,17béta-bis-(tétrahydro 2H-2-pyrannyloxy) 11béta-(4-éthynyl phényl) estra-1,3,5(10)-triène

On agite dans une cuve à ultrasons un mélange de : 0,1 $cm^3$ de chlorobutane, 14 mg de lithium poudre, 540 mg de 3-[4-[3,17béta-bis-(tétrahydro 2H-2-pyrannyloxy) estra-1,3,5(10)-trièn-11béta-yl] phényl] prop-2-yn-1-ol (obtenu au stade D de la préparation B de l'exemple 31 de la demande de brevet EP 384842) et 2 $cm^3$ de tétrahydrofuranne on agite pendant trente minutes à 30/35°C et ajoute 29 mg de paraformaldéhyde agite encore quelques minutes à 30/35°C, dilue à l'eau, acidifie avec du phosphate monosodique, extrait avec du chlorure de méthylène, sèche et évapore à sec sous pression réduite. On obtient 613 mg de produit que l'on chromatographie sur silice (éluant cyclohexane-acétate d'éthyle 1/1) on recueille ainsi 400 mg de produit recherché (F = 214°C).

| **Spectre I.R. :** | |
|---|---|
| C≡CH | absence |
| OH | 3609 $cm^{-1}$ |
| aromatique | 1607 - 1572 - 1555 - 1492 $cm^{-1}$ |

Stade B : 3,17béta-bis-(tétrahydro 2H-2-pyrannyloxy) 11béta-[4-(3-chloro-1-propynyl) phényl] estra-1,3,5(10)-triène

On agite 1 heure à température ambiante et 2 heures au reflux une solution renfermant : 570 mg du produit obtenu au stade précédent, 14 $cm^3$ de tétrachlorure de carbone, 2 $cm^3$ de tétrahydrofuranne et 4 $cm^3$ d'acétonitrile et enfin, par portions, 530 mg de triphényl phosphine. On ajoute 5 g de silice et évapore à sec sous pression réduite. On chromatographie le résidu obtenu sur silice (éluant cyclohexane-acétate d'éthyle 8-2). On recueille 370 mg de produit recherché.

| **Spectre I.R. :** | $CHCl_3$ sur Nicolet |
|---|---|
| OH | 3598 $cm^{-1}$ |
| C≡C | 2268 (f) - 2220 $cm^{-1}$ |
| aromatique | 1606 - 1560 - 1531 - 1506 $cm^{-1}$ |

Stade C : 3,17béta-bis-(tétrahydro 2H-2-pyrannyloxy) 11béta-[4-[3-[(1-méthyl 1H-imidazol-2-yl) thio] 1-propynyl] phényl] estra-1,3,5(10)-triène

A une solution refroidie à 0°C renfermant 90 mg de 2-mercapto-1-méthyl imidazole et 2 $cm^3$ de tétrahydrofuranne, on ajoute 38 mg d'hydrure de sodium en dispersion à 50 % dans l'huile. On agite 1 heure à 0°C. On ajoute une solution de 2 g du produit obtenu au stade précédent dans 2 $cm^3$ de tétrahydrofuranne. On laisse la température revenir à l'ambiante et agite encore 3 heures. On verse le mélange réactionnel sur une solution aqueuse de chlorure d'ammonium, extrait avec de l'acétate d'éthyle, sèche et évapore à sec sous pression réduite. Le produit obtenu est chromatographié sur silice (éluant acétate d'éthyle-cyclohexane 2/8). On obtient 180 mg du produit recherché.

| **Spectre I.R. :** | $CHCl_3$ sur Nicolet |
|---|---|
| OH | 3600 $cm^{-1}$ |
| C≡C | 2218 |
| aromatique + hétérocycle | 1608 - 1582 - 1506 $cm^{-1}$ complexe |

Stade D : 11béta(4(3-(méthyl lH-imidazol-2-yl) thio) 1-propynyl) phényl) estra-1,3,5(10)-triène-3,17béta-diol

On agite 2 heures à température ambiante un mélange contenant 143 mg du produit obtenu au stade précédent, 2 $cm^3$ d'éthanol et 2 $cm^3$ d'acide chlorhydrique 2 N. On concentre à volume réduit, extrait à l'acétate d'éthyle et évapore à sec sous pression réduite. Le résidu est chromatographié sur silice(éluant cyclohexane-acétate d'éthyle 1/1). On recueille 96 mg du produit recherché.

| **Spectre I.R. :** | $CHCl_3$ sur Nicolet |
|---|---|
| OH | 3608 $cm^{-1}$+ associé |
| C≡C | 2210 $cm^{-1}$ |
| aromatique hétérocycle | 1583 - 1554 - 1505 $cm^{-1}$ |

**EXEMPLE 3 : 11béta-[4-[5-[(2-furanylméthyl) thio] pentyloxy] phényl] estra-1,3,5(10)-triène-3,17béta-diol**

Stade A : 11béta-[4-[(5-chloro-pentyl) oxy] phényl] estra-4,9-diène-3,17-dione

On chauffe pendant 6 heures sous agitation une solution renfermant : 28,2 g de 11béta-(4-hydroxyphényl) estra-4,9-diène-3,17-dione (obtenu à la préparation de l'exemple 43 de la demande de brevet EP 384842), 450 $cm^3$ d'acétone, 45 $cm^3$ de soude 2 N et 18,5 $cm^3$ de 1-bromo-5-chloropentane. On évapore l'acétone et reprend le résidu avec 200 $cm^3$ de chlorure de méthylène, lave à l'eau, sèche et concentre jusqu'à un volume de 100 $cm^3$, ajoute 10 $cm^3$ d'éther isopropylique et concentre jusqu'à début de cristallisation, essore, sèche et recueille 26,3 g de produit que l'on chromatographie sur silice (éluant essence G-acétate d'éthyle 1/1). On obtient 4,2 g du produit attendu (F= 220°C).

| | |
|---|---|
| **Spectre I.R. :** | $CHCl_3$ |
| 17 céto | 1735 $cm^{-1}$ |
| 3 céto | 1658 $cm^{-1}$ |
| C=C et aromatique | 1609 - 1580 - 1509 $cm^{-1}$ |

Stade B : 3-acétyloxy 11béta-[4-[(5-chloro-pentyl) oxy] phényl] estra-1,3,5(10)-trièn-17-one

A une solution refroidie à +4°C comprenant 30 g du produit, obtenu au stade précédent, dans 300 $cm^3$ de chlorure de méthylène, on ajoute 30 $cm^3$ d'anhydride acétique et 15 $cm^3$ de bromure d'acétyle. On laisse revenir à température ambiante, agite une heure, ajoute en refroidissant, 30 $cm^3$ de méthanol et 500 $cm^3$ de solution saturée de bicarbonate de sodium. On agite 45 minutes à température ambiante, décante, 5 lave à l'eau, sèche puis évapore à sec.
On obtient 37 g de produit utilisé tel quel pour le stade suivant.

Stade C : 3-hydroxy 11béta-[4-[(5-chloro-pentyl) oxy] phényl] estra-1,3,5(10)-trièn-17-one

On agite pendant 40 minutes à température ambiante une solution renfermant 37 g du produit obtenu au stade précédent, 200 $cm^3$ de tétrahydrofuranne et 64 $cm^3$ de soude 2 N. On ajoute 64 $cm^3$ d'acide chlorhydrique 2 N et évapore les solvants sous pression réduite. On extrait avec du chlorure de méthylène, lave à l'eau, sèche et amène à sec sous pression réduite. On obtient 32 g de produit recherché que l'on utilise tel quel pour le stade suivant.

Stade D : 11béta-[4-[(5-chloro-pentyl) oxy] phényl] estra-1,3,5(10)-triène-3,17béta-diol

A une solution renfermant 32 g du produit obtenu au stade précédent dans 150 $cm^3$ de tétrahydrofuranne on ajoute 150 $cm^3$ de méthanol et, à 0+5°C en 10 minutes, 2,56 g d'hydrure de bore et de sodium (à 95 %). On agite 1 heure à 0+5°C et ajoute 10 $cm^3$ d'acétone, distille les solvants, reprend le résidu avec du chlorure de méthylène, lave à l'eau, sèche et évapore à sec On obtient 34 g de produit que l'on chromatographie sur silice (éluant toluène-acétate d'éthyle 8/2). On recueille 15,15 g du produit attendu (F=165°C recristallisé de l'acétate d'éthyle).

| | |
|---|---|
| **Spectre I.R. :** | (Nujol) |
| absorption région | NH/OH |
| aromatique | 1618 - 1608 - 1582 - 1512 - 1492 $cm^{-1}$ |

Stade E : 11béta-[4-[5-[(2-furanylméthyl) thio] pentyloxy] phényl] estra-1,3,5(10)-triène-3,17béta-diol

On agite au reflux pendant 4 heures un mélange renfermant 0,15 $cm^3$ de furfuryl mercaptan (à 90-95 %), 81 mg de méthylate de sodium, 90 mg d'iodure de sodium, 4 $cm^3$ de méthanol et 234 mg du produit obtenu au stade précédent. On distille le méthanol sous pression réduite, reprend avec de l'acétate d'éthyle, lave à l'eau, sèche et distille à sec sous pression réduite. On obtient 340 mg de produit que l'on chromatographie sur silice (éluant essence G-acétate d'éthyle 6-4). On recueille 260 mg de produit que l'on chromatographie sur Lichrosorb RP18 (éluant méthanol-eau 9-1). On obtient ainsi 140 mg du produit recherché.

| | |
|---|---|
| **Spectre I.R. :** | ($CHCl_3$) |
| OH | 3602 $cm^{-1}$ |
| aromatique + système conjugué | 1610 - 1581 - 1512 - 1504 $cm^{-1}$ |

**EXEMPLE 4 : 11béta-[4-[5-[(2-pyridinylméthyl) thio] pentyloxy] phényl] estra-1,3,5(10)-triène-3,17béta-diol**

On chauffe 3 heures au reflux, une solution renfermant 0,17 $cm^3$ de 2-pyridine méthanethiol, 81 mg de méthylate de sodium, 234 mg du produit obtenu au stade D de l'exemple 3 et 4 $cm^3$ de méthanol. On distille le méthanol, reprend avec de l'acétate d'éthyle, lave à l'eau, sèche et amène à sec sous pression réduite. On recueille 360 mg de résidu que l'on chromatographie sur silice (éluant acétate d'éthyle-essence G 8/2) et obtient ainsi 280 mg du produit attendu.

| | |
|---|---|
| **Spectre I.R. :** | ($CHCl_3$) |
| OH | 3605 $cm^1$ |
| aromatique | 1610 - 1581 - 1511 $cm^1$ |
| pyridine | 1594 - 1571 $cm^1$ |

**EXEMPLE 5 : 11béta-[4-[5-[(2-pyridinylméthyl) sulfinyl] pentyloxy] phényl] estra-1,3,5(1O)-triène-3,17béta-diol**

A une solution de 172 mg du produit obtenu à l'exemple 4 dans 12 $cm^3$ de méthanol, on ajoute 3,7 $cm^3$ d'une solution 0,1 M de métapériodate de sodium dans l'eau. On agite 1 heure 30 minutes et ajoute 3,4 $cm^3$ de méthanol et 0,6 $cm^3$ de la solution de métapériodate, après 3 heures 30 d'agitation le milieu réactionnel est coulé dans l'eau, extrait avec de l'acétate d'éthyle, lavé à l'eau salée et évaporé à sec sous pression réduite. On obtient 176 mg de résidu que l'on chromatographie sur silice par deux fois (éluant chlorure de méthylène-méthanol (92,5-7,5)). On recueille 107 mg de produit recherché.

| | |
|---|---|
| **Spectre I.R. :** | ($CHCl_3$ sur Nicolet) |
| OH | 3606 $cm^1$ |
| hétérocycle et aromatique | 1610 - 1596 - 1583 - 1572 - 1512 $cm^1$ |
| sulfoxyde | 1031 $cm^1$ |

**EXEMPLE 6 : 11béta-[4-[5-[(3-pyridinylméthyl) thio] pentyloxy] phényl] ostra-1,3,5(10)-trièno-3,17béta-diol**

Stade A : (11béta, 11'béta) 11,11'-[dithiobis-[5,1-pentoxy-(4,1-phénylène)]] di-estra-1,3,5(10)-triène-3,17béta-diol

On agite au reflux pendant 17 heures 1,9 g de 11béta-[4-[(5-chloro-pentyl) oxy] phényl] estra-1,3,5(10)-triéne-3,17béta-diol obtenu au stade D de l'exemple 3 et 910 mg de thioacétate de potassium dans 20 $cm^3$ d'éthanol. On ajoute 910 mg de thioacétate de potassium et agite 10 heures au reflux. On distille l'éthanol, reprend le résidu avec de l'acétate d'éthyle, lave à l'eau salée, sèche et évapore à sec sous pression réduite. On obtient 2,1 g de résidu que l'on chromatographie sur silice (éluant acétate d'éthyle-essence G 6/4) on recueille ainsi 1,72 g du produit recherché.

| | |
|---|---|
| **Spectre I.R. :** | (nujol) |
| absorption région | NH/OH |
| aromatique | 1609 - 1580 - 1510 $cm^{-1}$ |

Stade B : 11béta-[4-[5-[(3-pyridinylméthyl) thio] pentyloxy] phényl] estra-1,3,5(10)-triène-3,17béta-diol

a) réduction du disulfure :

Dans une solution de 5 $cm^3$ de méthanol à 10 % d'eau et 2 $cm^3$ de tétrahydrofuranne, préalablement dégazée, on introduit 465 mg du produit obtenu au stade précédent et 0,25 $cm^3$ de tributylphosphine. On agite 2 heures à température ambiante, extrait avec de l'acétate d'éthyle, lave à l'eau, sèche et évapore à sec sous pression réduite. On obtient 640 mg de mercaptan.

b) alkylation :

On dissout le mercaptan obtenu dans 5 $cm^3$ de méthanol et ajoute 160 mg de méthylate de sodium et 342 mg de chlorhydrate de (3-chlorométhyl) pyridine à 96 %.

On agite cette suspension pendant 55 minutes au reflux, ramène à température ambiante, acidifie avec 2 $cm^3$ d'acide chlorhydrique 2 N, alcalinise avec du bicarbonate de sodium, extrait avec de l'acétate d'éthyle, lave à l'eau salée, sèche et évapore à sec sous pression réduite. On recueille 650 mg de produit que l'on chromatographie sur silice (éluant acétate d'éthyle-essence G 8/2). On obtient 370 mg du produit recherché.

| | |
|---|---|
| **Spectre I.R. :** | $CHCl_3$ |
| OH | 3607 $cm^{-1}$ |
| aromatique | 1610 - 1580 - 1512 $cm^{-1}$ |

**EXEMPLE 7 : 11béta-[4-[5-[(3-pyridinylméthyl) sulfinyl] pentyloxy] phényl]estra-1,3,5(10)-triène-3,17béta-diol**

On opère comme à l'exemple 5 à partir de 177 mg du produit obtenu à l'exemple 6. On obtient 185 mg de produit brut auxquels on joint 24 mg d'une précédente venue et chromatographie le tout sur silice par deux fois (éluant chlorure de méthylène-méthanol 9/1). On recueille 138 mg du produit recherché.

| | |
|---|---|
| **Spectre I.R. :** | ($CHCl_3$ sur Nicolet) |
| OH | 3606 $cm^{-1}$ + absorption générale |
| aromatique + hétéroaromatique | 1610 - 1580 - 1512 $cm^{-1}$ |
| sulfoxyde | ≃1030 - 1040 $cm^{-1}$ |

**Exemple 8 : 11béta-[4-[6-[(4,4,5,5,5-pentafluoro pentyl) thio] hexyloxy] phényl] estra-1,3,5(10)-triène-3,17béta-diol**

Stade A : 11béta-[4-[(6-chloro hexyl) oxy] phényl] estra-4,9-diène-3,17-dione.

On agite 5 heures au reflux 362 mg de 11béta-(4-hydroxyphényl) estra-4,9-diène-3,17-dione (obtenu à la préparation de l'exemple 43 de la demande de brevet EP 384842), 5 $cm^3$ d'acétone, 1,5 $cm^3$ de 6-bromo-chloro hexane et 138 mg de carbonate de potassium. On ajoute alors 1 $cm^3$ de 6-bromo-chloro hexane et agite pendant 16 heures en laissant revenir à température ambiante. On acidifie avec de l'acide chlorhydrique 2 N, extrait avec de l'acétate d'éthyle, lave à l'eau, sèche et évapore à sec sous pression réduite. On recueille 3,48 g de produit que l'on chromatographie sur silice (éluant acétate d'éthyle-essence G 6/4). On recristallise le résidu obtenu dans un mélange chlorure de méthylène-éther isopropylique et obtient ainsi 290 mg du produit recherché (F = 221°C).

Stade B : 3-hydroxy 11béta-[4-[(6-chloro hexyl) oxy] phényl] estra-1,3,5(10)-trièn-17-one

On opère comme aux stades B et C de l'exemple 3 à partir de 481 mg de produit obtenu au stade A ci-dessus en utilisant 0,5 $cm^3$ d'anhydride acétique et 0,25 $cm^3$ de bromure d'acétyle. On obtient 483 mg du produit recherché.

Stade C : 11béta-[4-[(6-chloro hexyl) oxy] phényl] estra-1,3,5(10)-triène-3,17béta-diol

On opère comme au stade D de l'exemple 3 à partir de 465 mg du produit obtenu ci-dessus, en utilisant 60 mg d'hydrure de bore et de sodium. On obtient, après chromatographie sur silice (éluant essence G-acétate d'éthyle 6/4) et recristallisation dans le chlorure de méthylène, 300 mg du produit recherché (F = 176°C).

Stade D : 11béta-[4-[(6-iodo hexyl) oxy] phényl] estra-1,3,5(10)-triène-3,17béta-diol

A une solution de 310 mg de produit obtenu au stade C dans 6 $cm^3$ de méthyléthylcétone on ajoute 150 mg d'iodure de sodium et agite 24 heures au reflux. On ajoute 100 mg d'iodure de sodium, agite 2 heures au reflux et 16 heures en laissant revenir à température ambiante. On reprend à l'acétate d'éthyle, lave, sèche et évapore à sec sous pression réduite. On recueille 470 mg de produit attendu utilisé tel quel pour le stade suivant.

Stade E : 11béta-[4-[[6-(thioacétyl) hexyl] oxy] phényl] estra-1,3,5(10)-triène-3,17béta-diol

A une solution de 470 mg de produit obtenu au stade D dans 6 $cm^3$ d'éthanol, on ajoute 150 mg de thioacétate de potassium et agite 1 heure 50 minutes à 50 °C. On distille l'éthanol, reprend avec de l'acétate d'éthyle, lave à l'eau, sèche et évapore à sec sous pression réduite. On obtient 400 mg de résidu que l'on chromatographie sur silice (éluant acétate d'éthyle-essence G 6/4 puis 8/2). On recueille 265 mg de produit recherché (F # 90°C).

| | |
|---|---|
| **Spectre I.R. :** | $CHCl_3$ sur Nicolet |
| OH | 3602 $cm^{-1}$ |
| C=O | 1686 $cm^{-1}$ |
| aromatique | 1610 - 1581 - 1512 $cm^{-1}$ |

Stade F : 11béta-[4-[6-[(4,4,5,5,5-pentafluoro pentyl) thio] hexyloxy] phényl] estra-1,3,5(10)-triène-3,17béta-diol

A une solution de 200 mg du produit obtenu ci-dessus et 127 mg de 4,4,5,5,5-pentafluoroiodopentane (dont la préparation est donnée ci-après) dans 5 $cm^3$ de méthanol, on ajoute 0,1 $cm^3$ de lessive de soude. On agite pendant 1 heure à 50°C puis évapore le solvant sous pression réduite. On reprend le résidu avec du chlorure de méthylène et de l'acide chlorhydrique 2 N. On extrait avec du chlorure de méthylène, lave à l'eau, sèche et évapore à sec sous pression réduite. Le résidu obtenu est chromatographié sur silice (éluant essence G-acétate d'éthyle 65/35). On obtient 149 mg du produit recherché.

| | |
|---|---|
| **Spectre I.R. :** | $CHCl_3$ |
| OH | 3600 $cm^{-1}$ |
| aromatique | 1620 - 1580 - 1512 $cm^{-1}$ |

**PREPARATION DE L'EXEMPLE 8 : 4,4,5,5,5-pentafluoro iodopentane**

Stade A : 4,4,5,5,5-pentafluoro pentanol

On dissout 10 g de 4,4,5,5,5-pentafluoro-2-pentèn-1-ol (obtenu selon J. Am. Chem. Soc., 107, (1985), 5186-5191 : T. Kitazume et N. Ishikawa) dans 100 $cm^3$ de méthanol, et hydrogène en présence de 0,5 g de nickel de Raney. On filtre le catalyseur, lave avec de l'éthanol et après distillation à pression ordinaire, on recueille l'alcool attendu Eb : 133°C.
$n_D^{23}$ : 1,3305

Stade B : 4,4,5,5,5-pentafluoro iodopentane

A une solution de 2,65 g de triphénylphosphine et 0,69 g d'imidazole dans 20 $cm^3$ de chlorure de méthylène, on ajoute 2,54 g d'iode et, en maintenant la température inférieure à 25°C, une solution de 1,78 g du produit obtenu au stade A dans 3 $cm^3$ de chlorure de méthylène. On agite 3 heures, filtre, distille le chlorure de méthylène et reprend le résidu avec du pentane, plusieurs fois. Après distillation du pentane, on obtient 3,45 g de résidu contenant le produit attendu que l'on peut distiller (Eb. 42-45°C sous 40 mb).
$n_D^{23}$ : 1,4054

**EXEMPLE 9 : 11béta-[4-[6-[(4,4,5,5,5-pentafluoro pentyl) sulfinyl] hexyloxy] phényl] estra-1,3,5 (10)-triène-3,17bétadiol**

A une solution de 110 mg du produit obtenu à l'exemple 8 dans 5,5 $cm^3$ de méthanol, on ajoute 0,61 $cm^3$ d'une solution 0,5 M de métapériodate de sodium dans l'eau. On agite 1 heure à température ambiante et évapore le méthanol sous pression réduite. Le résidu est repris par de l'acide chlorhydrique 2 N, extrait avec du chlorure de méthylène. Après séchage et évaporation à sec du solvant, le résidu est chromatographié sur silice (éluant chlorure de méthylène-méthanol 95/5). On obtient 96 mg du produit recherché.

| | |
|---|---|
| **Spectre I.R. :** | $CHCl_3$ |
| OH | 3605 $cm^{-1}$ + associés |
| aromatique | 1610 - 1580 - 1512 $cm^{-1}$ |
| sulfoxyde | 1031 $cm^{-1}$ |

**EXEMPLE 9 a : 11béta-[4-[6-[(4,4,5,5,5-pentafluoro pentyl) sulfonyl] hexyloxy] phényl] estra-1,3,5 (10)-triène-3,17bétadiol**

A une solution de 150 mg du produit obtenu à l'exemple 8 dans 2 $cm^3$ de chlorure de méthylène, on ajoute 150 mg d'acide perphtalique à 70 %. On agite 1 heure 15 minutes puis on ajoute une solution aqueuse de thiosulfate de sodium et une solution aqueuse de bicarbonate de sodium. On extrait au chlorure de méthylène, sèche et évapore à sec sous pression réduite. Le résidu obtenu est chromatographié sur silice (éluant acétate d'éthyle-essence G 6/4). On obtient 130 mg de produit recherché.

| | |
|---|---|
| **Spectre I.R. :** | $CHCl_3$ |
| OH | 3603 $cm^{-1}$ + associés |
| aromatique | 1622 - 1610 - 1570 - 1511 - 1505 $cm^{-1}$ |

sulfone 1305 - 1132 $cm^{-1}$

**EXEMPLES 10 A 13 :**

En suivant la méthode de préparation de l'exemple 1, en partant du produit obtenu au stade D de l'exemple 1, on prépare le composé de l'exemple 10 ; en suivant la méthode de préparation de l'exemple 1 mais en partant des diacétates des composés de formule (II) correspondants, composés tels que définis ci-dessus dans lesquels Hal représente un atome de chlore (exemple 11) ou un groupement tosylate (exemples 12 et 13), en utilisant pour chaque exemple le mercaptan de formule (III) dans laquelle Za a la valeur donnée pour Z dans la table I, ou son sel de sodium, puis en saponifiant par la soude le diacétate obtenu pour obtenir le diol de formule (I) attendu, on prépare les composés des exemples 11 à 13.

Les composés des exemples 10 à 13 correspondant à des composés de formule (I) dans laquelle $R_{17}$ représente un hydroxyle, $R'_{17}$ représente un atome d'hydrogène, m a la valeur 0, X, Y et Z ont les valeurs données dans la table I ci-dessous.

Les spectres IR des produits des exemples 10 à 13 sont donnés dans la table I.

Les produits correspondants aux exemples 10, 12 et 13 de formule (I) dans laquelle m a la valeur 1 sont préparés en suivant la méthode de l'exemple 5.

Table I

| Exemples | X | Y | Z | IR$cm^{-1}$ |
|---|---|---|---|---|
| 10 | $CH_2$ | $(CH_2)_7$ | 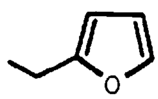 | 3602 (OH), 1609,1585,1501 |
| 12 | $CH_2$ | $(CH_2)_9$ | 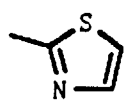 | 3602 (OH), 1615,1609,1583,1498 |
| 13 | $CH_2$ | $(CH_2)_9$ | 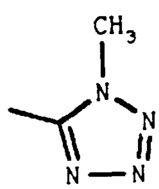 | 3603 (OH), 1609,1580,1500 |

**EXEMPLES 14 A 24 :**

En suivant la méthode de préparation de l'exemple 2, on prépare les composés des exemples 14 à 21 et en suivant la méthode de préparation de l'exemple 2 mais en partant des composés de formule (II) correspondants, on prépare les composés des exemples 22 à 24, composés de formule (I) dans laquelle $R_{17}$ représente un hydroxyle, $R'_{17}$ représente un atome d'hydrogène, m a la valeur 0, X, Y et Z ont les valeurs données dans la table II ci-dessous, en utilisant pour chaque exemple le mercaptan de formule (III) dans laquelle Za a la valeur donnée pour Z dans la table II.

Les spectres IR des produits des exemples 14 à 24 sont donnés dans la table II.

Les produits correspondants aux exemples 14 à 24 de formule (I) dans laquelle m a la valeur 1 sont préparés en suivant la méthode de l'exemple 5.

Table II

| Exemples | X | Y | Z | IR $cm^{-1}$ |
|---|---|---|---|---|
| 14 | Φ | $C≡C-(CH_2)$ | 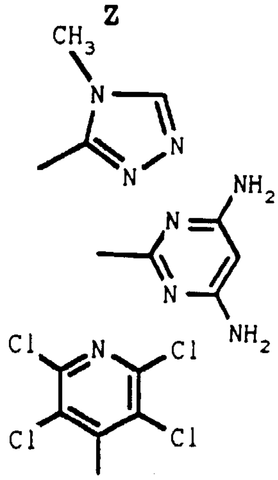 | OH,NH,2218 (C≡C), 1612,1575,1508,1499 |
| 15 | Φ | $C≡C-(CH_2)$ | | OH,NH 1614,1580,1543,1500 |
| 16 | Φ | $C≡C-(CH_2)$ | | 3601 (OH), 1605,1583,1555,1504 |
| 17 | Φ | $C≡C-(CH_2)$ | 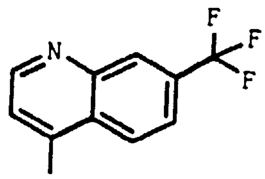 | 3601 (OH), 2212 (C≡C), 1609,1571,1505 |
| 18 | Φ | $C≡C-(CH_2)$ | 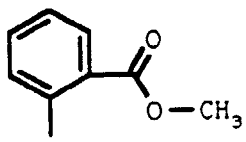 | 3600,3330 (OH), 1705 (C=O),1617,1603 1585,1575,1560,1505 |
| 19 | Φ | $C≡C-(CH_2)$ | 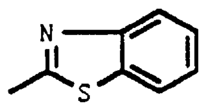 | 3601 (OH), 2212 (C≡C),1610 11584,1555,1505 |
| 20 | Φ | $C≡C-(CH_2)$ | 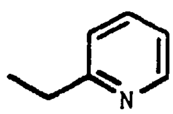 | 3602 (OH),1607, 1593,1571,1554,1505 |
| 21 | Φ | $C≡C-(CH_2)$ | 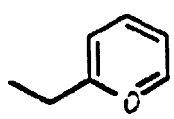 | OH,NH,1601,1504 |
| 22 | Φ | $C≡C-(CH_2)_4$ | 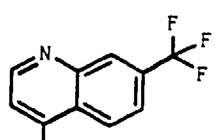 | 3604 (OH), 1613,1571,1505 |
| 23 | Φ | $C≡C-(CH_2)_6$ | 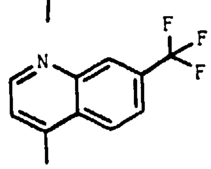 | 3605 (OH), 1615, 1610,1571,1505 |

| | | | | |
|---|---|---|---|---|
| 24 | Φ | C≡C-$(CH_2)_6$ | 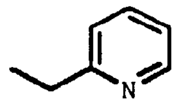 | 3600 (OH),1609,1596, 1571,1505 |

**EXEMPLES 25 A 29 :**

En suivant la méthode de préparation de l'exemple 6, on prépare les composés des exemples 25 à 27, en suivant la méthode de préparation de l'exemple 8 on prépare le composé de l'exemple 28, composés de formule (I) dans laquelle $R_{17}$ représente un hydroxyle, $R'_{17}$ représente un atome d'hydrogène, m a la valeur 0, X, Y et Z ont les valeurs données dans la table III ci-dessous, en utilisant pour chaque exemple le composé de formule (VI) dans laquelle Za a la valeur donnée pour Z dans la table III.

On prépare les composés correspondants aux exemples 25 à 28 de formule (I) dans laquelle m a la valeur 1 en suivant la méthode de l'exemple 5. L'exemple 29 est préparé selon cette méthode à partir du composé de l'exemple 25.

Les spectres IR des produits des exemples 25 à 29 sont donnés dans la table III.

**Table III**

| Exemples | X | Y | Z | IR $cm^{-1}$ |
|---|---|---|---|---|
| 25 | ΦO | $(CH_2)_5$ | 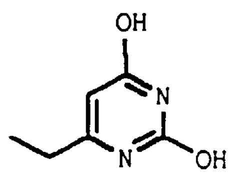 | OH,NH 1708,1660 (C=O), 1608,1578,1510 |
| 26 | ΦO | $(CH_2)_5$ | 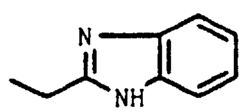 | OH,NH 1610,1580,1530, 1510 |
| 27 | ΦO | $(CH_2)_5$ | 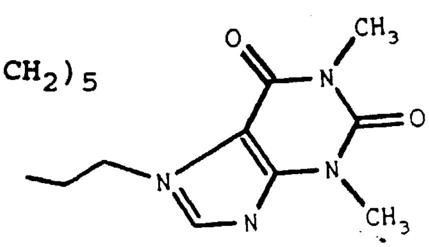 | 3608 (OH), 1705,1658 (C=O), 1609,1578,1551, |

| | | | | |
|---|---|---|---|---|
| | | | | 1512,1503 |
| 28 | ΦO | $(CH_2)_5$ | 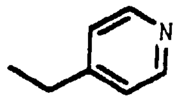 | 3606 (OH),<br>1609,1581,1565,1512<br>1609,1585,1501 |
| 29 | ΦO | $(CH_2)_5$ | 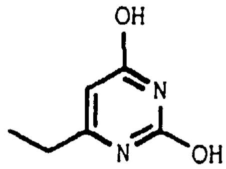 | OH,NH<br>1708,1660(C=O),1608,<br>1578,1510,1015 (SO) |

**EXEMPLE 30 : 11béta-[4-[5-[(4,4,5,5,5-pentafluoropentyl) thio] pentyloxy] phényl] estra-1,3,5 (10)-triène-3,17béta- diol**

En suivant la méthode de préparation de l'exemple 8, mais en faisant réagir au stade A le 5-bromochloropentane sur le 11béta-(4-hydroxy phényl) estra-4,9-diène-3,17-dione, on obtient le produit recherché.

| | |
|---|---|
| Spectre IR : | $CHCL_3$ sur Nicolet. |
| C=O | absence |
| OH : | 3600 $cm^{-1}$ |
| Aromatique : | 1625, 1613, 1570, 1511, 1500 $cm^{-1}$. |

**EXEMPLE 31 : 11béta-[4-[5-[(4,4,5,5,5-pentafluoropentyl) sulfinyl] pentyloxy] phényl] estra-1,3,5(10)-triéne-3,17bétadiol**

En suivant la méthode de préparation de l'exemple 9 et en partant du produit de l'exemple 30, on prépare le composé recherché.

| | |
|---|---|
| Spectre IR : | $CHCl_3$ sur Nicolet. |
| OH : | 3606 $cm^{-1}$ + Associé |
| Aromatique : | 1622, 1610, 1570, 1511, 1505 $cm^{-1}$ |
| Sulfoxyde : | 1030 $cm^{-1}$ |

**EXEMPLE 32 : 11béta-[4-[5-[(4,4,5,5,5-pentafluoropentyl) sulfonyl] pentyloxy] phényl] estra-1,3,5(10)-triène-3,17bétadiol**

En suivant la méthode de préparation de l'exemple 9a à partir de 225 mg du produit de l'exemple 30, on a obtenu 206 mg de produit attendu.

| | |
|---|---|
| Spectre IR : | $CHCl_3$ . |
| OH : | 3604 $cm^{-1}$ + Associés |
| Aromatique : | 1622 (ep), 1610, 1570, 1512 $cm^{-1}$ |
| $SO_2$ : | 1306, 1132 $cm^{-1}$ |

**EXEMPLE 33 : 11béta-[4-(5-(pentylthio) pentyloxy) phényl] estra-1,3,5(10)-triène-3,17béta- diol**

Dans une solution de 5cm$^3$ de méthanol à 10% d'eau et 2 cm$^3$ de tétrahydrofuranne, préalablement dégazée, on introduit 465 mg du produit obtenu au stade A de l'exemple 6 et 0,25 cm$^3$ de tributylphosphine. On agite 1 heure à température ambiante, ajoute 245 µl d'iodopentane et 0,3 cm$^3$ de soude concentrée. On agite 1 heure à 50°C, dilue à l'acétate d'éthyle, acidifie à l'aide d'acide chlorhydrique N, extrait à l'acétate d'éthyle, lave à l'eau salée, sèche et évapore le solvant. Après chromatographie du résidu sur silice (éluant : Essence G-acétate d'éthyle 70-30), on obtient 570 mg de produit attendu.

Spectre IR : $CHCl_3$ .
OH : 3600 $cm^{-1}$
Aromatique : 1610, 1582, 1511 $cm^{-1}$

**EXEMPLE 34 : 11béta-[4-[5-[[7-(trifluorométhyl) 4-quinoléinyl] thio] pentyloxy] phényl] estra-1,3,5(10)-triène-3,17béta- diol**

On mélange à 95°C pendant 43 heures 400 mg du dérivé chloré obtenu au stade D de l'exemple 3, 300 mg de 4-mercapto 7-trifluorométhyl quinoléine et 68 mg de méthylate de sodium. On évapore le solvant sous pression réduite, reprend le résidu au chlorure de méthylène à 5% de méthanol, lave la solution à l'acide chlorhydrique 2N, sèche et évapore le solvant. Après chromatographie sur silice (éluant : acétate d'éthyle-essence G-triéthylamine 70-30-1 puis acétate d'éthyle-triéthylamine 99-1), on recueille 391 mg de produit attendu que l'on purifie par une nouvelle chromatographie sur silice (éluant : chlorure de méthylène-méthanol 97-3).

Spectre IR : $CHCl_3$ .
OH : 3600 $cm^{-1}$
Aromatique : 1610, 1582, 1512, 1505 $cm^{-1}$
hétérocycle : 1571, 1328, 1287, 1135, 1068 $cm^{-1}$

**EXEMPLE 35 : 11bêta-[4-[4-[(4,4,5,5,5-pentafluoropentyl) thio] butyloxy] phényl] estra-1,3,5(10)-triène-3,17bêta- diol**

On opère comme au stade F de l'exemple 8 à partir de 262 mg de 4,4,5,5,5-pentafluoroiodopentane préparé à l'exemple 8 et 410 mg de thioacétate préparé ci-dessous. On obtient 335 mg de produit attendu.

Spectre IR : $CHCl_3$ .
OH : 3600 $cm^{-1}$
Aromatique : 1610, 1581, 1512 $cm^{-1}$
$C_2F_5$ : probable

**Préparation du 11bêta-[4-[[4-(acétylthio) butyl] oxy] phényl estra-1,3,5(10)-triéne 3,17bêta-diol utilisé à l'exemple 35.**

Stade A : 11bêta [4-[(4-chlorobutyl) oxy] phényl] estra-4,9-diène-3,17-dione.

On opère comme à l'exemple 8 stade A à partir de 11bêta-(4-hydroxyphényl) estra-4,9-diène-3,17-dione (obtenu à la préparation de l'exemple 43 de la demande de brevet EP 384842) et 1,04 $cm^3$ de 1-bromo 4-chlorobutane. On obtient 630 mg de produit attendu. F = 194°C.

Stade B : 3-hydroxy 11béta-[4-[(4-chlorobutyl) oxy] phényl] estra-1,3,5(10)-trièn-17-one.

On opère comme aux stades B et C de l'exemple 3 à partir de 1,35 mg de produit obtenu au stade A ci-dessus en utilisant 1,4 $cm^3$ d'anhydride acétique et 0,7 $cm^3$ de bromure d'acétyle. On obtient 1,48 g du produit recherché.

Stade C : 11béta-[4-[(46-chlorobutyl) oxy] phényl] estra-1,3,5(10)-triène-3,17béta-diol

On opère comme au stade D de l'exemple 3 à partir de 1,48 g du produit obtenu ci-dessus, en utilisant 60 mg d'hydrure de bore et de sodium. On obtient, après chromatographie sur silice (éluant essence G-acétate d'éthyle 6/4) et recristallisation dans l'éther isopropylique, 955 mg du produit recherché. F = 194°C.

Stade D : 11béta-[4-[(4-iodobutyl) oxy] phényl] estra-1,3,5(10)-triène-3,17béta-diol.

On opère comme au stade D de l'exemple 8 à partir de 890 mg de produit ci-dessus 600 mg de iodure de sodium et 10 $cm^3$ de méthyléthylcétone. On obtient 1,18 g de produit attendu.

Stade E : 11béta-[4-[[4-(thioacétyl) butyl] oxy] phényl] estra-1,3,5(10)-triène-3,17béta-diol.

On opère comme au stade E de l'exemple 8 à partir de 1,18 g du produit obtenu au stade D et 460 mg de thioacétate

de potassium. On obtient 850 mg de produit attendu. F # 90°C.

**<u>EXEMPLE 36</u> : 11béta-[4-[4-[(4,4,4-trifluoropentyl) thio] butyloxy] phényl] estra-1,3,5(10)-triène-3,17béta-diol.**

On opère comme au stade F de l'exemple 8 à partir de 317 mg de 4,4,4-trifluoro 1-iodobutane et 410 mg de thioacétate obtenu comme indiqué à la préparation 35. On obtient 335 mg de produit attendu.

Spectre I.R. : $CHCl_3$
OH : 3615 $cm^{-1}$
aromatique : 1610, 1579, 1512, 1501$cm^{-1}$

**<u>EXEMPLE 37</u> :11béta-[4-[5-[(4,4,4-trifluorobutyl) thio] pentyloxy] phényl] estra-1,3,5(10)-triène-3,17béta-diol.**

On opère comme au stade F de l'exemple 8 à partir de 393 mg de 4,4,4-trifluoro 1-iodobutane et 784 mg de thioacétate obtenu au cours de la préparation de l'exemple 28. On obtient 640 mg de produit attendu.

Spectre I.R. : $CHCl_3$
OH : 3601 $cm^{-1}$
aromatique : 1610, 1580, 1512 $cm^{-1}$

**<u>EXEMPLE 38</u> : 11béta-[4-[6-[(4,4,4-trifluorobutyl) thio] hexyloxy] phényl] estra-1,3,5(10)-triène-3,17béta-diol.**

On opère comme au stade F de l'exemple 8 à partir de 262 mg de 4,4,4-trifluoro 1-iodobutane et 523 mg de thioacétate obtenu à l'exemple 8 stade E. On obtient 380 mg de produit attendu.

Spectre I.R. : $CHCl_3$
OH : 3602 $cm^{-1}$
aromatique : 1610, 1580, 1512 $cm^{-1}$

**<u>EXEMPLE 39</u> : 11béta-[9-[(4,4,5,5,5-pentafluoropentyl) thio] nonyl] estra-1,3,5(10)-triène-3,17béta-diol.**

On dissout 446 mg de triphénylphosphine dans 2 cm3 de chlorure de méthylène, refroidit à 10°C, ajoute 116 mg d'imidazole, agite 15 minutes, ajoute 431 mg d'iode, agite 30 minutes, ajoute 0,21 $cm^3$ de 4,4,5,5,5-pentafluoro 2-penten 1-ol, agite 4 heures à température ambiante, lave la phase organique avec une solution aqueuse saturée en thiosulfate de sodium et obtient une solution chlorométhylénique de 4,4,5,5,5-pentafluoro iodopentane utilisé tel quel dans la suite de la synthèse. On opère comme indiqué à l'exemple 8 stade F, à partir de 618 mg de thioacétate préparé ci-dessous dans 7 $cm^3$ de méthanol et la solution chlorométhylénique de dérivé iodé préparée ci-dessus. On obtient 1,2 g de produit attendu.

Spectre I.R. : $CHCl_3$
OH : 3601 $cm^{-1}$
aromatique : 1609, 1585, 1501$cm^{-1}$
$CF_2$ -$CF_3$ : probable

**Préparation du 11bêta-[9-(acétylthio) nonyl] estra-1,3,5(10)triène-3,17bêta-diol de l'exemple 39.**

<u>Stade A</u> : [(9-bromononyl) oxy] diméthyl [(1,1-diméthyl) éthyl] silane.

On refroidit à 0°C 443,5 g de bromononanol dans 2090 $cm^3$ de chlorure de méthylène, 314 $cm^3$ de triéthylamine et 4,58 g de diméthylaminopyridine. On ajoute en 25 minutes 318 g de chlorure de terbutyldiméthylsilyle dissous dans 586 $cm^3$ de chlorure de méthylène. On agite pendant 17 heures, filtre, évapore le solvant, reprend le résidu à l'hexane, le lave avec une solution aqueuse d'acide chlorhydrique 0,1N puis à l'eau salée. On évapore le solvant et obtient 620 g de produit brut que l'on distille et recueille 570 g de produit pur (Eb : 110°-130°C sous 0,15 à 0,20 mbars).

<u>Stade B</u> : 11bêta-(9-hydroxynonyl) estra-4,9-diène-3,17-dione.

On refroidit à 0°/+2°C 5,2 $cm^3$ d'une solution de magnésien (0,42 M/l) préparée à partir du produit obtenu au stade A et 2,3 $cm^3$ de tétrahydrofuranne. On ajoute 30 mg de chlorure de cuivre, agite 30 minutes, refroidit à -30°C et ajoute

330 mg de 3-éthylènedioxy 5(10-époxy estr-9(11)-ène-17-one dissous dans 2 $cm^3$ de tétrahydrofuranne et agite 45 minutes. On ajoute 6 $cm^3$ d'acide chlorhydrique 2M et agite 2 heures à température ambiante. On verse dans une solution aqueuse saturée de bicarbonate de sodium, extrait à l'acétate d'éthyle, lave à l'eau salée, sèche et évapore le solvant. Après chromatographie du résidu sur silice (éluant : acétate d'éthyle-cyclohexane 6-4), on obtient 192 mg de produit attendu.

Stade C : 11bêta-[9-(4-méthylbenzènesulfonyloxy) nonyl] estra-4,9-diène-3,17-dione.

On agite pendant 1 heure et demie 13,35 g de produit préparé comme indiqué au stade B, 65 $cm^3$ de pyridine, 12,33 g de chlorure de tosyle et 0,573 g de diméthylaminopyridine. On verse dans l'eau, extrait à l'acétate d'éthyle, lave à l'eau salée, sèche, évapore le solvant, chromatographie le résidu sur silice (éluant : acétate d'éthyle-cyclohexane 4-6) et obtient 11,79 g de produit attendu.

Stade D : 3-hydroxy 11bêta-[9-(4-méthylbenzènesulfonyloxy)nonyl] estra-1,3,5(10)-triène-17-one.

On opère comme indiqué aux stades B et C de l'exemple 3, à partir de 11,09 g de la diénone obtenu au stade C précédent. On obtient 9,6 g de produit brut que l'on purifie par chromatographie sur silice (éluant : acétate d'éthyle-cyclohexane 3-7). On obtient 9,48 g de produit attendu.

Stade E : 11bêta-[9-(4-méthylbenzènesulfonyloxy) nonyl] estra-1,3,5(10)-triène-3,17bêta-diol.

On opère comme au stade D de l'exemple 3 à partir de 889 mg de produit obtenu au stade D ci-dessus et obtient 890 mg de produit attendu.

Stade F : 11bêta-[9-(acétylthio) nonyl] estra-1,3,5(10)-triène-3,17bêta-diol.

On opère comme au stade E de l'exemple 8 à partir de 1 g de produit obtenu comme au stade E ci-dessus et 402 mg de thioacétate de potassium. On obtient après chromatographie sur silice (éluant : acétate d'éthyle-cyclohexane 3-7) 636 mg de produit attendu.

| | |
|---|---|
| Spectre I.R. : | $CHCl_3$ |
| OH : | 3602 $cm^{-1}$ |
| aromatique : | 1610, 1584, 1499 $cm^{-1}$ |

**EXEMPLE 40 : 11bêta-[4-[2-[2-[(4,4,5,5,5-pentafluoropentyl) thio] éthoxy] éthoxy] phényl] estra-1,3,5 (10)-triène-3,17-bêta-diol.**

On opère comme au stade F de l'exemple 8 à partir de 194 mg de 4,4,5,5,5-pentafluoroiodopentane préparé à l'exemple 8 et 313 mg de thioacétate préparé ci-dessous. On obtient 330 mg de produit attendu.

| | |
|---|---|
| Spectre I.R. : | $CHCl_3$ |
| OH : | 3600 $cm^{-1}$ |
| aromatique : | 1610, 1584, 1512 $cm^{-1}$ |

**Préparation du 11bêta-[4-[2-[2-(acétylthio) éthoxy) éthoxy] phényl] estra-1,3,5(10)-triène-3,17bêta-diol.**

Stade A : 2-[2-[[(1,1-diméthyléthyl) diméthylsilyl] oxy] éthoxy] éthanol.

On ajoute 20 g de diéthylèneglycol à 9,05 g d'hydrure de sodium dans 320 $cm^3$ de tétrahydrofuranne, agite 45 minutes, ajoute 28,3 g de chlorure de diméthylterbutylsilane, agite 3 heures en laissant revenir à température ambiante. On extrait à l'éther, lave avec une solution aqueuse de bicarbonate de sodium, à l'eau salée, sèche et évapore le solvant. On obtient 40,25 g de produit attendu.

Stade B : [2-(2-bromoéthoxy) éthoxy] (1,1-diméthyléthyl) diméthylsilane.

On ajoute en 15 minutes à -15°/-20°C, 18,2 g de tétrabromure de carbone dans un mélange comprenant 11,2 g du produit du stade A et 14,4 g de triphénylphosphine dans 100 $cm^3$ de chlorure de méthylène. On agite pendant 1 heure 15 minutes à -15°C, évapore le solvant, reprend au pentane, agite à température ambiante, essore, lave au

pentane et sèche à 50°C sous pression réduite. On obtient 10,1 g de produit attendu. (Eb : 58°C/0,04 mbar).

Stade C : 11bêta-[4-[2-[2-[[(1,1-diméthyléthyl) diméthylsilyl] oxy] éthoxy] éthoxy] phényl] estra-4,9-diène-3,17-dione.

On ajoute 412 mg de produit obtenu au stade B en solution dans 2 $cm^3$ de diméthylformamide dans 362 mg d'un mélange comprenant 362 mg de 11bêta-(4-hydroxyphényl) 4,9-estra-diène-3,17-dione, 4 $cm^3$ de diméthylformamide et 55 mg d'hydrure de sodium. On agite 3 heures à température ambiante, ajoute une solution aqueuse de chlorure d'ammonium, extrait à l'acétate d'éthyle, lave à l'eau salée, sèche et évapore le solvant. Après chromatographie sur silice (éluant : acétate d'éthyleesence G 6-4), on obtient 400 mg de produit attendu.

Stade D : 11bêta-[4-[2-(2-hydroxyéthoxy) éthoxy] phényl] estra-4,9-diène-3,17-dione.

On ajoute 1,3 $cm^3$ d'acide chlorhydrique 2N à 740 mg de produit préparé comme au stade C en solution dans 8 $cm^3$ de méthanol. On agite 1 heure, évapore le solvant, reprend à l'acétate d'éthyle, lave avec une solution aqueuse de bicarbonate de sodium, à l'eau salée, sèche et évapore le solvant. On obtient 595 mg de produit attendu.

Stade E : 11bêta-[4-[2-[2-[(4-méthylbenzènesulfonyl) oxy] éthoxy] éthoxy] phényl] estra-4,9-diène-3,17-dione.

A 690 mg de produit obtenu au stade D dans 5 $cm^3$ de pyridine, on ajoute 320 mg de chlorure de tosyle et 70 mg de diméthylaminopyridine puis de nouveau 2 fois 115 mg de chlorure de tosyle. On agite 2 heures et demie, acidifie à l'aide d'acide chlorhydrique 6N, extrait à l'acétate d'éthyle, lave avec une solution aqueuse de bicarbonate de sodium, à l'eau salée, sèche et évapore le solvant sous pression réduite, chromatographie le résidu sur silice (éluant : acétate d'éthyle-essence G 7-3 puis acétate d'éthyle). On obtient 710 mg de produit attendu.

Stade F :3-hydroxy 11bêta-[4-[2-[2-[(4-méthylbenzènesulfonyl) oxy] éthoxy] éthoxy] phényl] estra-1,3,5 (10)-triène-17-one.

On opère comme indiqué aux stades B et C de l'exemple 3, à partir de 705 mg de la diénone obtenu au stade E précédent. On obtient 785 mg de produit attendu.

Stade G : 11bêta-[4-[2-[2-[(4-méthylbenzènesulfonyl) oxy] éthoxy] éthoxy] phényl] estra-1,3,5(10)-triène-3,17bêta-diol.

On opère comme au stade D de l'exemple 3 à partir de 785 mg de produit obtenu au stade F ci-dessus et obtient 434 mg de produit attendu après chromatographie sur silice (éluant : acétate d'éthyle-essence G 6-4).

Stade H : 11bêta-[4-[2-[2-(acétylthio) éthoxy] éthoxy] phényl] estra-1,3,5(10)-triène-3,17bêta-diol.

On opère comme au stade E de l'exemple 8 à partir de 432 mg de produit obtenu comme au stade G ci-dessus et 163 mg de thioacétate de potassium. On obtient après chromatographie sur silice (éluant : acétate d'éthyle-essence G 6-4) 325 mg de produit attendu.

| | |
|---|---|
| Spectre IR | ($CHCl_3$) |
| OH : | 3601 $cm^{-1}$ |
| S-C=O : | 1689 $cm^3$ |
| aromatique : | 1610, 1583, 1512 $cm^{-1}$ |

**EXEMPLE 41 : 11bêta-[4-[5-[(3,3,4,4,4-pentafluorobutyl) thio] pentyloxy] phényl] estra-1,3,5 (10)-triène-3,17bêta-diol.**

On mélange 589 mg de mercaptan obtenu comme à l'exemple 6 stade Ba, 790 mg de 3,3,4,4,4-pentafluoroiodobutane (préparé comme indiqué pour le pentafluoroiodopentane à l'exemple 8), 820 mg de carbonate de césium et 6 $cm^3$ de diméthylformamide. On chauffe 1 heure à 50°C, refroidit à température ambiante, acidifie à l'aide d'acide chlorhydrique 2N, extrait à l'acétate d'éthyle, lave à l'eau salée et évapore le solvant. Après chromatographie sur silice (éluant : essence G-acétate d'éthyle 6-4), on obtient 360 mg de produit attendu.

| | |
|---|---|
| Spectre IR | ($CHCl_3$) |
| OH : | 3600 $cm^{-1}$ |
| aromatique : | 1610, 1582, 1512 $cm^{-1}$ |

**EXEMPLE 42 : 11bêta-[4-[6-[(3,3,4,4,4-pentafluorobutyl) thio] hexyloxy] phényl] estra-1,3,5(10)-triène-3,17bêta-diol.**

On opère comme à l'exemple 41 à partir de 540 mg du mercaptan approprié (préparé comme indiqué à l'exemple 6) 700 mg de réactif iodé et 730 mg de carbonate de césium. On obtient 247 mg de produit attendu.

| | |
|---|---|
| Spectre IR | ($CHCl_3$) |
| OH : | 3608 $cm^{-1}$ |
| aromatique : | 1610, 1580, 1512, 1503 (ep.) $cm^{-1}$ |

En opérant comme à l'exemple 9 en utilisant au départ le diol approprié comportant une chaîne soufrée en position 11 et du métaperiodate de sodium, on a préparé les produits des exemples 43 à 53.

**EXEMPLE 43 : 3,7-dihydro 7-[2-[[5-[4-(3,17bêta-dihydroxy estra-1,3,5(10)-trièn-11bêta-yl) phénoxy] pentyl] sulfinyl] éthyl] 1,3-diméthyl 1H-purine-2,6-dione.**

| | |
|---|---|
| Spectre IR | ($CHCl_3$) |
| OH : | 3605 $cm^{-1}$ |
| C=O : | 1704, 1657 $cm^{-1}$ |
| Système conjugué + aromatique : | 1608, 1581, 1551, 1512 $cm^{-1}$ |
| S -> O ≈ | 1031 $cm^{-1}$ |

**EXEMPLE 44 : 11bêta-[4-[6-[(4,4,4-trifluorobutyl) sulfinyl] hexyloxy] phényl] estra-1,3,5(10)-triène-3,17bêta-diol.**

| | |
|---|---|
| Spectre IR | ($CHCl_3$) |
| OH ≈ | 3604 $cm^{-1}$ |
| aromatique : | 1610, 1581, 1512 $cm^{-1}$ |
| S -> O ≈ | 1031 $cm^{-1}$ |

**EXEMPLE 45 : 11bêta-[4-[5-[(4,4,4-trifluorobutyl) sulfinyl] pentyloxy] phényl] estra-1,3,5(10)-triène-3,17bêta-diol.**

| | |
|---|---|
| Spectre IR | ($CHCl_3$) |
| OH ≈ | 3607 $cm^{-1}$ |
| aromatique : | 1610, 1580, 1512 $cm^{-1}$ |
| S -> O ≈ | 1030 $cm^{-1}$ |

**EXEMPLE 46 : 11bêta-[4-[6-[(2,2,2-trifluoroéthyl) sulfinyl] hexyloxy] phényl] estra-1,3,5(10)-triène-3,17bêta-diol.**

| | |
|---|---|
| Spectre IR | ($CHCl_3$) |
| OH ≈ | 3602 $cm^{-1}$ + associé |
| aromatique : | 1610, 1581, 1512 $cm^{-1}$ |
| S -> O ≈ | 1044 $cm^{-1}$ |

**EXEMPLE 47 : 11bêta-[4-[4-[(4,4,4-trifluorobutyl) sulfinyl] butyloxy] phényl] estra-1,3,5(10)-triène-3,17bêta-diol.**

| | |
|---|---|
| Spectre IR | ($CHCl_3$) |
| OH ≈ | 3603 $cm^{-1}$ |
| aromatique : | 1610, 1581, 1512 $cm^{-1}$ |
| S -> O ≈ | 1030 $cm^{-1}$ |

**EXEMPLE 48 : 11bêta-[4-[4-[(4,4,5,5,5-pentafluoropentyl) sulfinyl] butyloxy] phényl] estra-1,3,5(10)-triène-3,17bêta- diol.**

Spectre IR ($CHCl_3$)

OH ≈ 3598 $cm^{-1}$
aromatique : 1610, 1584, 1512 $cm^{-1}$
S -> O ≈ 1031 $cm^{-1}$

**<u>EXEMPLE 49</u> : 11bêta-[4-[5-(pentylsulfinyl) pentyloxy] phényl] estra-1,3,5(10)-triène-3,17bêta-diol.**

Spectre IR ($CHCl_3$)
OH ≈ 3605 $cm^{-1}$
aromatique : 1610, 1581, 1512 $cm^{-1}$
S -> O ≈ 1020 $cm^{-1}$

**<u>EXEMPLE 50</u> : 11bêta-[9-[(4,4,5,5,5-pentafluoropentyl) sulfinyl] nonyl] estra-1,3,5(10)-triène-3,17bêta-diol.**

Spectre IR ($CHCl_3$)
OH ≈ 3598 $cm^{-1}$
aromatique : 1614, 1608, 1580, 1499 $cm^{-1}$
S -> O ≈ 1020 $cm^{-1}$

**<u>EXEMPLE 51</u> : 11bêta-[4-[5-[(3,3,4,4,4-pentafluorobutyl) sulfinyl] pentyloxy] phényl] estra-1,3,5(10)-triène-3,17bêta-diol.**

Spectre IR ($CHCl_3$)
OH ≈ 3596 $cm^{-1}$
aromatique : 1610, 1580, 1512 $cm^{-1}$

**<u>EXEMPLE 52</u> : 11bêta-[4-[6-[(3,3,4,4,4-pentafluorobutyl) sulfinyl] hexyloxy] phényl] estra-1,3,5(10)-triène-3,17bêta-diol.**

Spectre IR ($CHCl_3$)
OH ≈ 3505 $cm^{-1}$
aromatique : 1610, 1580, 1512 $cm^{-1}$
S -> O ≈ 1043 $cm^{-1}$

**<u>EXEMPLE 53</u> : 11bêta-[4-[2-[2-[(4,4,5,5,5-pentafluoropentyl) sulfinyl] éthoxy] éthoxy] phényl] estra-1,3,5(10)-triène-3,17bêta-diol.**

Spectre IR ($CHCl_3$)
OH ≈ 3606 $cm^{-1}$
aromatique : 1610, 1580, 1512 $cm^{-1}$
S -> O ≈ 1044 $cm^{-1}$

En opérant comme à l'exemple 9a à partir de 183 mg de produit préparé comme indiqué à l'exemple 34 et 172 mg d'acide perphtalique, on a préparé le produit des exemples 54 et 55.

**<u>EXEMPLE 54</u> : 11bêta-[4-[5-[(7-(trifluorométhyl) 4-quinoléinyl] sulfinyl] pentyloxy] phényl] estra-1,3,5(10)-triène-3,17bêta-diol.**

Spectre IR ($CHCl_3$)
OH ≈ 3602 $cm^{-1}$ + associé
aromatique + hétérocycle : 1610, 1583, 1511 $cm^{-1}$
S -> O ≈ 1056 $cm^{-1}$

**<u>EXEMPLE 55</u> : 11bêta-[4-[5-[(7-(trifluorométhyl) 4-quinoléinyl] sulfonyl] pentyloxy] phényl] estra-1,3,5(10)-triène-3,17bêta-diol.**

Spectre IR ($CHCl_3$)
OH ≈ 3604 $cm^{-1}$ + associé
aromatique + hétérocycle : 1610, 1583, 1511 $cm^{-1}$

| | |
|---|---|
| $SO_2$ ≈ | 1336 (ep.) et 1160 $cm^{-1}$ |

**EXEMPLE 56 : 11bêta-[4-[5-[(4,4,4-trifluorobutyl) sulfonyl] pentyloxy] phényl] estra-1,3,5(10)-triène-3,17bêta-diol.**

On ajoute 255 mg d'acide métachloro perbenzoïque dans 250 mg de diol préparé comme à l'exemple 37 dans 6 $cm^3$ de chlorure de méthylène refroidi à +4°C. On agite pendant 45 minutes, ajoute du thiosulfate de sodium puis une solution de bicarbonate de sodium, décante, évapore le solvant, chromatographie le résidu sur silice (éluant : acétate d'éthyle-essence G 6-4) et obtient 210 mg de produit attendu.

| | |
|---|---|
| Spectre IR | ($CHCl_3$) |
| OH ≈ | 3604 $cm^{-1}$ |
| aromatique : | 1610, 1580, 1512 $cm^{-1}$ |
| $SO_2$ : | 1309 et ≈ 1136 $cm^{-1}$ |

En opérant comme à l'exemple 56 en utilisant au départ le diol approprié comportant une chaîne soufrée en position 11 et l'acide métachloro perbenzoïque, on a préparé les produits des exemples 57 à 67.

**EXEMPLE 57 : 11bêta-[4-[6-[(4,4,4-trifluorobutyl) sulfonyl] hexyloxy] phényl] estra-1,3,5(10)-triène-3,17bêta-diol.**

| | |
|---|---|
| Spectre IR | ($CHCl_3$) |
| OH ≈ | 3602 $cm^{-1}$ |
| aromatique : | 1610, 1581, 1512 $cm^{-1}$ |
| $SO_2$ : | 1306 et ≈ 1135 $cm^{-1}$ |

**EXEMPLE 58 : 11bêta-[4-[4-[(4,4,5,5,5-pentafluoropentyl) sulfonyl] butyloxy] phényl] estra-1,3,5(10)-triène-3,17bêta-diol.**

| | |
|---|---|
| Spectre IR | ($CHCl_3$) |
| OH ≈ | 3605 $cm^{-1}$ |
| aromatique : | 1610, 1582, 1512 $cm^{-1}$ |
| $SO_2$ : | 1305 et ≈ 1132 $cm^{-1}$ |

**EXEMPLE 59 : 11bêta-[4-[4-[(4,4,4-trifluorobutyl) sulfonyl] butyloxy] phényl] estra-1,3,5(10)-triène-3,17bêta-diol.**

| | |
|---|---|
| Spectre IR | ($CHCl_3$) |
| OH/NH | absorption complexe |
| aromatique : | 1610, 1580, 1511$cm^{-1}$ |
| $SO_2$ : | 1296 et 1134 $cm^{-1}$ |

**EXEMPLE 60 : 11bêta-[4-[4-(pentylsulfonyl) pentyloxy] phényl] estra-1,3,5(10)-triène-3,17bêta-diol.**

| | |
|---|---|
| Spectre IR | ($CHCl_3$) |
| OH : | 3600 $cm^{-1}$ |
| aromatique : | 1610, 1584, 1512 $cm^{-1}$ |
| $SO_2$ : | 1297 et 1130 $cm^{-1}$ |

**EXEMPLE 61 : 11bêta-[9-[(4,4,5,5,5-pentafluoropentyl) sulfonyl] nonyl] estra-1,3,5 (10)-triène-3,17bêta-diol.**

| | |
|---|---|
| Spectre IR | ($CHCl_3$) |
| OH : | 3601$cm^{-1}$ |
| aromatique : | 1609, 1584, 1500 $cm^{-1}$ |
| $SO_2$ : | 1309 et 1134 $cm^{-1}$ |

**EXEMPLE 62 : 11bêta-[4-[5-[(3,3,4,4,4-pentafluorobutyl) sulfonyl] pentyloxy] phényl] estra-1,3,5(10)-triène-3,17bêta-diol.**

| | |
|---|---|
| Spectre IR | ($CHCl_3$) |
| OH : | 3596 $cm^{-1}$ |
| aromatique : | 1610, 1580, 1512 $cm^{-1}$ |

**EXEMPLE 63 : 11bêta-[4-[6-[(3,3,4,4,4-pentafluorobutyl) sulfonyl] hexyloxy) phényl] estra-1,3,5(10)-triène-3,17bêta-diol.**

| | |
|---|---|
| Spectre IR | ($CHCl_3$) |
| OH : | 3600 $cm^{-1}$ |
| aromatique : | 1610, 1580, 1512, 1504 $cm^{-1}$ |
| $SO_2$ : | 1298 et 1134 $cm^{-1}$ |

**EXEMPLE 64 : 11bêta-[4-[2-[2-[(4,4,5,5,5-pentafluoropentyl) sulfonyl] éthoxy] éthoxy] phényl] estra-1,3,5(10)-triène-3,17bêta-diol.**

| | |
|---|---|
| Spectre IR | ($CHCl_3$) |
| OH : | 3600 $cm^{-1}$ |
| aromatique : | 1610, 1580, 1512 $cm^{-1}$ |
| $SO_2$ : | 1317 et 1129 $cm^{-1}$ |

**EXEMPLE 65 : 11bêta-[4-[5-[(7-(trifluorométhyl) 4-quinoléinyl] sulfinyl] pentyloxy] phényl] estra-1,3,5(10)-triène-3,17bêta-diol.**

Ce produit est identique à celui obtenu à l'exemple 54.

| | |
|---|---|
| Spectre IR | ($CHCl_3$) |
| OH ≈ | 3602 $cm^{-1}$ + associé |
| aromatique : | 1610, 1583, 1511 $cm^{-1}$ |
| S -> O ≈ | 1056 $cm^{-1}$ |

**EXEMPLE 66 : Butanedioate de 3-hydroxy 11bêta-[4-[5-[(4,4,5,5,5-pentafluoropentyl) sulfinyl] pentyloxy] phényl] estra-1,3,5(10)-triène-17bêta-yl et de sodium.**

a) Préparation de l'acide.

On agite 4 heures à 120°C 380 mg de produit obtenu à l'exemple 31, 355 mg d'anhydride succinique, 40 mg de diméthylaminopyridine et 4 $cm^3$ de pyridine. On laisse revenir à température ambiante, ajoute 4 $cm^3$ de méthanol, 4 $cm^3$ d'eau et 650 mg de carbonate de potassium. On agite 6 heures 30 minutes, refroidit à +4°C, acidifie à l'aide d'acide chlorhydrique, extrait à l'acétate d'éthyle, lave à l'eau salée, sèche et évapore les solvants. Après chromatographie sur silice (éluant : essence G-acétone 5-5), on obtient 400 mg d'acide.

| | |
|---|---|
| Spectre IR | ($CHCl_3$) |
| OH ≈ | 3600 $cm^{-1}$ + absorption générale de type OH acide |
| C=O : | 1717 $cm^{-1}$ + complexe |
| aromatique : | 1610, 1585, 1512 $cm^{-1}$ |

b) Salification :

On dissout 367 mg de l'acide obtenu ci-dessus dans 4 $cm^3$ d'éthanol, ajoute 36 mg de bicarbonate de sodium dissous dans 4 $cm^3$ d'eau, agite 30 minutes, évapore l'éthanol, ajoute 20 $cm^3$ d'eau, filtre puis lyophilise. On recueille 250 mg de produit attendu.

**EXEMPLE 67 : Butanedioate de 3-hydroxy 11bêta-[4-[5-[(4,4,5,5,5-pentafluoropentyl) sulfonyl] pentyloxy] phényl] estra-1,3,5(10)-triène-17bêta-yl et de sodium.**

On opérant comme à l'exemple 66 à partir de 290 mg de produit obtenu à l'exemple 32, on obtient 285 mg de produit sous forme d'acide.

| | |
|---|---|
| Spectre IR | ($CHCl_3$) |
| OH : | 3598, 3518 $cm^{-1}$ + absorption générale |
| C=O : | 1716 $cm^{-1}$ |
| aromatique : | 1610, 1582, 1512 $cm^{-1}$ |
| $SO_2$ : | 1304, 1133 $cm^{-1}$ |

On salifie 273 mg d'acide et obtient 235 mg de produit brut attendu que l'on purifie par chromatographie sur silice (éluant : méthanol-eau 8-2).

**EXEMPLE 68 : 3-cyclopentyloxy 11bêta-[4-[5-[(4,4,5,5,5-pentafluoropentyl) sulfinyl] pentyloxy] phényl] estra-1,3,5(10)-triène-17bêta-ol.**

On agite pendant 30 minutes 1 g de produit préparé à l'exemple 31, 8,7 $cm^3$ de diméthylformamide et 82 mg d'hydrure de sodium. On ajoute ensuite 486 mg de 4-méthylbenzènesulfonate de cyclopentyle, agite pendant 5 heures, verse dans une solution aqueuse de chlorure d'ammonium, extrait à l'acétate d'éthyle, lave à l'eau salée, sèche et évapore le solvant. On chromatographie le résidu sur silice (éluant : chlorure de méthylène-isopropanol 95-5) et obtient 861 mg de produit que l'on cristallise dans le mélange chlorure de méthylène/éther éthylique et récupère 777 mg de produit attendu. F = 189°C.

| | |
|---|---|
| Spectre IR | ($CHCl_3$) |
| OH ≈ | 3608 $cm^{-1}$ |
| aromatique : | 1610, 1580, 1570, 1512, 1498 $cm^{-1}$ |
| S -> O ≈ | 1043 $cm^{-1}$ |

**Préparation du 4-méthylbenzènesulfonate de cyclopentyle.**

On agite 3 heures à température ambiante 1 g de cyclopentanol, 23 $cm^3$ de pyridine, 4,42 g de chlorure de tosyle et 195,8 mg de 4-diméthylaminopyridine, évapore la pyridine, verse dans l'eau, extrait à l'acétate d'éthyle, lave avec une solution aqueuse d'acide chlorhydrique M puis à l'eau salée et avec une solution aqueuse saturée de bicarbonate de sodium. On évapore les solvants, dissout dans le méthanol, ajoute de nouveau du bicarbonate de sodium, évapore, chromatographie le résidu sur silice (éluant : acétate d'éthyle-essence G 1-9) et obtient 1,41 g de tosylate attendu.

**EXEMPLE 69 : 3-cyclopentyloxy 11bêta-[4-[5-[(4,4,5,5,5-pentafluoropentyl) sulfonyl] pentyloxy] phényl] estra-1,3,5 (10)-triène-17bêta-ol.**

En opérant comme à l'exemple 56 en utilisant au départ 698 mg de produit obtenu à l'exemple 68 et 228 mg d'acide métachloro perbenzoïque, on obtient 567 mg de produit attendu. F = 166°C.

| | |
|---|---|
| Spectre IR | ($CHCl_3$) |
| OH ≈ | 3608 $cm^{-1}$ |
| aromatique : | 1610, 1580, 1570, 1512, 1498 $cm^{-1}$ |

**EXEMPLE 70 : Butanedioate de 3-cyclopentyloxy 11bêta-[4-[5-[(4,4,5,5,5-pentafluoropentyl) sulfonyl] pentyloxy] phényl] estra-1,3,5(10)-triène-17bêta-yl et de sodium.**

En opérant comme indiqué à l'exemple 66 à partir de 384 mg de produit obtenu comme à l'exemple 68, on obtient 301 mg d'acide après cristallisation dans le mélange chlorure de méthylène/éther isopropylique. F = 188°C.

| | |
|---|---|
| Spectre IR | ($CHCl_3$) |
| C=O : | 1716 $cm^{-1}$ |
| aromatique : | 1610, 1580, 1572, 1512, 1498 $cm^{-1}$ |

On salifie 285,5 mg de l'acide ci-dessus et obtient 295 mg de produit attendu après lyophilisation.

Spectre IR (CHCl$_3$)
OH/NH : absorption générale
C=O : 1724 cm$^{-1}$
aromatique : 1609, 1510, 1498 cm$^{-1}$
COO$^-$ ≈ 1578 cm$^{-1}$

**EXEMPLE 71 : 3-hydroxy 11bêta-[4-[5-[(4,4,5,5,5-pentafluoropentyl) thio] pentyloxy] phényl] estra-1,3,5(10)-triène-17bêta-one.**

Stade A : 3-hydroxy 11bêta-[4-[(5-iodopentyl) oxy] phényl] estra-1,3,5(10)-triène-17-one.

On opère comme au stade D de l'exemple 8 en utilisant au départ 14,9 g de produit obtenu comme à l'exemple 3C. On obtient 13,2 g de produit attendu. Rf = 0,18 (cyclohexane-acétate d'éthyle 7-3).

Stade B : 3-hydroxy 11bêta-[4-[[5-(thioacétyl) pentyl] oxy] phényl] estra-1,3,5(10)-triène-17-one.

On opère comme au stade E de l'exemple 8 en utilisant au départ 13,2 g de produit obtenu au stade A. On obtient 9 g de produit attendu. Rf = 0,58 (cyclohexane-acétate d'éthyle 7-3).

Stade C : 3-hydroxy 11bêta-[4-[5-[(4,4,5,5,5-pentafluoropentyl) thio] pentyloxy] phényl] estra-1,3,5(10)-triène-17-one.

On opère comme au stade F de l'exemple 8 en utilisant au départ 9 g de produit obtenu au stade B. On obtient 8,63 g de produit attendu. Rf = 0,6 (cyclohexane-acétate d'éthyle 6-4).

**EXEMPLE 72 : 3-hydroxy 11bêta-[4-[5-[(4,4,5,5,5-pentafluoropentyl) sulfinyl] pentyloxy] phényl] estra-1,3,5 (10)-triène-17-one.**

On opère comme à l'exemple 9 en utilisant 180 mg de produit obtenu à l'exemple 71. On obtient 120 mg de produit attendu. Rf = 0,37 (chlorure de méthylène-méthanol 95-5).

**EXEMPLE 73 : 3-hydroxy 11bêta-[4-[5-[(4,4,5,5,5-pentafluoropentyl) sulfonyl] pentyloxy] phényl] estra-1,3,5 (10)-triène-17-one.**

On opère comme à l'exemple 56 en utilisant au départ 180 mg de produit obtenu à l'exemple 71 et 162 mg d'acide métachloro perbenzoïque. On obtient 160 mg de produit attendu.

**EXEMPLE 74 : 17-méthylène 11bêta-[4-[5-[(4,4,5,5,5-pentafluoropentyl) sulfonyl] pentyloxy] phényl] estra-1,3,5 (10)-triène-3-ol.**

On ajoute 500 mg de produit obtenu à l'exemple 73 dans un mélange comprenant 815 mg de bromure de méthyltriphénylphosphonium, 5 cm$^3$ de dioxanne et 125 mg de méthylate de sodium. On agite 16 heures à température ambiante, ajoute 100 cm$^3$ d'une solution aqueuse de chlorure d'ammonium, extrait au chlorure de méthylène, sèche, élimine le solvant sous pression réduite, chromatographie le résidu sur silice (éluant : cyclohexane-acétate d'éthyle 7-3). On obtient 80 mg de produit attendu.

Spectre IR (CHCl$_3$)
Peu ou pas de C=O
OH : 3596 cm$^{-1}$
C=C : 1656 cm$^{-1}$
aromatique : 1610, 1580, 1512 cm$^{-1}$

**EXEMPLE 75 : 17-bêta (hydroxyméthyl) 11bêta-[4-[5-[(4,4,5,5,5-pentafluoropentyl) sulfonyl] pentyloxy] phényl] estra-1,3,5(10)-triène-3-ol.**

On refroidit à +4°C 60 mg de produit obtenu à l'exemple 74 dans 3 cm$^3$ de tétrahydrofuranne et ajoute 100 µl de sulfure de diméthylborane. On agite 2 heures à température ambiante, refroidit à 0°C, ajoute 200 µl de lessive de

soude puis 200 µl d'eau oxygénée à 30%. On laisse revenir à température ambiante puis acidifie avec une solution d'acide chlorhydrique. On extrait au chlorure de méthylène, sèche, élimine le solvant, chromato-graphie le résidu sur silice (éluant : acétate d'éthyle-cyclo-hexane 3-7) et obtient 35 mg de produit attendu.

| | |
|---|---|
| Spectre IR | ($CHCl_3$) |
| OH phénolique : | 3590 $cm^{-1}$ |
| aromatique : | 1610, 1580, 1511 $cm^{-1}$ |
| C=C ou C=O | conjuguée ≈ 1660 $cm^{-1}$ |

**EXEMPLE 76 : Oxime de 3-hydroxy 11bêta-[4-[5-[(4,4,5,5,5-pentafluoropentyl) sulfonyl] pentyloxy] phényl] estra-1,3,5(10)-triène-17-one.**

A 1 g de produit obtenu comme à l'exemple 73, on ajoute un mélange comprenant 30 $cm^3$ d'éthanol et 5 $cm^3$ d'eau puis 320 mg de chlorhydrate d'hydroxylamine et 501 mg d'acétate de sodium. On chauffe 2 heures au reflux, ajoute 50 $cm^3$ d'eau, extrait au chlorure de méthylène, sèche, élimine le solvant sous pression réduite, cristallise le résidu dans l'éther isopropylique, filtre, chromatographie le résidu sur silice (cyclohexane-acétate d'éthyle 7-3) et obtient 90 mg de produit attendu.

| | |
|---|---|
| Spectre IR | ($CHCl_3$) |
| OH : | 3593 $cm^{-1}$ + associés |
| aromatique : | 1610, 1582, 1512, 1502 (ep.) $cm^{-1}$ |

**EXEMPLE 77 : Hydrazone de 3-hydroxy 11bêta-[4-[5-[(4,4,5,5,5-pentafluoropentyl) sulfonyl] pentyloxy] phényl] estra-1,3,5(10)-triène-17-one.**

On ajoute 100 µl d'hydrate d'hydrazine et 3 mg d'acide paratoluènesulfonique à 300 mg de produit obtenu à l'exemple 73 dans 3 $cm^3$ de méthanol. On agite 16 heures à température ambiante, ajoute 50 $cm^3$ d'une solution aqueuse de bicarbonate de sodium, extrait au chlorure de méthylène, évapore le solvant, chromatographie le résidu sur silice (éluant : acétate d'éthyle) et obtient 190 mg de produit attendu. Spectre IR ($CHCl_3$)

| | |
|---|---|
| OH : | 3599 $cm^{-1}$ |
| $NH_2$ : | 3395 $cm^{-1}$ |
| C=N et aromatique : | 1610, 1588, 1512, 1503 $cm^{-1}$ |

**EXEMPLE 78 : 11bêta-[4-[5-[(4,4,5,5,5-pentafluoropentyl) sulfonyl] pentyloxy] phényl] 17alpha-méthyl estra-1,3,5(10)-triène-3,17bêta-diol.**

On déshydrate pendant 2 heures à 180°C sous pression réduite du chlorure de cérium heptahydraté, laisse refroidir, on en introduit 1,5 g dans 15 $cm^3$ de tétrahydrofuranne, agite 1 heure, ajoute à -60°C 4 $cm^3$ de méthyllithium en solution 1,5M dans l'éther, agite 30 minutes, refroidit à -78°C et ajoute 300 mg du produit obtenu à l'exemple 71. On maintient sous agitation à -78°C pendant 1 heure, ajoute une solution aqueuse de chlorure d'ammonium, extrait au chlorure de méthylène, évapore le solvant, chromatographie le résidu sur silice (éluant : cyclohexane-acétate d'éthyle 7-3) et obtient 198 mg de dérivé 17-méthylé. On opère comme à l'exemple 56 à partir de 198 mg du produit obtenu ci-dessus et 150 $cm^3$ d'acide métachloro perbenzoïque et obtient 62 mg de produit attendu. Rf = 0,37 (cyclohexane-acétate d'éthyle 1-1).

**EXEMPLE 79 : 3-(cyclopontyloxy) 11bêta-[4-[5-[(4,4,5,5,5-pentafluoropentyl) sulfonyl] pentyloxy] phényl] estra-1,3,5(10)-triène-17-one.**

On opère comme à l'exemple 68 en utilisant au départ 300 mg de produit obtenu à l'exemple 71 et 170 mg de 4-méthyl benzène sulfonate de cyclopentyle. On extrait au chlorure de méthylène le milieu réactionnel obtenu, sèche, évapore le solvant, chromatographie le résidu sur silice (éluant : acétate d'éthyle-cyclohexane 3-7) et obtient 290 mg de produit attendu utilisé tel quel.

**EXEMPLE 80 : 3-(cyclopentyloxy) 11bêta-[4-[5-[(4,4,5,5,5-pentafluoropentyl) sulfonyl] pentyloxy] phényl] 19-nor 17alpha-pregna-1,3,5(10)-triène-20-yn-17bêta-ol.**

On refroidit à -78°C 150 µl de triméthylsilyl acétylène dans 5 $cm^3$ de tétrahydrofuranne et ajoute goutte à goutte

625 µl d'une solution de n-butyllithium dans l'hexane (1,6M). On agite 30 minutes, ajoute 140 mg de produit obtenu à l'exemple 79 en solution dans 1 $cm^3$ de tétrahydrofuranne et agite 2 heures à température ambiante. On ajoute 2 $cm^3$ d'une solution 1M de fluorure de tétrabutylammonium dans le tétrahydrofuranne. On agite 1 heure à température ambiante, ajoute 10 $cm^3$ d'eau, extrait au chlorure de méthylène, sèche et évapore le solvant. Après chromatographie sur silice (éluant : cyclohexane-acétate d'éthyle 7-3), on obtient 60 mg de produit attendu.

**EXEMPLE 81 : 17alpha-éthynyl 11bêta-[4-[5-[(4,4,5,5,5-pentafluoropentyl) thio] pentyloxy] phényl] estra-1,3,5 (10)-triène-3,17bêta-diol.**

On ajoute 126 mg d'acétylure de lithium sous forme de complexe avec l'éthylènediamine (Li-C≡CH.EDA) à 1 g de produit obtenu à l'exemple 71 dans 10 $cm^3$ de tétrahydrofuranne. On agite 2 heures, ajoute de nouveau 180 mg de (Li-C≡CH.EDA) et agite 12 heures à température ambiante. On verse dans une solution saturée au chlorure d'ammonium, extrait au chlorure de méthylène, lave avec une solution d'acide chlorhydrique N, sèche et élimine le solvant. Après chromatographie sur silice (éluant : acétate d'éthyle-cyclohexane 2-8 puis chlorure de méthylène-méthanol 98-2), on obtient 720 mg de produit attendu utilisé tel quel pour l'exemple suivant.

**EXEMPLE 82 : 17alpha-éthynyl 11bêta-[4-[5-[(4,4,5,5,5-pentafluoropentyl) sulfonyl] pentyloxy] phényl] estra-1,3,5(10)-triène-3,17bêta-diol.**

On opère comme à l'exemple 56 en utilisant au départ 570 mg de produit obtenu à l'exemple 79 et 1 g d'acide métachloro perbenzoïque. On obtient 100 mg de produit attendu.

| | |
|---|---|
| Spectre IR | ($CHCl_3$) |
| OH : | 3598 $cm^{-1}$ |
| C≡CH : | 3308 $cm^{-1}$ µl |
| aromatique : | 1610, 1580, 1510, 1502 $cm^{-1}$ |

**EXEMPLE 83 : 11bêta-[4-[5-[(nonafluorobutyl) thio] pentyloxy] phényl] estra-1,3,5(10)-triène-3,17bêta-diol.**

On ajoute sous atmosphère inerte, 50 mg d'hydrure de sodium à 466 mg de produit obtenu à l'exemple 6Ba en solution dans 10 $cm^3$ de diméthylformamide, agite 30 minutes, ajoute 0,18 $cm^3$ d'iodure de perfluorobutyle, refroidit et soumet 15 minutes aux radiations d'une lampe à vapeur de mercure. On acidifie avec de l'acide chlorhydrique 2N, extrait à l'acétate d'éthyle, lave à l'eau, sèche, évapore le solvant, chromato-graphie le résidu sur silice (éluant : essence G-acétate d'éthyle 6-4 puis acétate d'éthyle seul, puis méthanol-eau 9-1). On recueille 287 mg de produit attendu. Rf = 0,24.

| | |
|---|---|
| Spectre IR | ($CHCl_3$) |
| OH : | 3602 $cm^{-1}$ |
| aromatique : | 1610, 1583, 1512 $cm^{-1}$ |

**EXEMPLE 84 : 3-hydroxy 11bêta-[4-[5-[(4,4,5,5,5-pentafluoropentyl) sulfonyl] pentyloxy] phényl] 16alpha-méthyl estra-1,3,5(10)-triène-17-one.**

Stade A : 3-hydroxy 11bêta-[4-[5-[(4,4,5,5,5-pentafluoropentyl) thio] pentyloxy] phényl] 16alpha-méthyl estra-1,3,5 (10)-triène-17-one.

On refroidit à -78°C 500 mg de 3-tétrahydropyranyloxy 11bêta-[4-[6-[(4,4,5,5,5-pentafluoropentyl) thio] pentyloxy] phényl] estra-1,3,5(10)-triène-17-one, ajoute 750 µl de lithium nitrate triméthylsulfure, agite 10 minutes, ajoute 200 mg d'iodure de méthyle, agite 1 heure à température ambiante, ajoute une solution aqueuse saturée en chlorure d'ammonium, extrait au chlorure de méthylène, sèche, évapore le solvant, chromatographie le résidu sur silice (éluant : cyclohexane-acétate d'éthyle 8-2) et obtient 280 mg de dérivé 16-méthylé auquel on ajoute 5 $cm^3$ d'éthanol puis 2 $cm^3$ d'acide chlorhydrique, agite 3 heures à température ambiante, ajoute une solution saturée en chlorure d'ammonium, extrait au chlorure de méthylène, sèche et évapore le solvant. On chromatographie le résidu sur silice (éluant : cyclohexane-acétate d'éthyle 7-3). On obtient 150 mg de produit attendu.

Stade B : 3-hydroxy 11bêta-[4-[5-[(4,4,5,5,5-pentafluoropentyl) sulfonyl] pentyloxy] phényl] 16alpha-méthyl estra-1,3,5 (10)-triène-17-one.

On refroidit à 0°C 100 mg de produit obtenu ci-dessus dans 5 $cm^3$ de chlorure de méthylène, ajoute 90 mg d'acide métachloroperbenzoïque, agite 1 heure à 0°C, ajoute une solution aqueuse de thiosulfate de sodium et de bicarbonate de sodium 1-1), extrait au chlorure de méthylène, sèche, évapore le solvant, chromatographie le résidu sur silice (éluant : cyclohexane-acétate d'éthyle 7-3) et obtient 60 mg de produit attendu.

| | |
|---|---|
| Spectre IR | ($CHCl_3$) |
| C=O : | 1730 $cm^{-1}$ |
| aromatique : | 1610, 1578, 1510, 1498 $cm^{-1}$ |

**Préparation du 3-tétrahydropyranyloxy 11bêta-[4-[6-[(4,4,5,5,5-pentafluoropentyl) thio] pentyloxy] phényl] estra-1,3,5(10)-triène-17one.**

On agite 3 heures à température ambiante 4 g de 3-hydroxy 11bêta-[4-[5-[(4,4,5,5,5-pentafluoro-pentyl) thio] pentyloxy] phényl] estra-1,3,5(10)-triène-17-one, 40 $cm^3$ de dioxane, 3 $cm^3$ de dihydropyrane et 100 $cm^3$ d'acide paratoluènesulfonique. On ajoute 100 $cm^3$ d'une solution aqueuse saturée en bicarbonate de sodium, extrait au chlorure de méthylène, sèche, évapore le solvant, chromatographie le résidu sur silice (éluant : cyclohexane-acétate d'éthyle 7-3) et obtient 4,5 g de produit attendu.

En opérant comme au stade D de l'exemple 3 au départ du produit obtenu à l'exemple 84, on a préparé le 11bêta-[4-[5-[(4,4,5,5,5-pentafluoropentyl) sulfonyl] pentyloxy] phényl] 16alpha-méthyl estra-1,3,5(10)-triène-3,17bêta-diol.

**<u>EXEMPLE 85</u> : 11bêta-[4-[5-[(pentafluorophényl) méthylthio] pentyloxy] phényl] estra-1,3,5(10)-triène-3,17bêta-diol.**

On opère comme à l'exemple 6Ba à partir de 350 mg de disulfure et 0,19 $cm^3$ de tributylphosphine. A la solution de mercaptan obtenue, on ajoute 0,12 $cm^3$ de alpha-bromo 2,3,4,5,6-pentafluorotoluène et 0,15 $cm^3$ de lessive de soude. On agite à 50°C pendant 40 minutes, refroidit, coule dans une solution d'acide chlorhydrique 2N, extrait à l'acétate d'éthyle, sèche, évapore le solvant, chromatographie le résidu sur silice (éluant : essence G-acétate d'éthyle 7-3) et obtient 227 mg de produit attendu.

| | |
|---|---|
| Spectre IR | ($CHCl_3$) |
| OH : | 3598 $cm^{-1}$ |
| aromatique : | 1654, 1610, 1580, 1520, 1506 $cm^{-1}$ |

**<u>EXEMPLE 86</u> : 11bêta-[4-[5-[[(pentafluorophényl) méthyl] sulfonyl] pentyloxy] phényl] estra-1,3,5(10)-triène-3,17bêta-diol.**

On opère comme à l'exemple 56 en utilisant au départ 207 mg de produit obtenu à l'exemple 85 et 169 mg d'acide métachloroperbenzoïque. On obtient 222 mg de produit attendu après chromatographie sur silice (éluant : AcOEt-cyclohexane 5-5). Spectre IR ($CHCl_3$)

| | |
|---|---|
| OH : | 3603 $cm^{-1}$ |
| aromatique : | 1654, 1610, 1580, 1520, 1506 $cm^{-1}$ |
| $SO^2$ : | 1331, 1131$cm^{-1}$ |

**<u>EXEMPLE 87</u> : 11bêta-[4-[5-[(2-(pentafluorophényl) éthyl] thio] pentyloxy] phényl] estra-1,3,5(10)-triène-3,17bêta-diol et 11bêta-[4-[5-[(4-éthényl 2,3,5,6-tétrafluorophényl) thio] pentyloxy] phényl] estra-1,3,5(10)-triène-3,17bêta-diol.**

On opère comme à l'exemple 6Ba en utilisant au départ 500 mg de disulfure et 0,27 $cm^3$ de tri-n-butyltriphénylphosphine. La solution de mercaptan obtenue est traitée comme indiqué à l'exemple 85 en utilisant 0,30 $cm^3$ de 1-(2-bromoéthyl) 2,3,4,5,6-pentafluorobenzène. On obtient 420 mg de produit attendu sous forme de mélange.

**Préparation du 1-(2-bromoéthyl) 2,3,4,5,6-pentafluorobenzène.**

Stade A : 2-(2,3,4,5-pentafluorophényl) éthanol.

On chauffe au reflux pendant 2 heures 2,5g d'acide 2,3,4,5,6-pentafluorophényl acétique dans 25 $cm^3$ de tétrahydrofuranne en présence de 3,2 $cm^3$ de complexe borohydrure diméthyl sulfure. On refroidit, verse lentement dans l'eau glacée, ajoute une solution aqueuse de bicarbonate de sodium, extrait à l'acétate d'éthyle, lave à l'eau, sèche et évapore le solvant. On obtient 2,24 g de produit attendu.

Stade B : 1-(2-bromoéthyl) 2,3,4,5,6-pentafluorobenzène.

On dissout 2,225 g d'alcool obtenu au stade A dans 21,5 $cm^3$ de chlorure de méthylène, refroidit à -20°C, ajoute 4,35 g de tétrabromométhane et 3,44 g de triphénylphosphine. On agite à +4°C pendant 4 heures, évapore le solvant, reprend le résidu dans du pentane, agite, filtre, lave l'insoluble au pentane, réunit les filtrats et évapore le solvant sous pression réduite. On obtient 3,22 g de produit attendu.

**EXEMPLE 88 : 11bêta-[4-[s-[(2-(pentafluorophényl) éthyl] sulfonyl] pentyloxy] phényl] estra-1,3,5(10)-triène-3,17bêta-diol et 11bêta-[4-[5-[(4-éthényl 2,3,5,6-tétrafluorophényl) sulfinyl] pentyloxy] phényl] estra-1,3,5(10)-triène-3,17bêta-diol.**

On opère comme à l'exemple 56 en utilisant au départ 420 mg de mélange obtenu à l'exemple 87 et 346 mg d'acide métachloroperbenzoïque. On obtient 443 mg de produit attendu sous forme de mélange. Après chromatographie sur silice (éluant : acétate d'éthyle-cyclohexane 5-5 puis acétone-chlorure de méthylène 1-9), on obtient 84 mg de dérivé pentafluoré et 201 mg de dérivé tétrafluoré.

Spectre IR ($CHCl_3$)

. dérivé pentafluoré

| | |
|---|---|
| OH ≈ | 3602 $cm^{-1}$ |
| aromatique : | 1656, 1610, 1580, 1522, 1508 $cm^{-1}$ |
| $SO^2$ ≈ | 1323 $cm^{-1}$ |

. dérivé tétrafluoré

| | |
|---|---|
| OH ≈ | 3600 $cm^{-1}$ |
| C=C : | 1648 $cm^{-1}$ |
| aromatique : | 1610, 1578, 1512, 1478 $cm^{-1}$ (F) |
| S -> O ≈ | 1055 $cm^{-1}$ |

**EXEMPLE 89 : 11bêta-[4-[5-[3-(pentafluorophényl) propyl] thio] pentyloxy] phényl] estra-1,3,5(10)-triène-3,17bêta-diol.**

On opère comme à l'exemple 85 en utilisant au départ 500 mg de disulfure obtenu comme à l'exemple 6Ba puis 2 $cm^3$ de solution chlorométhylénique de 3-iodopropyl pentafluoro benzène. On obtient 472 mg de produit attendu.

| | |
|---|---|
| Spectre IR | ($CHCl_3$) |
| OH ≈ | 3598 $cm^{-1}$ |
| aromatique : | 1655, 1610, 1580, 1521, 1512, 1504 $cm^{-1}$ |

**Préparation du 3-iodopropyl pentafluoro benzène.**

Stade A : Acide (2,3,4,5,6-pentafluorobenzène) propionique.

On ajoute 971 mg de rhodium chloro-tris-triphénylphosphine à une solution comprenant 2,5 g d'acide 2,3,4,5-pentafluorocinnamique, 34 $cm^3$ d'éthanol et 34 $cm^3$ de toluène puis hydrogène (1700 mbar) pendant 6 heures à température ambiante. On évapore le solvant, reprend le résidu dans du chlorure de méthylène, extrait avec une solution aqueuse de soude 1N, lave au chlorure de méthylène, acidifie à l'aide d'acide chlorhydrique, extrait au chlorure de

méthylène, sèche et évapore le solvant sous pression réduite. On cristallise le résidu dans du cyclohexane et obtient 2,06 g de produit attendu.

Stade B : 3-(pentafluorophényl) propanol.

On opère comme à la préparation de l'exemple 87 stade A en utilisant au départ 1,99 g d'acide obtenu ci-dessus et 2,4 $cm^3$ de complexe borohydrure-diméthylsulfure. On obtient 1,89 g de produit brut attendu que l'on chromatographie sur silice (éluant : acétate d'éthyle-cyclohexane 4-6).

Stade C : 3-iodopropyl pentafluoro benzène.

On refroidit à +10°C, 356 mg de triphénylphosphine dans 2 $cm^3$ de chlorure de méthylène, ajoute 95 mg d'imidazole, agite 15 minutes, refroidit à 0°C, ajoute 357 mg d'iode, agite 30 minutes en laissant revenir à température ambiante, ajoute 315 mg d'alcool obtenu au stade B, agite 4 heures et filtre. On lave la solution chlorométhylénique à l'aide d'une solution aqueuse de thiosulfate de sodium 0,2N puis à l'eau et obtient la solution chlorométhylénique de produit attendu utilisé telle quelle dans l'exemple 89.

**EXEMPLE 90 : 11bêta-[4-[5-[3-(pentafluorophényl) propylsulfonyl) pentyloxy] phényl] estra-1,3,5(10)-triène-3,17bêta-diol.**

On opère comme à l'exemple 56 en utilisant au départ 451 mg de produit obtenu à l'exemple 89 et 365 mg d'acide métachloroperbenzoïque. On obtient 305 mg de produit attendu après chromatographie sur silice (éluant : acétate d'éthyle-cyclo-hexane 6-4) et cristallisation dans l'éthanol. F = 130°C.

| | |
|---|---|
| Spectre IR | (Nujol) |
| | absorption complexe région OH |
| aromatique : | 1659, 1610, 1580, 1522, 1510, 1501$cm^{-1}$ |
| $SO_2$ : | 1358, 1130 $cm^{-1}$ |

**EXEMPLE 91 : 11bêta-[4-[5-[[2,3,5,6-tétrafluoro-4-(trifluorométhyl) phényl] méthylthio] pentyloxy] phényl] estra-1,3,5(10)-triène-3,17bêta-diol.**

On opère comme à l'exemple 85 en utilisant au départ 500 mg de disulfure obtenu comme à l'exemple 6Ba puis 857 mg de bromure d'alpha,alpha,alpha, 2,3,5,6-heptafluoro p-xylène. On obtient 259 mg de produit attendu.

| | |
|---|---|
| Spectre IR | ($CHCl_3$) |
| OH : | 3600 $cm^{-1}$ |
| aromatique : | 1664, 1610, 1578, 1512, 1500 $cm^{-1}$ |

**Préparation du bromure d'alpha,alpha,alpha, 2,3,5,6-hepta-fluoro p-xylène.**

On chauffe au reflux 2 g d'alpha,alpha,alpha, 2,3,5,6-hepta-fluoro p-xylène dans 7,4 $cm^3$ de tétrachlorure de carbone en présence de 15 mg d'azo isobutyronitrile et ajoute en 30 minutes 1,53 g de N-bromosuccinimide puis maintient le reflux pendant 96 heures. On refroidit, filtre, évapore le filtrat, reprend le résidu dans du pentane, évapore le solvant et obtient 1,40 g de produit utilisé tel quel à l'exemple 91.

**EXEMPLE 92 : 11bêta-[4-[[5-[[2,3,5,6-tétrafluoro-4-(trifluorométhyl) phényl] méthyl] sulfonyl] pentyloxy] phényl] estra-1,3,5(10)-triène-3,17bêta-diol.**

On opère comme à l'exemple 56 en utilisant au départ 241 mg de produit obtenu à l'exemple 91 et 193 mg d'acide métachloroperbenzoïque. On obtient 161 mg de produit attendu après chromatographie sur silice (éluant : acétate d'éthyle-cyclo-hexane 5-5).

| | |
|---|---|
| Spectre IR | ($CHCl_3$) |
| OH : | 3610 $cm^{-1}$ |
| aromatique : | 1622, 1611, 1578, 1511, 1504 $cm^{-1}$ |
| région | $CF_3 + SO_2$ : 1336 $cm^{-1}$ |

**EXEMPLE 93 : 3,17-diacétate de 11bêta-[9-[(4,4,5,5,5-pentafluoropentyl) sulfonyl] nonyl] estra-1,3,5(10)-triène-3,17bêta-diol.**

On agite 45 minutes à température ambiante 3,39 g de produit obtenu à l'exemple 61 dans 16,6 $cm^3$ de pyridine, 3,14 $cm^3$ d'anhydride acétique et 154 mg de diméthylaminopyridine. On ajoute 5,4 $cm^3$ de méthanol, agite 10 minutes, évapore les solvants, reprend dans l'acétate d'éthyle, lave avec une solution aqueuse d'acide chlorhydrique M, puis de chlorure de sodium, sèche et évapore le solvant. Après chromatographie du résidu sur silice (éluant : acétate d'éthyle-cyclohexane 5-5 puis 3-7), on obtient 3,25 g de produit attendu.

| | |
|---|---|
| Spectre IR | ($CHCl_3$) |
| C=O : | max. 1725 $cm^{-1}$ ep. 1746 $cm^{-1}$ |
| aromatique : | 1610, 1602, 1583, 1493 $cm^{-1}$ |
| $SO_2$ : | 1309, 1134 $cm^1$ |

**EXEMPLE 94 : 17-acétate de 11bêta-[9-[(4,4,5,5,5-pentafluoropentyl) sulfonyl] nonyl] estra-1,3,5 (10)-triène-3,17bêta-diol.**

On refroidit à 0°C 1,37 g de diacétate obtenu à l'exemple 93 dans 27,5 $cm^3$ de méthanol à 10% d'eau et ajoute 203 mg de bicarbonate de potassium, laisse revenir à température ambiante et agite pendant 19 heures. On verse dans 220 $cm^3$ d'eau additionnée de 55 $cm^3$ d'acide chlorhydrique 0,1N, extrait à l'acétate d'éthyle, sèche et évapore le solvant. On chromatographie le résidu sur silice (éluant : acétate d'éthyle-cyclohexane 35-65) puis cristallise le résidu dans un mélange chlorure de méthylène/éther isopropylique. On obtient 973 mg de produit attendu. F = 130°C.

| | |
|---|---|
| Spectre IR | ($CHCl_3$) |
| OH ≈ | 3600 $cm^{-1}$ + associé |
| OAC : | 1724, 1252 $cm^{-1}$ |
| aromatique : | 1611, 1585, 1498 $cm^{-1}$ |
| $SO_2$ : | 1308, 1134 $cm^{-1}$ |

**EXEMPLE 95 : Acétate de 3-méthoxy 11bêta-[9-[(4,4,5,5,5-pentafluoropentyl) sulfonyl] nonyl] estra-1,3,5(10)-triène-17bêta-ol.**

On ajoute 2,9 $cm^3$ d'eau à 1,05 g de phénol obtenu à l'exemple 94 en solution dans 3,9 $cm^3$ d'acétone, agite, ajoute 0,3 $cm^3$ de sulfate de méthyle et 0,83 $cm^3$ d'une solution aqueuse de soude 2N et agite 1 heure à température ambiante. On acidifie le milieu réactionnel avec 1 $cm^3$ d'acide chlorhydrique 2N, verse dans une solution aqueuse de bicarbonate de sodium, extrait à l'acétate d'éthyle, sèche et évapore le solvant. Après chromatographie du résidu sur silice (éluant : acétate d'éthyle-cyclohexane 3-7), on obtient 959 mg de produit attendu.

| | |
|---|---|
| Spectre IR | ($CHCl_3$) |
| C=O : | 1723 $cm^{-1}$ |
| aromatique : | 1609, 1575, 1500 $cm^{-1}$ |

**EXEMPLE 96 : 3,17-diacétate de 11bêta-[4-[5-[(4,4,5,5,5-pentafluoropentyl) thio] pentyloxy) estra-1,3,5(10)-triène-3,17bêta-diol.**

A 200 mg de produit obtenu à l'exemple 71 en solution dans 2,5 $cm^3$ d'acétate d'isopropényle, on ajoute 0,1 $cm^3$ d'acide sulfurique concentré en solution dans 2,5 $cm^3$ d'acétate d'isopropényle. On chauffe a 97°C, distille lentement le mélange acétone/acétate d'isopropényle, ajoute de nouveau la solution d'acide sulfurique et distille comme précédemment. On reprend le résidu dans une solution aqueuse de bicarbonate de sodium et extrait à l'éther. On sèche, évapore le solvant et obtient 510 mg de produit attendu utilisé tel quel pour l'exemple suivant.

**EXEMPLE 97 : 3-hydroxy 11bêta-[4-[5-[(4,4,5,5,5-pentafluoropentyl) thio] pentyloxy] phényl] 16alpha-bromo-estra-1,3,5(10)-triène-17-one.**

Au produit obtenu à l'exemple 96 en solution dans 3 $cm^3$ d'acide acétique, on ajoute goutte à goutte une solution comprenant 0,025 $cm^3$ de brome, 43 mg d'acétate de sodium et 5 $cm^3$ d'un mélange acide acétique-eau (40-1). On agite 12 heures à température ambiante, ajoute une solution aqueuse saturée en bicarbonate de sodium, extrait au chlorure de méthylène, sèche, chromatographie le résidu sur silice (éluant : acétate d'éthyle cyclohexane 3-7) et obtient

20 mg de produit attendu.

**<u>EXEMPLE 98 :</u> 3-hydroxy 11bêta-[4-[5-[(4,4,5,5,5-pentafluoropentyl) sulfonyl] pentyloxy] phényl] 16alpha-bromo-estra-1,3,5(10)-triène-17-one.**

On opère comme à l'exemple 56 en utilisant au départ 120 mg de produit obtenu à l'exemple 97 et 100 mg d'acide métachloroperbenzoïque. On obtient 38 mg de produit attendu.

| | |
|---|---|
| Spectre IR | ($CHCl_3$) |
| C=O : | 1750 $cm^{-1}$ |
| OH : | 3595 $cm^{-1}$ |
| aromatique : | 1611, 1580, 1512, 1502 $cm^{-1}$ |
| $SO_2$ : | 1305, 1133 $cm^{-1}$ |

**<u>EXEMPLE 99 :</u> 11bêta-(4-[5-[(4,4,5,5,5-pentafluoropentyl) sulfonyl] pentyloxy] phényl] l6alpha-bromo-estra-1,3,5(10)-triène-3,17bêta-diol.**

En opérant comme au stade D de l'exemple 3 à partir du produit obtenu à l'exemple 98, on a obtenu le produit attendu.

**<u>COMPOSITIONS PHARMACEUTIQUES</u> :**

On a préparé des comprimés répondant à la formule suivante :

- Produit de l'exemple 5 50 mg
- Excipient (talc, amidon, stéarate de magnésium) q.s. pour un comprimé terminé à 120 mg

<u>Etude pharmacologique des produits de l'invention</u>

**1 - Etude de l'activité des produits de l'invention sur le récepteur estrogène de l'utérus de souris :**

Des souris femelles impubères âgées de 18 à 21 jours sont sacrifiées, les utérus sont prélevés puis homogénéisés à 0°C à l'aide d'un Potter teflon-verre dans une solution tamponnée TS (Tris 10 mM, saccharose 0,25 M, HCl pH 7,4 (1 g de tissu pour 25 ml de TS). L'homogénat est ensuite ultracentrifugé (209 000 g x 30 mn) à 0°C. Des aliquotes du surnageant ainsi obtenu, sont incubées à 25°C pendant 5 heures avec une concentration constante (T) d'estradiol tritié en présence de concentration croissantes soit d'estradiol froid (0 - $1000.10^{-9}$ M), soit du produit froid à tester (1 à 25 000 x $10^{-9}$ M). La concentration d'estradiol tritié lié (B) est ensuite mesurée dans chaque incubat par la technique d'adsorption au charbon-dextran.
Calcul de l'affinité relative de liaison (ARL).

On trace les 2 courbes suivantes : pourcentage de l'hormone tritiée liée B/BO en fonction du logarithme de la concentration de l'hormone de référence froide ou en fonction du logarithme de la concentration du produit froid testé.

On détermine la droite d'équation suivante :

$$I_{50} = 100\ (B/BO + Bmin/BO)/2 \text{ soit } I_{50} = 100\ (1+Bmin/BO)/2 = 50\ (1+Bmin/BO).$$

BO = % de liaison de l'hormone tritiée liée en l'absence de tout produit froid.
B = % de l'hormone tritiée liée en présence d'une concentration X de produit froid.
B min = % de l'hormone tritiée liée en présence d'un grand excès de l'hormone froide de référence (500 nM).

Les intersections de la droit $I_{50}$ et des courbes, permettent d'évaluer les concentrations de l'hormone de référence froide (CH) et du produit froid testé (CX) qui inhibent de 50 % la liaison spécifique de l'hormone tritiée sur le récepteur.

L'affinité relative de liaison (ARL) du produit testé est déterminée par l'équation :

$$ARL = 100\ (CH)/(CX).$$

| Produits des exemples | Récepteur Oestrogène incubation 5H à 25°C |
|---|---|
| 5 | 21,2 |
| 7 | 26,3 |
| 32 | 56 |
| 60 | 18,2 |
| 78 | 21,8 |

**2 - Activité anti-proliférative des produits de l'invention sur la croissance de cellules tumorales mammaires MCF-7.**

Description du test :

a) Culture cellulaire :

Les lignées MCF-7 sont maintenues en culture en milieu SVF (d'après 1) à 37°C en atmosphère humide contenant 5 % $CO_2$. Les cellules à subconfluence sont récoltées par trypsination (trypsine 0,05 %, EDTA 0,02 %) puis rincées par centrifugation douce. Un échantillon des cellules en suspension est compté sur cellule de Malassez.

b) Etude de la croissance :

Les cellules resuspendues dans du milieu DSE (d'après 1) sont ensemencées à raison de 50 000 cellules par puits dans des plaques multipuits (24 puits de 2,5 $cm^2$). Vingt quatre heures après l'ensemencement (JO), le produit à tester est ajouté au milieu en solution éthanolique (concentration finale en éthanol : 0,1 %), à la concentration de $10^{-11}$ à $10^{-6}$M, les puits contrôles recevant la même concentration en éthanol. Les milieux sont renouvelés toutes les 48 heures. En fin d'expérience (J7 à J9), le milieu est aspiré et les cellules sont immédiatement fixées par 150 microlites de méthanol afin de doser l'ADN.

L'activité anti-proliférative des produits est évaluée par leur capacité à inhiber l'augmentation d'ADN.

**c) Dosage de l'ADN :**

L'ADN est dosé par une méthode fluorimétrique utilisant le DABA (Acide 3,5 diaminobenzoïque) (d'après 2) : 150 microlitres de DABA sont ajoutés dans chaque puits ; les plaques sont alors incubées 45 mn à 56°C, puis 2 ml d'HCL 1N sont ajoutés. La fluorescence est mesurée à l'aide d'un fluorimètre (longueur d'onde excitatrice : 408 nm, longueur d'onde d'émission : 510 nm).

La quantité d'ADN par puits est évaluée par rapport à une gamme étalon obtenue en traitant dans les mêmes conditions un standard d'ADN de thymus de veau.

Résultats :

La concentration en nM qui inhibe de 50 % la croissance des cellules $MCF_7$ ($CI_{50}$) a été déterminée de la manière indiquée ci-dessus :

Résultats :

| | |
|---|---|
| produit de l'exemple 1 | CI 50 = 0,024 nM |
| produit de l'exemple 5 | CI 50 = 0,012 nM |
| produit de l'exemple 7 | CI 50 = 0,026 nM |
| produit de l'exemple 32 | CI 50 = 0,1 nM |
| produit de l'exemple 60 | CI 50 = 0,25 nM |
| produit de l'exemple 78 | CI 50 = 0,03 nM |

(1) Un milieu de base est préparé comme suit :
Milieu MEM (Minimal Essential Medium) auquel sont ajoutés :

- acides aminés non essentiels (GIBCO) 1 %,
- péni-strepto (pénicilline 100 U/ml, streptomycine 0,1 mg/ml),
- fungizone 0,1 %,
- glutamine 2 mM,
- bicarbonate de sodium 2,25 mg/ml.

Le milieu SVF est composé de 95 % de milieu de base et 5 % de sérum de veau foetal.

Le milieu DSE est composé de 95 % de milieu de base, 5 % de sérum de veau foetal déstéroïdé sur charbon-dextran et $10^{-10}$ M d'oestradiol.

(2) Puzas et Goodman, Analytical Biochemistry, Vol 86, p. 50, 1978.

## Revendications

1. Les composés de formule (I) :

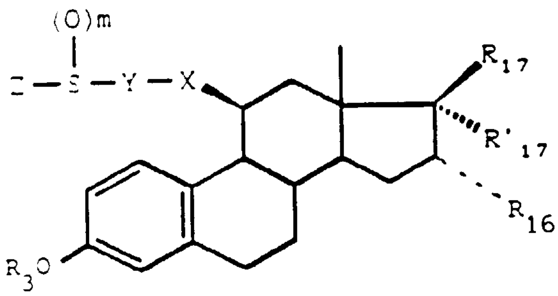

(I)

dans laquelle $R_{17}$ et $R'_{17}$ sont tels que :

- soit $R_{17}$ et $R'_{17}$ forment ensemble une fonction cétone, une fonction oxime, une fonction hydrazono ou un radical méthylène,
- soit $R_{17}$ est un radical hydroxyle, un radical hydroxyméthyle ou un radical acyloxy ayant au plus 12 atomes de carbone et $R'_{17}$ représente un atome d'hydrogène, un radical alkyle, alcényle ou alcynyle ayant au plus 8 atomes de carbone, chacun de ces substituants étant éventuellement substitué,
- $R_3$ représente un atome d'hydrogène, un radical alkyle linéaire ou ramifié ou un radical alkyle cyclique ayant au plus 8 atomes de carbone ou un radical acyle ayant au plus 12 atomes de carbone,
- $R_{16}$ représente un atome d'hydrogène, un atome d'halogène ou un radical alkyle ayant au plus 8 atomes de carbone,

m a la valeur 0, 1 ou 2,
X, Y et Z sont tels que :

- X représente un radical méthylène, un groupement arylène ou arylènoxy ayant au plus 10 atomes de carbone lié au stéroïde par un atome de carbone,
- Y représente une chaîne aliphatique linéaire ou ramifiée, saturée ou insaturée, renfermant de 1 à 18 atomes de carbone, éventuellement interrompue par un atome d'oxygène,
- Z représente :

un radical alkyle linéaire ou ramifié, renfermant de 1 à 8 atomes de carbone et éventuellement substitué ou un radical aryle ou arylalkyle, chacun de ces radicaux étant éventuellement substitué, dans lesquels le radical alkyle renferme au plus 6 atomes de carbone et le radical aryle représente un radical monocyclique comprenant 5 ou 6 chaînons ou un radical constitué de cycles condensés comprenant 8 à 10 chaînons, ces radicaux renfermant éventuellement 1 ou plusieurs hétéroatomes choisis parmi les atomes d'oxygène, d'azote ou de soufre,
les radicaux alkyle que peuvent représenter $R'_{17}$ et Z, les radicaux alcényle ou alcynyle que peut représenter $R'_{17}$ et les radicaux aryle ou arylalkyle que peut représenter Z étant éventuellement substitués par un ou plusieurs radicaux choisis parmi les radicaux :

- halogènes,
- amino, alkylamino ou dialkylamino dans lesquels le ou les radicaux alkyle ont de 1 à 4 atomes de carbone,
- hydroxyle,
- carboxy libres, estérifiés ou salifiés,
- alkyle ayant de 1 à 8 atomes de carbone, éventuellement substitué par un ou plusieurs atomes d'halogène,
- oxo, cyano, nitro,
- acyle ou acyloxy ayant au plus 12 atomes de carbone,
- alkoxy ou alkylthio ayant de 1 à 4 atomes de carbone,
- alcényle ou alcynyle ayant au plus 4 atomes de carbone,
- aryle tel que défini ci-dessus,

et les sels d'addition de ceux-ci.

**2.** Les composés de formule (I) telle que définie à la revendication 1, pour lesquels $R_{17}$ est un radical hydroxyle et $R'_{17}$ est un atome d'hydrogène ou un radical méthyle.

**3.** Les composés de formule (I) telle que définie à la revendication 1 ou 2 pour lesquels X représente un radical méthylène et Y est une chaîne linéaire saturée renfermant de 7 à 9 atomes de carbone.

**4.** Les composés de formule (I) telle que définie à la revendication 1 ou 2 pour lesquels X représente un radical phénylène et Y représente une chaîne linéaire saturée ou insaturée renfermant de 3 à 8 atomes de carbone, étant entendu que, lorsque la chaîne est insaturée, elle comporte un groupement vinylène ou éthynylène lié directement au radical phénylène.

**5.** Les composés de formule (I) telle que définie à la revendication 1 ou 2 pour lesquels X représente un radical phénylènoxy et Y représente une chaîne linéaire saturée renfermant de 4 à 7 atomes de carbone éventuellement interrompue par un atome d'oxygène.

**6.** Les composés de formule (I) telle que définie à l'une quelconque des revendications 1 à 5 pour lesquels Z représente un radical alkyle linéaire renfermant de 1 à 5 atomes de carbone, un radical trifluoroalkyle renfermant de 2 à 4 atomes de carbone, un radical pentafluoroalkyle renfermant 4 ou 5 atomes de carbone, un radical nonafluoroalkyle renfermant de 1 à 4 atomes de carbone,

- un radical comprenant un radical phényle substitué choisi parmi :

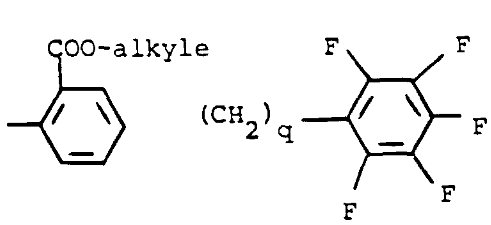

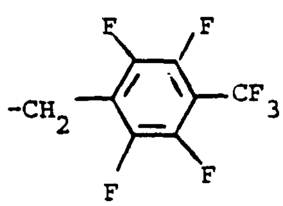

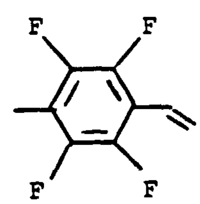

dans lequel alkyle représente un radical alkyle renfermant de 1 à 4 atomes de carbone et q représente les valeurs 1, 2 ou 3.

- un radical comprenant un hétérocycle à 5 chaînons choisi parmi :

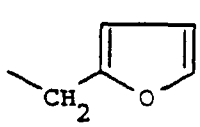

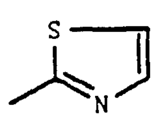

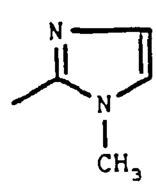

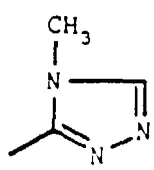 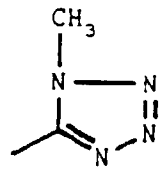

- un radical comprenant un hétérocycle à 6 chaînons choisi parmi :

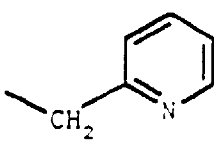 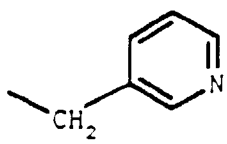 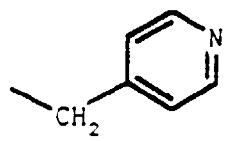 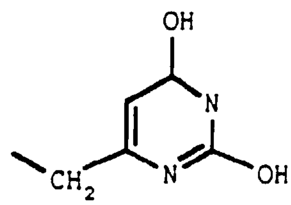

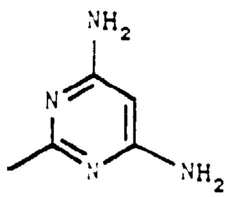 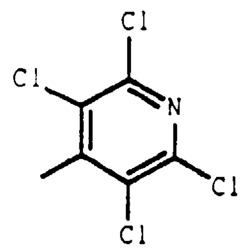

- un radical comprenant un hétérocycle à deux cycles condensés choisi parmi :

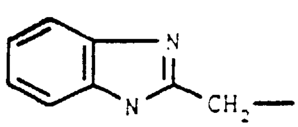 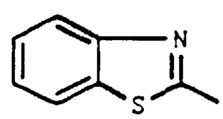 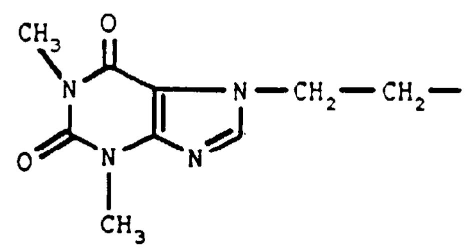

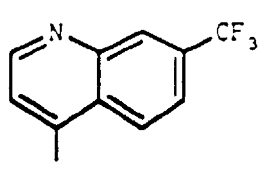

**7.** Les composés de formule (I) telle que définie à l'une quelconque des revendications 1 à 6 dont les noms suivent :

le 11béta-[8-[(2-pyridinylméthyl) thio] octyl] estra-1,3,5(10)-triène-3,17béta-diol,
le 11béta-[4-[3-[(1-méthyl 1H-imidazol-2-yl) thio] 1-propynyl] phényl] estra-1,3,5(10)-triène-3,17béta-diol,
le 11béta-[4-[5-[(2-furanylméthyl) thio] pentyloxy] phényl] estra-1,3,5(10)-triène-3,17béta-diol,
le 11béta-[4-[5-[(2-pyridinylméthyl) sulfinyl] pentyloxy] phényl] estra-1,3,5(10)-triène-3,17béta-diol,
le 11béta-[4-[5-[(3-pyridinylméthyl) sulfinyl] pentyloxy] phényl] estra-1,3,5(10)-triène-3,17béta-diol,
le 11béta-[4-[6-[(4,4,5,5,5-pentafluoro pentyl) sulfinyl] hexyloxy] phényl] estra-1,3,5(10)-triène-3,17béta-diol,

le 11béta-[4-[5-[(4,4,5,5,5-pentafluoro pentyl) sulfonyl] pentyloxy] phényl] estra-1,3,5(10)-triène-3,17béta-diol,
le 11béta-[4-[5-(pentylsulfonyl] pentyloxy] phényl] estra-1,3,5(10)-triène-3,17béta-diol,
le 11béta-[4-[5-[(4,4,5,5,5-pentafluoro pentyl) sulfonyl] pentyloxy] phényl] 17alpha-méthyl estra-1,3,5(10)-triène-3,17béta-diol.

**8.** Procédé de préparation des composés de formule (I) telle que définie à la revendication 1, caractérisé en ce que l'on soumet un composé de formule (II) :

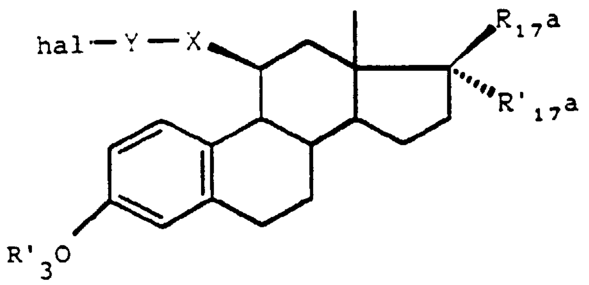

(II)

dans laquelle :

- $R_{17a}$ et $R'_{17a}$ ont les significations indiquées à la revendication 1 pour $R_{17}$ et $R'_{17}$ et dans lesquelles les éventuelles fonctions réactives sont éventuellement protégées,
- X et Y ont la même signification qu'à la revendication 1,
- $R'_3$ représente un atome d'hydrogène ou un groupement protecteur de la fonction hydroxyle,
- hal représente un atome d'halogène ou un groupement pseudohalogène tel qu'un tosylate,

à l'action d'un mercaptan de formule (III) :

Za-SH (III)

ou un sel de celui-ci, dans laquelle Za a la signification indiquée à la revendication 1 pour Z dans lequel les éventuelles fonctions réactives sont éventuellement protégées, puis, le cas échéant, à l'action d'un agent d'élimination des groupements protecteurs, pour obtenir le composé de formule (IA) correspondant au produit de formule (I) dans laquelle m = 0, produit de formule (IA) que l'on soumet, si désiré et si nécessaire, à l'une quelconque des réactions suivantes, dans un ordre quelconque :

- réduction de la fonction cétone que peuvent représenter ensemble $R_{17}$ et $R'_{17}$,
- addition sur la fonction cétone que peut représenter $R_{17}$ et $R'_{17}$ d'un complexe métallique de formule (IV) :

  $M\text{-}R'_{17a}$ (IV)

  dans laquelle M représente un atome métallique et $R'_{17a}$ a la même signification que précédemment, étant entendu qu'il ne s'agit pas d'un atome d'hydrogène,
- transformation de la fonction cétone que peut représenter ensemble $R_{17}$ et $R'_{17}$ en fonction oxime, en groupement hydrazono ou en groupement méthylène,
- acylation sélective en position 17 lorsque $R_{17}$ est un hydroxyle,
- halogénation ou alkylation en position 16,
- alkylation ou acylation du radical hydroxyle en position 3,
- réduction partielle ou totale du groupement éthynylène, lorsque Y représente une chaîne insaturée,
- oxydation du soufre en sulfoxyde ou en sulfone,
- salification éventuelle par un acide ou une base.

**9.** Procédé de préparation des composés de formule (I) telle que définie à la revendication 1, caractérisé en ce que l'on soumet un composé de formule (V) :

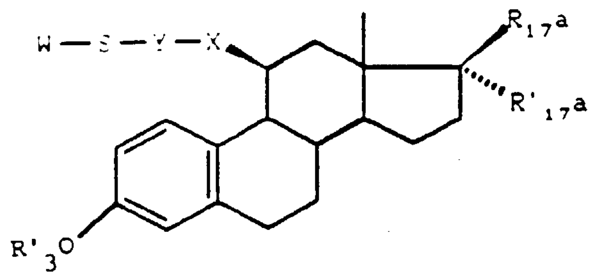

(V)

dans laquelle :

- $R_{17a}$ et $R'_{17a}$ ont les significations indiquées à la revendication 1 pour $R_{17}$ et $R'_{17}$ dans lesquelles les éventuelles fonctions réactives sont éventuellement protégées,
- X et Y ont la même signification qu'à la revendication 1,
- $R'_3$ représente un atome d'hydrogène ou un groupement protecteur de la fonction hydroxyle,
- W représente un atome d'hydrogène ou un radical acyle -COR dans lequel R représente un radical alkyle qui comporte de 1 à 5 atomes de carbone,

à l'action d'un composé de formule (VI) :

Za-hal' (VI)

ou un sel de celui-ci, dans laquelle Za a la signification indiquée à la revendication 1 pour Z dans lequel les éventuelles fonctions réactives sont éventuellement protégées et hal' représente un groupement réactif tel qu'un halogène ou un pseudohalogène tel qu'un groupement mésylate ou tosylate, en présence d'une base, puis le cas échéant à l'action d'un agent d'élimination des groupements protecteurs, pour obtenir le composé de formule (IA) ayant la même définition qu'à la revendication 8 et que, si désiré et si nécessaire, on soumet à l'une quelconque des réactions indiquées pour le composé de formule (IA) à la revendication 8.

**10.** A titre de médicament, les composés de formule (I) telle que définie à l'une quelconque des revendications 1 à 6.

**11.** A titre de médicament, les composés de formule (I) définis à la revendication 7.

**12.** Les compositions pharmaceutiques renfermant comme principe actif au moins l'un des médicaments définis à la revendication 10 ou 11.

**Patentansprüche**

**1.** Verbindungen der allgemeinen Formel (I)

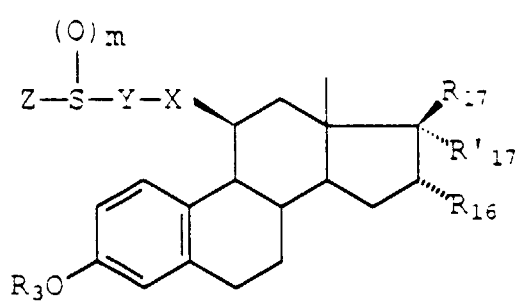

(I)

in der $R_{17}$ und $R'_{17}$ bedeuten:

- entweder bilden $R_{17}$ und $R'_{17}$ zusammen eine Ketonfunktion, eine Oximfunktion, eine Hydrazonofunktion oder

einen Rest Methylen,

- oder $R_{17}$ ist ein Rest Hydroxyl, ein Rest Hydroxymethyl oder ein Rest Acyloxy mit höchstens 12 Kohlenstoffatomen und $R'_{17}$ stellt ein Wasserstoffatom, einen Rest Alkyl, Alkenyl oder Alkinyl mit höchstens 8 Kohlenstoffatomen dar, wobei jeder dieser Substituenten gegebenenfalls substituiert sein kann,
- $R_3$ bedeutet ein Wasserstoffatom, einen geraden oder verzweigten Rest Alkyl oder einen Rest cyclisches Alkyl mit höchstens 8 Kohlenstoffatomen oder einen Rest Acyl mit höchstens 12 Kohlenstoffatomen,
- $R_{16}$ stellt ein Wasserstoffatom, ein Halogenatom oder einen Rest Alkyl mit höchstens 8 Kohlenstoffatomen dar,

m besitzt den Wert 0, 1 oder 2,
X, Y und Z bedeuten:

- X stellt einen Rest Methylen, eine Gruppe Arylen oder Arylenoxy mit höchstens 10 Kohlenstoffatomen dar, die mit dem Steroid durch ein Kohlenstoffatom verbunden sind,
- Y stellt eine gerade oder verzweigte, gesättigte oder ungesättigte aliphatische Kette mit 1 bis 18 Kohlenstoffatomen dar, gegebenenfalls unterbrochen durch ein Sauerstoffatom,
- Z stellt einen geraden oder verzweigten und gegebenenfalls substituierten Rest Alkyl mit 1 bis 8 Kohlenstoffatomen dar oder einen Rest Aryl oder Arylalkyl, wobei jeder dieser Reste gegebenenfalls substituiert sein kann, und worin der Rest Alkyl höchstens 6 Kohlenstoffatome umfaßt und der Rest Aryl einen monocyclischen Rest mit 5 oder 6 Ringgliedern oder einen Rest darstellt, der aus kondensierten Ringen mit 8 bis 10 Ringgliedern besteht, wobei diese Reste gegebenenfalls ein oder mehrere Heteroatome umfassen, ausgewählt unter den Atomen von Sauerstoff, Stickstoff oder Schwefel,

und die Reste Alkyl, die $R'_{17}$ und Z darstellen können, die Reste Alkenyl oder Alkinyl, die $R'_{17}$ sein kann und die Reste Aryl oder Arylalkyl, die Z bedeuten kann, gegebenenfalls durch einen oder mehrere Reste substituiert sind, ausgewählt unter den Resten:

- Halogene,
- Amino, Alkylamino oder Dialkylamino, worin der oder die Reste Alkyl 1 bis 4 Kohlenstoffatome besitzen,
- Hydroxyl,
- freies, verestertes oder in Salz überführtes Carboxy,
- Alkyl mit 1 bis 8 Kohlenstoffatomen, gegebenenfalls substituiert durch ein oder mehrere Halogenatome,
- Oxo, Cyano, Nitro,
- Acyl oder Acyloxy mit höchstens 12 Kohlenstoffatomen,
- Alkoxy oder Alkylthio mit 1 bis 4 Kohlenstoffatomen,
- Alkenyl oder Alkinyl mit höchstens 4 Kohlenstoffatomen,
- Aryl wie oben definiert,

und deren Additionssalze.

**2.** Verbindungen der Formel (I) wie in Anspruch 1 definiert, worin $R_{17}$ ein Rest Hydroxyl ist und $R'_{17}$ ein Wasserstoffatom oder einen Rest Methyl darstellt.

**3.** Verbindungen der Formel (I) wie in Anspruch 1 oder 2 definiert, worin X ein Rest Methylen ist und Y eine gerade gesättigte Kette mit 7 bis 9 Kohlenstoffatomen darstellt.

**4.** Verbindungen der Formel (I) wie in Anspruch 1 oder 2 definiert, worin X ein Rest Phenylen ist und Y eine gerade gesättigte oder ungesättigte Kette mit 3 bis 8 Kohlenstoffatomen darstellt, mit der Maßgabe, daß im Fall einer gesättigt Kette diese eine Gruppe Vinylen oder Ethinylen umfaßt, die direkt mit dem Rest Phenylen verbunden ist.

**5.** Verbindungen der Formel (I) wie in Anspruch 1 oder 2 definiert, worin X ein Rest Phenylenoxy ist und Y eine gerade gesättigte Kette mit 4 bis 7 Kohlenstoffatomen darstellt, gegebenenfalls unterbrochen durch ein Sauerstoffatom.

**6.** Verbindungen der Formel (I) wie in irgendeinem der Ansprüche 1 bis 5 definiert, worin Z darstellt:
einen geraden Rest Alkyl mit 1 bis 5 Kohlenstoffatomen, einen Rest Trifluoralkyl mit 2 bis 4 Kohlenstoffatomen, einen Rest Pentafluoralkyl mit 4 oder 5 Kohlenstoffatomen, einen Rest Nonafluoralkyl mit 1 bis 4 Kohlenstoffatomen,

- einen Rest, der einen substituierten Rest Phenyl umfaßt, ausgewählt unter:

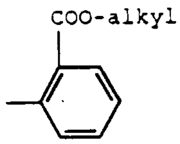
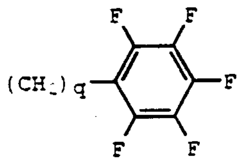
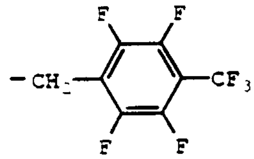
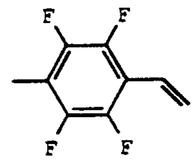

worin alkyl einen Rest Alkyl mit 1 bis 4 Kohlenstoffatomen darstellt und q die Werte 1, 2 oder 3 besitzt;

- einen Rest, der einen Heterocyclus mit 5 Ringgliedern umfaßt, ausgewählt unter:

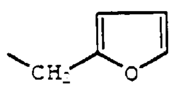
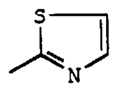
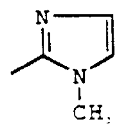
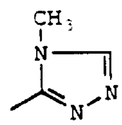
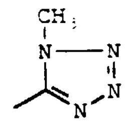

- einen Rest, der einen Heterocyclus mit 6 Ringgliedern umfaßt, ausgewählt unter:

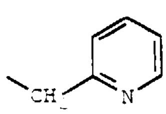
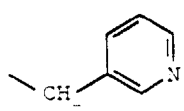
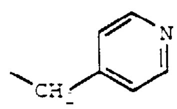
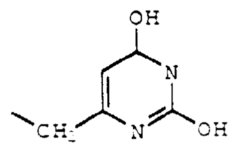

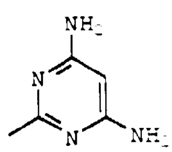
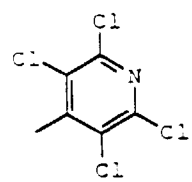

- einen Rest, der einen Heterocyclus mit zwei kondensierten Ringen umfaßt, ausgewählt unter:

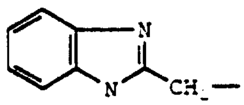
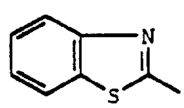
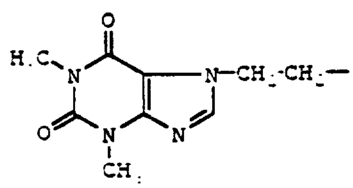
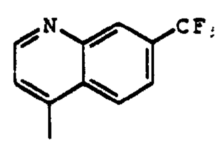

**7.** Verbindungen der Formel (I) wie in irgendeinem der Ansprüche 1 bis 6 definiert, deren Namen folgen:

11beta-{8-[(2-Pyridinylmethyl)-thio]-octyl}-estra-1,3,5 (10)-trien-3,17beta-diol,
11beta-{4-[3-[(1-Methyl-1H-imidazol-2-yl)-thio]-1-propinyl]phenyl}-estra-1,3,5(10)-trien-3,17beta-diol,
11beta-{4-[5-[(2-Furanylmethyl)-thio]-pentyloxyl-phenyl}-estra-1,3,5(10)-trien-3,17beta-diol,
11beta-{4-[5-[(2-Pyridinylmethyl)-sulfinyl]-pentyloxy]-phenyl}estra-1,3,5(10)-trien-3,17beta-diol,
11beta-{4-[5-[(3-Pyridinylmethyl)-sulfinyl]-pentyloxy]-phenyl}estra-1,3,5(10)-trien-3,17beta-diol,
11beta-{4-[6-[(4,4,5,5,5-Pentafluorpentyl)-sulfinyl]-hexyloxy]phenyl}-estra-1,3,5(10)-trien-3,17beta-diol,
11beta-{4-[5-[(4,4, 5,5,5-Pentafluorpentyl)-sulfonyl]-pentyloxy]phenyl}-estra-1,3,5(10)-trien-3,17beta-diol,
11beta-{4-[5-(Pentylsulfonyl)-pentyloxy]-phenyl}-estra-1,3,5(10)trien-3,17beta-diol,
11beta-{4-[5-[(4,4,5, 5,5-Pentafluorpentyl)-sulfonyl]-pentyloxy]phenyl}-17alpha-methyl-estra-1,3,5(10)-trien-3,17beta-diol.

**8.** Verfahren zur Herstellung von Verbindungen der Formel (I) wie in Anspruch 1 definiert, dadurch gekennzeichnet, daß man eine Verbindung der Formel (II)

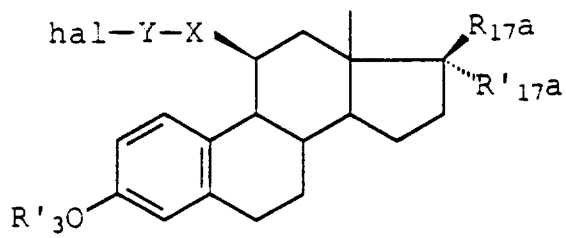

(II)

in der

- $R_{17}a$ und $R'_{17}a$ die gleichen Bedeutungen wie in Anspruch 1 für $R_{17}$ und $R'_{17}$ angegeben besitzen und in denen die eventuellen reaktiven Funktionen gegebenenfalls geschützt sind,
- X und Y die gleichen Bedeutungen wie in Anspruch 1 besitzen,
- $R'_3$ ein Wasserstoffatom oder eine Schutzgruppe für die Hydroxylfunktion darstellt,
- hal ein Halogenatom oder eine Gruppe Pseudotalogen, wie ein Tosylat bedeutet,

der Einwirkung eines Mercaptans der Formel (III)

$$Za\text{-}SH \qquad (III)$$

oder einem Salz von diesem unterzieht, worin Za die in Anspruch 1 für Z angegebene Bedeutung besitzt und worin die eventuellen reaktiven Funktionen gegebenenfalls geschützt sind, und man anschließend gegebenenfalls der Einwirkung eines Mittels zur Entfernung der Schutzgruppen unterwirft, um die dem Produkt der Formel (I) entsprechende Verbindung der Formel (IA) zu erhalten, in der m = 0 ist, und man dieses Produkt der Formel (IA), wenn erwünscht und wenn notwendig, irgendeiner der folgenden Reaktionen in irgendeiner Reihenfolge unterzieht:

- Reduktion der Ketonfunktion, die $R_{17}$ und $R'_{17}$ gemeinsam darstellen können,
- Addition an der Ketonfunktion, die $R_{17}$ und $R'_{17}$ darstellen können, eines Metallkomplexes der Formel (IV)

$$M\text{-}R'_{17}a \qquad (IV)$$

  worin M ein Metallatom darstellt und $R'_{17}a$ die gleiche Bedeutung wie vorstehend besitzt, mit der Maßgabe, daß es sich nicht um ein Wasserstoffatom handelt,
- Umwandlung der Ketonfunktion, die $R_{17}$ und $R'_{17}$ gemeinsam darstellen können, in eine Oximfunktion, eine Gruppe Hydrazono oder eine Gruppe Methylen,
- selektive Acylierung in Position 17, wenn $R_{17}$ ein Hydroxyl ist,
- Halogenierung oder Alkylierung in Position 16,
- Alkylierung oder Acylierung des Restes Hydroxyl in Position 3,
- partielle oder vollständige Reduktion der Gruppe Ethinylen, wenn Y eine ungesättigte Kette darstellt,
- Oxidation des Schwefels zum Sulfoxid oder Sulfon,
- gegebenenfalls Bildung eines Salzes durch eine Säure oder eine Base.

**9.** Verfahren zur Herstellung von Verbindungen der Formel (I) wie in Anspruch 1 definiert, dadurch gekennzeichnet, daß man eine Verbindung der Formel (V)

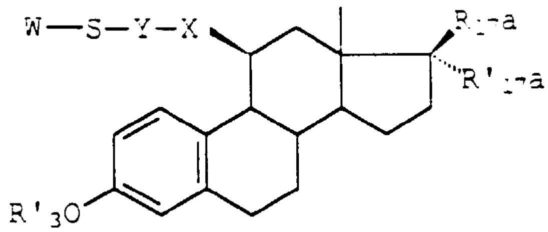

(V)

in der

- $R_{17}a$ und $R'_{17}a$ die gleichen Bedeutungen wie in Anspruch 1 für $R_{17}$ und $R'_{17}$ angegeben besitzen und in denen die eventuellen reaktiven Funktionen gegebenenfalls geschützt sind,
- X und Y die gleichen Bedeutungen wie in Anspruch 1 besitzen,
- $R'_3$ ein Wasserstoffatom oder eine Schutzgruppe für die Hydroxylfunktion darstellt,
- W ein Wasserstoffatom oder einen Rest Acyl -COR bedeutet, worin R einen Rest Alkyl bedeutet, der 1 bis 5 Kohlenstoffatome umfaßt, der Einwirkung einer Verbindung der Formel (VI)

Za-hal' (VI)

oder einem Salz von dieser in Anwesenheit einer Base unterzieht, worin Za die in Anspruch 1 für Z angegebene Bedeutung besitzt und worin die eventuellen reaktiven Funktionen gegebenenfalls geschützt sind, und

hal' eine reaktive Gruppe wie ein Halogen oder ein Pseudohalogen, beispielsweise eine Gruppe Mesylat oder Tosylat, darstellt, und man anschließend gegebenenfalls der Einwirkung eines Mittels zur Entfernung der Schutzgruppen unterwirft, um die Verbindung der Formel (IA) mit der gleichen Bedeutung wie in Anspruch 8 angegeben zu erhalten, die man, wenn erwünscht und wenn notwendig, irgendeiner der bei der Formel (IA) in Anspruch 8 angegebenen Reaktionen unterzieht.

10. Als Medikament die Verbindungen der Formel (I) wie in irgendeinem der Ansprüche 1 bis 6 definiert.

11. Als Medikament die Verbindungen der Formel (I) wie in Anspruch 7 definiert.

12. Pharmazeutische Zusammensetzungen, die als Wirkstoff mindestens eines der in Anspruch 10 oder 11 definierten Medikamente umfassen.

## Claims

1. The compounds of formula (I):

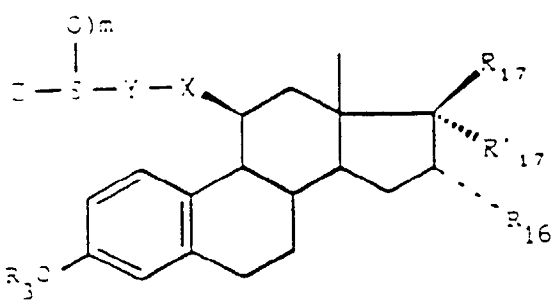

(I)

in which $R_{17}$ and $R'_{17}$ are such that:

- either $R_{17}$ and $R'_{17}$ form together a ketone function, an oxime function, a hydrazono function or a methylene

radical,

- or $R_{17}$ is a hydroxyl radical, a hydroxymethyl radical or an acyloxy radical having at most 12 carbon atoms and $R'_{17}$ represents a hydrogen atom, an alkyl, alkenyl or alkynyl radical having at most 8 carbon atoms, each of these substituents being optionally substituted,
- $R_3$ represents a hydrogen atom, a linear or branched alkyl radical or a cyclic alkyl radical having at most 8 carbon atoms or an acyl radical having at most 12 carbon atoms,
- $R_{16}$ represents a hydrogen atom, a halogen atom or an alkyl radical having at most 8 carbon atoms,

m has the value 0, 1 or 2,
X, Y and Z are such that:

- X represents a methylene radical, an arylene or arylenoxy group having at most 10 carbon atoms linked to the steroid by a carbon atom,
- Y represents a saturated or unsaturated, linear or branched aliphatic chain containing 1 to 18 carbon atoms, optionally interrupted by an oxygen atom,
- Z represents:

a linear or branched alkyl radical containing 1 to 8 carbon atoms and optionally substituted or an aryl or arylalkyl radical, each of these radicals being optionally substituted, in which the alkyl radical contains at most 6 carbon atoms and the aryl radical represents a monocyclic radical containing 5 or 6 members or a radical constituted by condensed rings containing 8 to 10 members, these radicals optionally containing 1 or more heteroatoms chosen from oxygen, nitrogen or sulphur atoms,
the alkyl radicals that can be represented by $R'_{17}$ and Z, the alkenyl or alkynyl radicals that can be represented by $R'_{17}$ and the aryl or arylalkyl radicals that can be represented by Z being optionally substituted by one or more radicals chosen from the following radicals:

- halogens,
- amino, alkylamino or dialkylamino in which the alkyl radical or radicals have 1 to 4 carbon atoms,
- hydroxyl,
- free, esterified or salified carboxy,
- alkyl having 1 to 8 carbon atoms, optionally substituted by one or more halogen atoms,
- oxo, cyano, nitro,
- acyl or acyloxy having at most 12 carbon atoms,
- alkoxy or alkylthio having 1 to 4 carbon atoms,
- alkenyl or alkynyl having at most 4 carbon atoms,
- aryl as defined above,

and the addition salts of the above radicals.

**2.** The compounds of formula (I) as defined in claim 1, in which $R_{17}$ is a hydroxyl radical and $R'_{17}$ is a hydrogen atom or a methyl radical.

**3.** The compounds of formula (I) as defined in claim 1 or 2 in which X represents a methylene radical and Y is- a saturated linear chain containing 7 to 9 carbon atoms.

**4.** The compounds of formula (I) as defined in claim 1 or 2 in which X represents a phenylene radical and Y represents a saturated or unsaturated linear chain containing 3 to 8 carbon atoms, it being understood that, when the chain is unsaturated, it contains a vinylene or ethynylene group linked directly to the phenylene radical.

**5.** The compounds of formula (I) as defined in claim 1 or 2 in which X represents a phenylenoxy radical and Y represents a saturated linear chain containing 4 to 7 carbon atoms optionally interrupted by an oxygen atom.

**6.** The compounds of formula (I) as defined in any one of claims 1 to 5 in which Z represents a linear alkyl radical containing 1 to 5 carbon atoms, a trifluoroalkyl radical containing 2 to 4 carbon atoms, a pentafluoroalkyl radical containing 4 or 5 carbon atoms, a nonafluoroalkyl radical containing 1 to 4 carbon atoms,

- a radical containing a substituted phenyl radical chosen from:

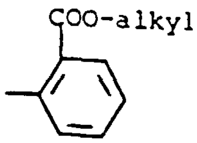 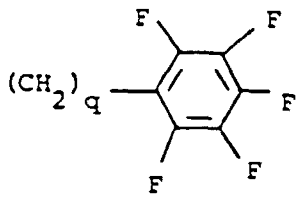 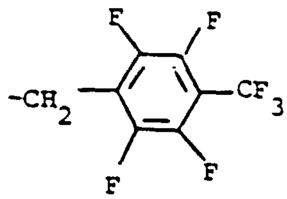 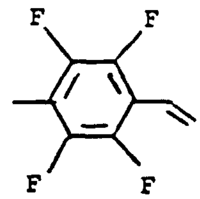

in which alkyl represents an alkyl radical containing 1 to 4 carbon atoms and q represents the values 1, 2 or 3;

- a radical containing a heterocycle with 5 members chosen from:

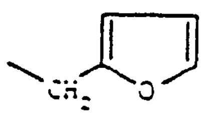 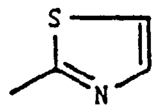 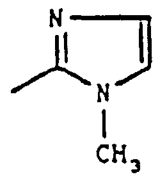

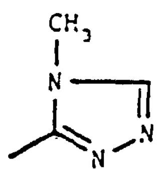 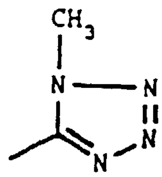

- a radical containing a heterocycle with 6 members chosen from:

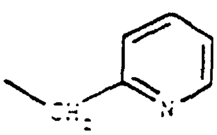 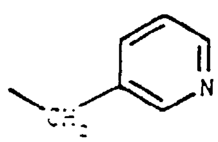 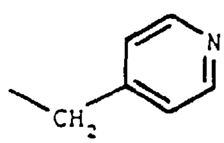 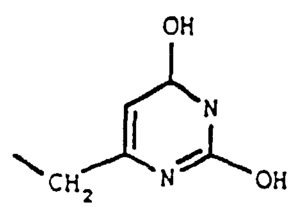

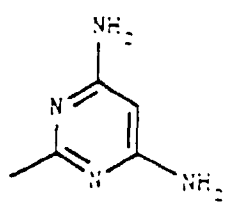 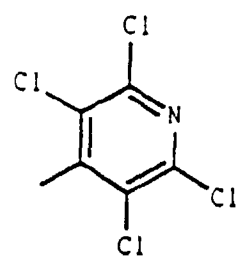

- a radical containing a heterocycle with two condensed rings chosen from:

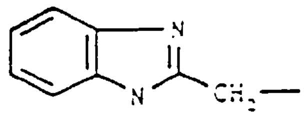
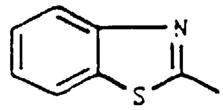
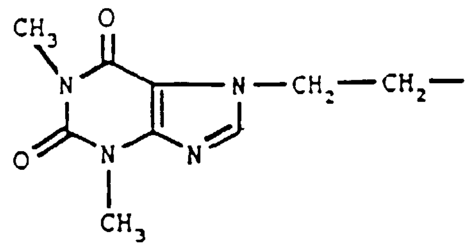
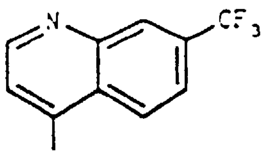

7. The compounds of formula (I) as defined in any one of claims 1 to 6 of which the names follow:

11beta-[8-[(2-pyridinylmethyl) thio] octyl] estra-1,3,5(10)-triene-3,17beta-diol,
11beta-[4-[3-[(1-methyl 1H-imidazol-2-yl) thio] 1-propynyl] phenyl] estra-1,3,5(10)-triene-3,17beta-diol,
11beta-[4-[5-[(2-furanylmethyl) thio] pentyloxy] phenyl] estra-1,3,5(10)-triene-3,17beta-diol,
11beta-[4-[5-[(2-pyridinylmethyl) sulphinyl] pentyloxy] phenyl] estra-1,3,5(10)-triene-3,17beta-diol,
11beta-[4-[5-[(3-pyridinylmethyl) sulphinyl] pentyloxy] phenyl] estra-1,3,5(10)-triene-3,17beta-diol,
11beta-[4-[6-[(4,4,5,5,5-pentafluoro pentyl) sulphinyl] hexyloxy] phenyl] estra-1,3,5(10)-triene-3,17beta-diol,
11beta-[4-[5-[(4,4,5,5,5-pentafluoro pentyl) sulphonyl] pentyloxy] phenyl] estra-1,3,5(10)-triene-3,17beta-diol,
11beta-[4-[5-(pentylsulphonyl] pentyloxy] phenyl] estra-1,3,5(10)-triene-3,17beta-diol,
11beta-[4-[5-[(4,4,5,5,5-pentafluoro pentyl) sulphonyl] pentyloxy] phenyl] 17alpha-methyl estra-1,3,5(10)-triene-3,17beta-diol.

8. Preparation process for the compounds of formula (I) as defined in claim 1, characterized in that a compound of formula (II):

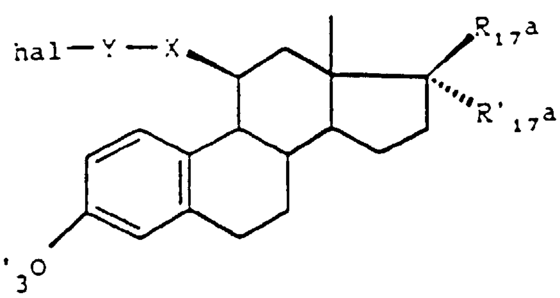

(II)

in which:

- $R_{17a}$ and $R'_{17a}$ have the meanings indicated in claim 1 for $R_{17}$ and $R'_{17}$ and in which the optional reactive functions are optionally protected,
- X and Y have the same meaning as in claim 1,
- $R'_3$ represents a hydrogen atom or a protective group of the hydroxyl function,
- hal represents a halogen atom or a pseudohalogen group such as a tosylate,

is subjected to the action of a mercaptan of formula (III):

Za-SH (III)

or a salt of the latter, in which Za has the meaning indicated in claim 1 for Z in which the optional reactive functions are optionally protected, then, if appropriate, is subjected to the action of an elimination agent of the protective

groups, in order to obtain the compound of formula (IA) corresponding to the product of formula (I) in which m = 0, which product of formula (IA) is subjected, if desired and if necessary, to any one of the following reactions, in any order:

- reduction of the ketone function that can be represented by $R_{17}$ and $R'_{17}$ together,
- addition to the ketone function that can be represented by $R_{17}$ and $R'_{17}$ of a metal complex of formula (IV):

$$M\text{-}R'_{17a} \qquad (IV)$$

  in which M represents a metal atom and $R'_{17a}$ has the same meaning as previously, it being understood that it is not a hydrogen atom,
- conversion of the ketone function that can be represented by $R_{17}$ and $R'_{17}$ together into an oxime function, a hydrazono group or a methylene group,
- selective acylation in position 17 when $R_{17}$ is a hydroxyl,
- halogenation or alkylation in position 16,
- alkylation or acylation of the hydroxyl radical in position 3,
- total or partial reduction of the ethynylene group, when Y represents an unsaturated chain,
- oxidation of the sulphur into sulphoxide or sulphone,
- optional salification by an acid or a base.

**9.** Preparation process for the compounds of formula (I) as defined in claim 1, characterized in that a compound of formula (V):

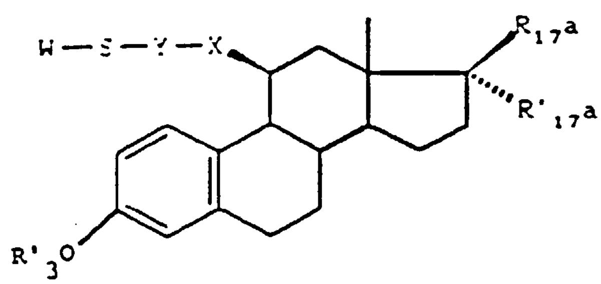

(V)

in which:

- $R_{17a}$ and $R'_{17a}$ have the meanings indicated in claim 1 for $R_{17}$ and $R'_{17}$ in which the optional reactive functions are optionally protected,
- X and Y have the same meaning as in claim 1,
- $R'_3$ represents a hydrogen atom or a protective group of the hydroxyl function,
- W represents a hydrogen atom or an acyl -COR radical in which R represents an alkyl radical which contains 1 to 5 carbon atoms,

is subjected to the action of a compound of formula (VI):

$$Za\text{-}hal' \qquad (VI)$$

or a salt of the latter, in which Za has the meaning indicated in claim 1 for Z in which the optional reactive functions are optionally protected and hal' represents a reactive group such as a halogen or a pseudohalogen such as a mesylate or tosylate group, in the presence of a base, then if appropriate is subjected to the action of an elimination agent of the protective groups, in order to obtain the compound of formula (IA) having the same definition as in claim 8 and which, if desired and if necessary, is subjected to any one of the reactions indicated for the compound of formula (IA) in claim 8.

**10.** As medicaments, the compounds of formula (I) as defined in any one of claims 1 to 6.

11. As medicaments, the compounds of formula (I) defined in claim 7.

12. The pharmaceutical compositions containing as active ingredient at least one of the medicaments defined in claim 10 or 11.